(19) Europäisches Patentamt
European Patent Office
Office européen des brevets

(11) **EP 3 928 859 A1**

(12) **EUROPEAN PATENT APPLICATION**

(43) Date of publication:
**29.12.2021 Bulletin 2021/52**

(51) Int Cl.:
*B01J 13/10* (2006.01)    *B01J 13/22* (2006.01)
*A01N 25/28* (2006.01)    *A23P 10/30* (2016.01)
*A61K 8/11* (2006.01)    *A61K 9/50* (2006.01)

(21) Application number: **20181641.0**

(22) Date of filing: **23.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Omya International AG**
**4665 Oftringen (CH)**

(72) Inventors:
• **SCHOELKOPF, Joachim**
**5727 Oberkulm (CH)**
• **HILTY-VANCURA, Florentine Marianne**
**8706 Meilen (CH)**
• **KIRYUKHIN, Maxim**
**670626 Singapore (SG)**

(74) Representative: **Glas, Holger**
**Maiwald Patentanwalts- und Rechtsanwaltsgesellschaft mbH**
**Elisenhof**
**Elisenstraße 3**
**80335 München (DE)**

(54) **SURFACE-REACTED CALCIUM CARBONATE IN A PROCESS FOR THE PRODUCTION OF A LOADED MICROCAPSULE**

(57)     The present invention relates to a process for the production of a microcapsule comprising an active ingredient or inactive precursor thereof, as well as a microcapsule obtainable thereby. The process involves the use of a surface-reacted calcium carbonate as a template for encapsulating the active ingredient or inactive precursor thereof. The active ingredient or inactive precursor thereof is loaded onto the surface-reacted calcium carbonate and subsequently encased by a multilayer shell comprising at least two complementary layers using layer-by-layer assembly. Further aspects of the invention relate to the use of a surface-reacted calcium carbonate as a template for encapsulating the active ingredient or inactive precursor thereof, a product comprising said microcapsule, as well as the use of said microcapsule.

Fig. 4b.

**Description**

The present invention relates to a process for the production of a microcapsule comprising an active ingredient or inactive precursor thereof, a microcapsule comprising at least one active ingredient or inactive precursor thereof obtainable by the inventive process, the use of a surface-reacted calcium carbonate as a template for encapsulating an active ingredient or inactive precursor thereof, a product comprising the microcapsule comprising an active ingredient or inactive precursor thereof, as well as the use of the microcapsule comprising an active ingredient or inactive precursor thereof.

Encapsulation of active ingredients is a well-known strategy for protecting active ingredients from external influences, such as aggressive media, or for delaying or controlling their release into the respective target. However, several of the conventional approaches for encapsulation, such as pan coating, centrifugal extrusion, hot melt extrusion, spray-drying, coacervation-phase separation, ionotropic gelation, interfacial polycondensation, or matrix polymerization, do not allow for the precise control of the encapsulation process, which may lead to comparatively large particles.

Layer-by-layer encapsulation has been used for depositing thin films onto a substrate, making use of attractive interactions between complementary layers, for example electrostatic interactions between oppositely charged polyelectrolytes, hydrogen bonding, hydrophobic interactions, covalent bonding, and complementary base pairing. Said substrate may also be a particulate material, such as a micelle, an emulsion droplet, a gas bubble or a solid non-porous or porous particle. The approach allows for obtaining small encapsulated particles. However, for the encapsulation of active ingredients, these have to be dissolved in emulsion droplets or adsorbed onto or absorbed into solid carriers, or templates, prior to encapsulation. Therefore, these approaches typically only achieve a low loading of the microcapsule with the active ingredient.

US 2008/0020051 A1 discloses a method for producing microcapsules by layer-by-layer encapsulation, in which at least one active compound is adsorbed in porous organic or inorganic templates.

D. V. Volodkin et al. report on the use of precipitated calcium carbonate microparticles formed by rapidly mixing solutions of calcium chloride and sodium carbonate for the production of microcapsules comprising dextran- or bovine serum albumin-bound dyes ("Matrix Polyelectrolyte Microcapsules: New System for Macromolecule Encapsulation", Langmuir 2004, 20, 3398-3406).

Application WO 2010/097814 A2 relates to a micro-capsule for the controlled release of flavonoid compounds for osteogenic action, wherein the flavonoid is absorbed in vaterite microparticles.

JP 2011-144056 A discloses a process for the encapsulation of proteins, peptides and low molecular weight substances in vaterite microparticles using layer-by-layer assembly.

The conventional templates as described hereinabove have the drawback that they cannot be loaded with high amounts of the active ingredient, e.g. amounts exceeding 1.5 wt.-%. Furthermore, known carriers for the production of microcapsules that can be loaded with higher amounts of the active ingredient, such as porous silica, cannot be removed under mild conditions, if this is desired or required for the intended application.

Surface-reacted calcium carbonate was first described in FR 2787802 B1, subsequently in WO 00/39222 A1 and US 2004/0020410 A1, and is based on the reaction of natural ground calcium carbonate with gaseous $CO_2$ and with one or more medium-strong to strong $H_3O^+$ ion providers. The obtained product is a porous calcium carbonate having a special surface structure, porosity, and specific surface area.

WO 2018/011343 A1 discloses a dosage form comprising a surface-reacted calcium carbonate, which is optionally loaded with an active agent, wherein the surface-reacted calcium carbonate is dispersed in a hot melt extruded polymer resin.

EP 2 591 772 A1 relates to coated controlled release active agent carriers, which may be obtained by loading an active agent onto a surface-reacted calcium carbonate, which is pelletized and coated.

It is disclosed in WO 2014/057026 A1 that a gastro-retentive drug formulation may be obtained by mixing a functionalized calcium carbonate with a formulating aid and a pharmaceutically active ingredient.

Document WO 2016/096997 A1 discloses a method for the production of a pharmaceutical delivery system, wherein a surface-reacted calcium carbonate is used as an excipient for improving the friability of said delivery system, the method comprising the step of roller compaction.

However, the aforementioned documents relate to the formation of larger aggregates, such as coated tablets or mini-tablets, having a size of well above 1 mm, and even in the range of centimetres.

In view of the foregoing, there is an ongoing need for production methods for microcapsules containing active ingredients, and in particular, for microcapsules containing high amounts of active ingredients.

Accordingly, it is an objective of the present invention to provide a process for the production of a microcapsule comprising an active ingredient or inactive precursor thereof, wherein one or more of the aforementioned drawbacks are overcome. It is desirable that the microcapsule can be loaded with high amounts of the active ingredients or inactive precursors thereof.

The foregoing and other objects are solved by the subject-matter as defined in the independent claims.

According to one aspect, the present invention provides a process for the production of a microcapsule comprising an active ingredient or inactive precursor thereof, wherein the process comprises the following steps:

a) providing a surface-reacted calcium carbonate, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment,
b) providing an active ingredient or inactive precursor thereof,
c) contacting the active ingredient or inactive precursor thereof with the surface-reacted calcium carbonate to obtain a loaded surface-reacted calcium carbonate, and
d) encasing the loaded surface-reacted calcium carbonate with a multilayer shell by consecutively depositing at least two complementary layers using layer-by-layer assembly onto the loaded surface-reacted calcium carbonate.

According to a further aspect of the present invention, a microcapsule comprising an active ingredient or inactive precursor thereof obtainable by a process according to the present invention is provided.

According to still a further aspect of the present invention, the use of a surface-reacted calcium carbonate as a template for encapsulating an active ingredient or inactive precursor thereof using layer-by-layer assembly is provided, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment.

According to still a further aspect of the present invention, a product comprising a microcapsule according to the present invention is provided, wherein the product is a pharmaceutical product, a foodstuff, a feedstuff, a food supplement, a feed supplement, a cosmetic product, a paper product, a painting product, a coating product, or an agricultural product.

According to still a further aspect of the present invention, the use of a microcapsule according to the present invention in pharmaceutical, cosmetic, nutraceutical, paper, paint, coating, biological, industrial or agricultural applications is provided.

Advantageous embodiments of the present invention are defined in the corresponding subclaims.

According to one embodiment the process further comprises the step e) of reacting the microcapsule obtained in step d) with an acidic compound to decompose the loaded surface-reacted calcium carbonate within the multilayer shell, preferably the acidic compound is selected from the group consisting of hydrochloric acid, hydrobromic acid, sulphuric acid, nitric acid, acetic acid, formic acid, hydrogen sulfate, dihydrogen phosphate, hydrogen phosphate, calcium scavenging agents, preferably ethylene diamine tetraacetic acid (EDTA), and salts and mixtures thereof, and more preferably the acidic compound is hydrochloric acid. According to a further embodiment step e) is carried out in an aqueous medium, preferably wherein the acidic compound is added until the pH of the aqueous medium is in the range from 1 to 6, more preferably from 1.5 to 5, even more preferably from 2 to 4.5, and most preferably from 2.5 to 3.5.

According to one embodiment the surface-reacted calcium carbonate has a BET specific surface area from 20 to 200 g/m$^2$, preferably 40 to 150 g/m$^2$, more preferably 70 to 120 g/m$^2$; and/or a volume median particle size $d_{50}$ from 0.1 to 75 $\mu$m, preferably from 0.5 to 50 $\mu$m, more preferably from 1 to 40 $\mu$m, even more preferably from 1.2 to 30 $\mu$m, and most preferably from 1.5 to 15 $\mu$m; and/or a volume top cut particle size $d_{98}$ from 0.2 to 150 $\mu$m, preferably from 1 to 100 $\mu$m, more preferably from 2 to 80 $\mu$m, even more preferably from 2.4 to 60 $\mu$m, and most preferably from 3 to 30 $\mu$m; and/or an intra-particle intruded specific pore volume in the range from 0.1 to 2.5 cm$^3$/g, more preferably from 0.2 to 2.2 cm$^3$/g, still more preferably from 0.4 to 2.0 cm$^3$/g and most preferably from 0.6 to 1.8 cm$^3$/g, determined by mercury porosimetry measurement. According to a further embodiment the active ingredient is a pharmaceutically active ingredient, a cosmetic active ingredient, a nutraceutical active ingredient, a biocide, a pesticide, a wetting agent, a UV protecting agent, a scavenger, a pro-drug, a precursor of a cosmetic active ingredient, a precursor of a nutraceutical active ingredient, a precursor of a biocide, a precursor of a pesticide, a precursor of a wetting agent, a precursor of a UV protecting agent, a precursor of a scavenger, or a mixture thereof, preferably the active ingredient or inactive precursor thereof is a macromolecular active ingredient or an inactive precursor thereof, more preferably a protein, and most preferably lactoferrin, bovine serum albumin, or an enzyme.

According to one embodiment the at least two complementary layers are formed from encapsulants independently selected from the group consisting of nanomaterials, macromolecular encapsulants and mixtures thereof, preferably the encapsulants are independently selected from the group consisting of polysaccharides, polyphenols, proteins, nucleic acids, cationic polyelectrolytes, anionic polyelectrolytes, nanomaterials and mixtures thereof, more preferably the encapsulants are independently selected from the group consisting of tannic acid, polyacrylic acid, poly(4-vinylpyridine), poly(glutamic acid), poly(lactic acid), chondroitin sulphate, dextran sulphate, poly(styrene sulfonate), sodium alginate, hyaluronic acid, polyphosphoric acid, polyvinyl sulfonic acid, polyvinyl phosphonic acid, polysulfates, dextrans, gum arabic, pectin, anionic cellulose derivatives, preferably carboxymethyl cellulose, pepsin, poly(dimethylammonium chloride), poly(ethylene oxide), poly(allylamine hydrochloride), poly(lysine), poly(lactic-co-glycolic acid), poly(arginine), protamine sulphate, polyethyleneimine, chitosan, glycol chitosan, montmorillonite, polyvinylamine, amine-containing polymers, such as polyamidoamine-epichlorohydrin and poly[2-(dimethylamino)ethyl methacrylate], bovine serum albumin, corresponding salts thereof, metal nanoparticles, metal oxide nanoparticles, carbon nanotubes, graphene, graphene oxide, nanoclays, and mixtures thereof, even more preferably the encapsulants are independently selected from the group consisting of tannic acid, poly(styrene sulfonate), poly(allylamine hydrochloride), bovine serum albumin, pepsin, corresponding salts thereof, and mixtures thereof, and most preferably the encapsulants are independently selected from the group consisting of tannic acid, pepsin, and corresponding salts thereof.

According to one embodiment step d) comprises the steps of i) incubating the loaded surface-reacted calcium carbonate in a first liquid medium comprising a first encapsulant to encase the loaded surface-reacted calcium carbonate with a first layer, ii) removing the first liquid medium and washing the loaded surface-reacted calcium carbonate obtained in step i) one or more times with a first solvent, preferably water, iii) incubating the loaded surface-reacted calcium carbonate obtained in step ii) in a second liquid medium comprising a second encapsulant being complementary to the first encapsulant to encase the loaded surface-reacted calcium carbonate with a second layer, iv) removing the second liquid medium and washing the loaded surface-reacted calcium carbonate obtained in step iii) one or more times with a second solvent, preferably water, and v) optionally repeating steps i) to iv) one or more times with the same or different first and second encapsulants and the same or different first and second liquid media and/or solvents.

According to a further embodiment the first encapsulant is a protein and the second encapsulant is a polyphenol; or the first encapsulant is a polyphenol and the second encapsulant is a protein; or the first encapsulant is a cationic polyelectrolyte and the second encapsulant is an anionic polyelectrolyte; or the first encapsulant is an anionic polyelectrolyte and the second encapsulant is a cationic polyelectrolyte; preferably one of the first encapsulant or the second encapsulant is selected from the group consisting of tannic acid, polyacrylic acid, poly(glutamic acid), poly(lactic acid), poly(lactic-co-glycolic acid), chondroitin sulphate, dextran sulphate, poly(styrene sulfonate), montmorillonite, alginic acid, sodium alginate, polyphosphoric acid, polyvinyl sulfonic acid, polyvinyl phosphonic acid, polysulfates, gum arabic, poly(ethylene oxide), anionic cellulose derivatives, preferably carboxymethyl cellulose, corresponding salts thereof, and mixtures thereof, preferably the one of the first encapsulant or the second encapsulant is selected from the group consisting of tannic acid, poly(styrene sulfonate), corresponding salts thereof, and mixtures thereof, and the other one of the first or the second encapsulant is selected from the group consisting of pepsin, poly(dimethylammonium chloride), poly(allylamine hydrochloride), poly(lysine), poly(arginine), protamine sulphate, polyethyleneimine, poly(4-vinylpyridine), chitosan, glycol chitosan, polyvinylamine, amine-containing polymers, preferably polyamidoamine-epichlorohydrin or poly[2-(dimethylamino)ethyl methacrylate], bovine serum albumin, corresponding salts thereof, and mixtures thereof, preferably the other one of the first encapsulant or the second encapsulant is selected from the group consisting of pepsin, poly(allylamine hydrochloride), corresponding salts thereof, and mixtures thereof.

According to one embodiment the process further comprises the step of f) drying the microcapsule, preferably the drying is freeze-drying. According to a further embodiment the microcapsule has a particle size in the range from 0.2 to 80 $\mu$m , preferably 0.5 to 60 $\mu$m, more preferably from 1 to 40 $\mu$m, even more preferably from 1 to 20 $\mu$m, and most preferably from 2 to 10 $\mu$m, determined by scanning electron microscopy, and/or the microcapsule comprises from 1 to 5, preferably 2 or 3 individual loaded surface-reacted calcium carbonate particles.

According to still a further embodiment the microcapsule comprises the active ingredient or inactive precursor thereof in an amount of at least 5 wt.-%, preferably at least 10 wt.-%, more preferably at least 25 wt.-%, and most preferably at least 50 wt.-%, based on the total weight of the microcapsule.

It should be understood that, for the purposes of the present invention, the following terms have the following meanings.

A "microcapsule" in the sense of the present invention is a spherical or ovoid particle with a particle size between 100 nm and 100 $\mu$m composed of a solid shell, which surrounds a core-forming space available to permanently or temporarily entrapped substances (cf. Vert et al., "Terminology for biorelated polymers and applications (IUPAC Recommendations 2012)", Pure Appl. Chem. 2012, 84(2), 377-410).

"Layer-by-layer assembly" refers to a process for preparing thin film multilayers on a template by consecutively depositing complementary layers onto the template. Thus, a multilayer, i.e., a periodic shell consisting of two or more alternating monolayers, is formed due to one of, or a combination of "complementary" interactions, e.g., electrostatic attraction, such as between encapsulants being oppositely charged polyelectrolytes; hydrogen bonding, such as between encapsulants bearing hydrogen bond donors and/or acceptors, e.g., proteins and polyphenols; hydrophobic interactions, such as $\pi$-$\pi$ interactions; covalent bonding, and complementary base pairing. More precisely, in the present invention, layer-by-layer assembly is used as a process for preparing a multilayer shell on a particulate template material. The layer-by-layer assembly technique is well-known to the skilled person, e.g., described in Wang et al., "Template Synthesis of Nanostructured Materials via Layer-by-Layer Assembly", Chem. Mater. 2008, 20, 848-858, and in "Multilayer Thin Films: Sequential Assembly of Nanocomposite Materials", 2nd Edition, Eds: G. Decher, J. Schlenoff. Weinheim: Wiley, 2012, pages 1 to 19.

For the purposes of the present invention, the term "multilayer shell" refers to a solid shell composed of at least two complementary layers formed from at least two encapsulants.

An "encapsulant" is understood to be any material, which is capable of forming a layer during layer-by-layer assembly, and includes macromolecular encapsulants and/or nanomaterials.

A "macromolecular encapsulant" is understood to be a macromolecular material capable of forming a layer during layer-by-layer assembly, and includes polyphenols, proteins, nucleic acids, polyelectrolytes and mixtures thereof.

A "polyelectrolyte" in the sense of the present invention is understood to be a macromolecular material having a net ionic charge, or a material, which is able to at least partially dissociate when dissolved or suspended in a polar solvent (e.g., water, acetone, ethanol, dimethyl sulfoxide or dimethyl formamide). Polyelectrolytes having a net cationic charge or being able to at least partially dissociate to form a polycationic residue are considered "cationic polyelectrolytes". A specific example of a cationic polyelectrolyte is poly(allylamine) hydrochloride. Similarly, polyelectrolytes having a net anionic charge or being able to at least partially dissociate to form a polyanionic residue are considered "anionic polyelectrolytes". A specific example of an anionic polyelectrolyte is polyacrylic acid.

A "macromolecular" material, such as a "macromolecular encapsulant", in the meaning of the present invention refers to a molecule having a molecular weight of more than 500 g/mol, preferably more than 800 g/mol, more preferably more than 1500 g/mol, and most preferably more than 10000 g/mol.

A "nanomaterial" in the sense of the present invention refers to a material of organic or inorganic, synthetic, biologic or mineral origin composed of discrete pieces, wherein said discrete pieces have at least one external dimension in the nanoscale range, i.e., below 100 nm. Thus, the term nanomaterial includes, e.g., nanoparticles, nanotubes, nanoribbons and nanosheets.

An "active ingredient" in the meaning of the present document is understood to be a chemical compound which causes a specific activity when applied to a target organism (e.g., human body, animal body or plant).

An "inactive precursor" of the active ingredient is understood to be a chemical compound, which is transformed into or releases an active ingredient by means of an activation step, e.g., upon exposure to a chemical stimulus, e.g., an acid, a base or an enzyme, in particular during metabolization, or upon exposure to visible or UV light.

For the purposes of the present invention, the term "prodrug" refers to an inactive precursor of a pharmaceutically active ingredient, i.e., a pharmaceutically inactive precursor, which is transformed into a pharmaceutically active ingredient after administration, preferably by metabolization.

A "surface-reacted calcium carbonate" according to the present invention is a reaction product of ground natural calcium carbonate (GNCC) or precipitated calcium carbonate (PCC) treated with carbon dioxide and one or more $H_3O^+$ ion donors, wherein the carbon dioxide is formed in situ by the $H_3O^+$ ion donors treatment. An $H_3O^+$ ion donor in the context of the present invention is a Brønsted acid and/or an acid salt.

The "particle size" of surface-reacted calcium carbonate herein, if not explicitly stated otherwise, is described as volume-based particle size distribution $d_x(vol)$, or $d_x$. Therein, the value $d_x(vol)$ represents the diameter relative to which x % by volume of the particles have diameters less than $d_x(vol)$. This means that, for example, the $d_{20}(vol)$ value is the particle size at which 20 vol.% of all particles are smaller than that particle size. The $d_{50}(vol)$ value is thus the volume median particle size, also referred to as average particle size, i.e. 50 vol.% of all particles are smaller than that particle size and the $d_{98}(vol)$ value, referred to as volume-based top cut particle size, is the particle size at which 98 vol.% of all particles are smaller than that particle size. If a particle size is given herein as weight-based particle size, then, e.g., the $d_{20}(wt)$ value is the particle size at which 20 wt.-% of all particles are smaller than that particle size. The $d_{50}(wt)$ value is thus the volume median particle size, also referred to as weight median particle size, i.e. 50 wt.-% of all particles are smaller than that particle size and the $d_{98}(wt)$ value, referred to as weight-based top cut particle size, is the particle size at which 98 wt.-% of all particles are smaller than that particle size. The term "grain diameter" is used synonymously with the term "particle size".

For the purpose of the present invention the "porosity" or "pore volume" refers to the intraparticle intruded specific pore volume.

In the context of the present invention, the term "pore" is to be understood as describing the space that is found between and/or within particles, i.e. that is formed by the particles as they pack together under nearest neighbour contact (interparticle pores), such as in a powder or a compact, and/or the void space within porous particles (intraparticle pores), and that allows the passage of liquids under pressure when saturated by the liquid and/or supports absorption of surface wetting liquids.

Throughout the present document, the term "specific surface area" (in $m^2/g$), which is used to define functionalized calcium carbonate or other materials, refers to the specific surface area as determined by using the BET method (using nitrogen as adsorbing gas), according to ISO 9277:2010.

The term "aqueous" suspension or solution refers to a system, wherein the liquid phase comprises, preferably consists of, water. However, said term does not exclude that the liquid phase of the aqueous suspension comprises minor amounts of at least one water-miscible organic solvent selected from the group comprising methanol, ethanol, acetone, acetonitrile, tetrahydrofuran and mixtures thereof. If the aqueous suspension comprises at least one water-miscible organic solvent, the liquid phase of the aqueous suspension comprises the at least one water-miscible organic solvent in an amount of from 0.1 to 40.0 wt.-% preferably from 0.1 to 30.0 wt.-%, more preferably from 0.1 to 20.0 wt.-% and most preferably from 0.1 to 10.0 wt.-%, based on the total weight of the liquid phase of the aqueous suspension. For example, the liquid phase of the aqueous suspension consists of water.

Where the term "comprising" is used in the present description and claims, it does not exclude other non-specified elements of major or minor functional importance. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Whenever the terms "including" or "having" are used, these terms are meant to be equivalent to "comprising" as defined above.

Where an indefinite or definite article is used when referring to a singular noun, e.g. "a", "an" or "the", this includes a plural of that noun unless something else is specifically stated.

Terms like "obtainable" or "definable" and "obtained" or "defined" are used interchangeably. This e.g. means that, unless the context clearly dictates otherwise, the term "obtained" does not mean to indicate that, e.g., an embodiment must be obtained by, e.g., the sequence of steps following the term "obtained" even though such a limited understanding is always included by the terms "obtained" or "defined" as a preferred embodiment.

The present invention provides a process for the production of a microcapsule comprising an active ingredient or inactive precursor thereof, wherein the process comprises the steps of a) providing a surface-reacted calcium carbonate, b) providing an active ingredient or inactive precursor thereof, c) contacting the active ingredient or inactive precursor thereof with the surface-reacted calcium carbonate to obtain a loaded surface-reacted calcium carbonate, and d) encasing the loaded surface-reacted calcium carbonate with a multilayer shell by consecutively depositing at least two complementary layers using layer-by-layer assembly onto the loaded surface-reacted calcium carbonate. The surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment.

When in the following reference is made to embodiments or technical details of the inventive process for the production of said microcapsule, it is to be understood that these embodiments or technical details also refer to the microcapsule obtainable by the inventive process, the inventive use of a surface-reacted calcium carbonate as a template for encapsulating an active ingredient or inactive precursor thereof using layer-by-layer assembly, the inventive product comprising the inventive microcapsule and the inventive use of the inventive microcapsule.

**Brief description of the figures**

Figure 1 shows SEM micrographs of (a) vaterite, (b) SRCC1 (c) SRCC2 and (d) SRCC3.

Figure 2 shows the calibration curve used to convert the peak area on an HPLC chromatogram into Lactoferrin concentration in analysed solution containing 8M of urea at pH 8.9.

Figure 3 shows SEM micrographs of the freeze-dried microcapsules comprising lactoferrin and a multilayer shell formed of 4 tannic acid-pepsin bilayers (altogether 8 monolayers), which have been assembled using SRCC1 as a template, wherein the template has been removed by hydrochloric acid, at different levels of magnification.

Figure 4 shows SEM micrographs of the freeze-dried microcapsules comprising lactoferrin and a multilayer shell formed of 4 tannic acid-pepsin bilayers (altogether 8 monolayers), which have been assembled using SRCC3 as a template, wherein the template has been removed by hydrochloric acid, at different levels of magnification.

**The surface-reacted calcium carbonate**

According to process step a) a surface-reacted calcium carbonate is provided. The surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment.

A $H_3O^+$ ion donor in the context of the present invention is a Brønsted acid and/or an acid salt.

In a preferred embodiment of the invention the surface-reacted calcium carbonate is obtained by a process comprising the steps of: (a) providing a suspension of natural or precipitated calcium carbonate, (b) adding at least one acid having a $pK_a$ value of 0 or less at 20°C or having a $pK_a$ value from 0 to 2.5 at 20°C to the suspension of step (a), and (c) treating the suspension of step (a) with carbon dioxide before, during or after step (b). According to another embodiment the surface-reacted calcium carbonate is obtained by a process comprising the steps of: (A) providing a natural or precipitated calcium carbonate, (B) providing at least one water-soluble acid, (C) providing gaseous $CO_2$, (D) contacting said natural or precipitated calcium carbonate of step (A) with the at least one acid of step (B) and with the

$CO_2$ of step (C), characterised in that: (i) the at least one acid of step B) has a $pK_a$ of greater than 2.5 and less than or equal to 7 at 20°C, associated with the ionisation of its first available hydrogen, and a corresponding anion is formed on loss of this first available hydrogen capable of forming a water-soluble calcium salt, and (ii) following contacting the at least one acid with natural or precipitated calcium carbonate, at least one water-soluble salt, which in the case of a hydrogen-containing salt has a $pK_a$ of greater than 7 at 20°C, associated with the ionisation of the first available hydrogen, and the salt anion of which is capable of forming water-insoluble calcium salts, is additionally provided.

"Natural ground calcium carbonate" (GCC) preferably is selected from calcium carbonate containing minerals selected from the group comprising marble, chalk, limestone and mixtures thereof. Natural calcium carbonate may comprise further naturally occurring components such as magnesium carbonate, alumino silicate etc.

In general, the grinding of natural ground calcium carbonate may be a dry or wet grinding step and may be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the calcium carbonate containing mineral material comprises a wet ground calcium carbonate containing mineral material, the grinding step may be performed under conditions such that autogenous grinding takes place and/or by horizontal ball milling, and/or other such processes known to the skilled man. The wet processed ground calcium carbonate containing mineral material thus obtained may be washed and dewatered by well-known processes, e.g. by flocculation, filtration or forced evaporation prior to drying. The subsequent step of drying (if necessary) may be carried out in a single step such as spray drying, or in at least two steps. It is also common that such a mineral material undergoes a beneficiation step (such as a flotation, bleaching or magnetic separation step) to remove impurities.

"Precipitated calcium carbonate" (PCC) in the meaning of the present invention is a synthesized material, generally obtained by precipitation following reaction of carbon dioxide and calcium hydroxide in an aqueous environment or by precipitation of calcium and carbonate ions, for example $CaCl_2$ and $Na_2CO_3$, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the precipitated calcium carbonate is precipitated calcium carbonate, preferably comprising aragonitic, vateritic or calcitic mineralogical crystal forms or mixtures thereof.

Precipitated calcium carbonate may be ground prior to the treatment with carbon dioxide and at least one $H_3O^+$ ion donor by the same means as used for grinding natural calcium carbonate as described above.

According to one embodiment of the present invention, the natural or precipitated calcium carbonate is in form of particles having a weight median particle size $d_{50}$ of 0.05 to 10.0 $\mu$m, preferably 0.2 to 5.0 $\mu$m, more preferably 0.4 to 3.0 $\mu$m, most preferably 0.6 to 1.2 $\mu$m, especially 0.7 $\mu$m. According to a further embodiment of the present invention, the natural or precipitated calcium carbonate is in form of particles having a weight-based top cut particle size $d_{98}$ of 0.15 to 55 $\mu$m, preferably 1 to 40 $\mu$m, more preferably 2 to 25 $\mu$m, most preferably 3 to 15 $\mu$m, especially 4 $\mu$m.

The natural and/or precipitated calcium carbonate may be used dry or suspended in water. Preferably, a corresponding slurry has a content of natural or precipitated calcium carbonate within the range of 1 wt.-% to 90 wt.-%, more preferably 3 wt.-% to 60 wt.-%, even more preferably 5 wt.-% to 40 wt.-%, and most preferably 10 wt.-% to 25 wt.-% based on the weight of the slurry.

The one or more $H_3O^+$ ion donor used for the preparation of surface reacted calcium carbonate may be any strong acid, medium-strong acid, or weak acid, or mixtures thereof, generating $H_3O^+$ ions under the preparation conditions. According to the present invention, the at least one $H_3O^+$ ion donor can also be an acidic salt, generating $H_3O^+$ ions under the preparation conditions.

According to one embodiment, the at least one $H_3O^+$ ion donor is a strong acid having a $pK_a$ of 0 or less at 20°C.

According to another embodiment, the at least one $H_3O^+$ ion donor is a medium-strong acid having a $pK_a$ value from 0 to 2.5 at 20°C. If the $pK_a$ at 20°C is 0 or less, the acid is preferably selected from sulphuric acid, hydrochloric acid, or mixtures thereof. If the $pK_a$ at 20°C is from 0 to 2.5, the $H_3O^+$ ion donor is preferably selected from $H_2SO_3$, $H_3PO_4$, oxalic acid, or mixtures thereof. The at least one $H_3O^+$ ion donor can also be an acidic salt, for example, $HSO_4^-$ or $H_2PO_4^-$, being at least partially neutralized by a corresponding cation such as $Li^+$, $Na^+$ or $K^+$, or $HPO_4^{2-}$, being at least partially neutralised by a corresponding cation such as Li+, $Na^+$, $K^+$, $Mg^{2+}$ or $Ca^{2+}$. The at least one $H_3O^+$ ion donor can also be a mixture of one or more acids and one or more acidic salts.

According to still another embodiment, the at least one $H_3O^+$ ion donor is a weak acid having a $pK_a$ value of

greater than 2.5 and less than or equal to 7, when measured at 20°C, associated with the ionisation of the first available hydrogen, and having a corresponding anion, which is capable of forming water-soluble calcium salts. Subsequently, at least one water-soluble salt, which in the case of a hydrogen-containing salt has a $pK_a$ of greater than 7, when measured at 20°C, associated with the ionisation of the first available hydrogen, and the salt anion of which is capable of forming water-insoluble calcium salts, is additionally provided. According to the preferred embodiment, the weak acid has a $pK_a$ value from greater than 2.5 to 5 at 20°C, and more preferably the weak acid is selected from the group consisting of acetic acid, formic acid, propanoic acid, and mixtures thereof. Exemplary cations of said water-soluble salt are selected from the group consisting of potassium, sodium, lithium and mixtures thereof. In a more preferred embodiment, said cation is sodium or potassium. Exemplary anions of said water-soluble salt are selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of drop wise addition, this addition preferably takes place within a time period of 10 minutes. It is more preferred to add said salt in one step.

According to one embodiment of the present invention, the at least one $H_3O^+$ ion donor is selected from the group consisting of hydrochloric acid, sulphuric acid, sulphurous acid, phosphoric acid, citric acid, oxalic acid, acetic acid, formic acid, and mixtures thereof. Preferably the at least one $H_3O^+$ ion donor is selected from the group consisting of hydrochloric acid, sulphuric acid, sulphurous acid, phosphoric acid, oxalic acid, $H_2PO_4^-$, being at least partially neutralised by a corresponding cation such as $Li^+$, $Na^+$ or $K^+$, $HPO_4^{2-}$, being at least partially neutralised by a corresponding cation such as $Li^+$, $Na^+$, $K^+$, $Mg^{2+}$, or $Ca^{2+}$ and mixtures thereof, more preferably the at least one acid is selected from the group consisting of hydrochloric acid, sulphuric acid, sulphurous acid, phosphoric acid, oxalic acid, or mixtures thereof, and most preferably, the at least one $H_3O^+$ ion donor is phosphoric acid.

The one or more $H_3O^+$ ion donor can be added to the suspension as a concentrated solution or a more diluted solution. Preferably, the molar ratio of the $H_3O^+$ ion donor to the natural or precipitated calcium carbonate is from 0.01 to 4, more preferably from 0.02 to 2, even more preferably 0.05 to 1 and most preferably 0.1 to 0.58.

As an alternative, it is also possible to add the $H_3O^+$ ion donor to the water before the natural or precipitated calcium carbonate is suspended.

In a next step, the natural or precipitated calcium carbonate is treated with carbon dioxide, wherein the carbon dioxide is formed in-situ by the $H_3O^+$ ion donor treatment. In addition, the carbon dioxide can be supplied from an external source.

$H_3O^+$ ion donor treatment and treatment with carbon dioxide can be carried out simultaneously which is the case when a strong or medium-strong acid is used. It is also possible to carry out $H_3O^+$ ion donor treatment first, e.g. with a medium strong acid having a $pK_a$ in the range of 0 to 2.5 at 20°C, wherein carbon dioxide is formed in situ, and thus, the carbon dioxide treatment will automatically be carried out simultaneously with the $H_3O^+$ ion donor treatment, followed by the additional treatment with carbon dioxide supplied from an external source.

In a preferred embodiment, the $H_3O^+$ ion donor treatment step and the carbon dioxide treatment step are repeated at least once, more preferably several times. According to one embodiment, the at least one $H_3O^+$ ion donor is added over a time period of at least about 5 min, preferably at least about 10 min, typically from about 10 to about 20 min, more preferably about 30 min, even more preferably about 45 min, and sometimes about 1 h or more.

Subsequent to the $H_3O^+$ ion donor treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the surface-reacted natural or precipitated calcium carbonate as an aqueous suspension having a pH of greater than 6.0, preferably greater than 6.5, more preferably greater than 7.0, even more preferably greater than 7.5.

Further details about the preparation of the surface-reacted natural calcium carbonate are disclosed in WO 00/39222 A1, WO 2004/083316 A1, WO 2005/121257 A2, WO 2009/074492 A1, EP 2 264 108 A1, EP 2 264 109 A1 and US 2004/0020410 A1, the content of these references herewith being included in the present application.

Similarly, surface-reacted precipitated calcium carbonate is obtained. As can be taken in detail from WO 2009/074492 A1, surface-reacted precipitated calcium carbonate is obtained by contacting precipitated calcium carbonate with $H_3O^+$ ions and with anions being solubilized in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by $H_3O^+$ ions, where said $H_3O^+$ ions are provided solely in the form of a counterion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in situ.

Said $H_3O^+$ ions may be provided by the addition of an acid or an acid salt of said anion, or the addition of an acid or an acid salt which simultaneously serves to provide all or part of said excess solubilized calcium ions.

In a further preferred embodiment of the preparation of the surface-reacted natural or precipitated calcium carbonate, the natural or precipitated calcium carbonate is reacted with the one or more $H_3O^+$ ion donors and/or the carbon dioxide in the presence of at least one compound selected from the group consisting of silicate, silica, aluminium hydroxide, earth alkali aluminate such as sodium or potassium aluminate, magnesium oxide, or mixtures thereof. Preferably, the at least one silicate is selected from an aluminium silicate, a calcium silicate, or an earth alkali metal silicate. These components can be added to an aqueous suspension comprising the natural or precipitated calcium carbonate before adding the one or more $H_3O^+$ ion donors, and optionally, carbon dioxide.

Alternatively, the silicate and/or silica and/or aluminium hydroxide and/or earth alkali aluminate and/or magnesium oxide components) can be added to the aqueous suspension of natural or precipitated calcium carbonate while the reaction of natural or precipitated calcium carbonate with the one or more $H_3O^+$ ion donors and carbon dioxide has already started. Further details about the preparation of the surface-reacted natural or precipitated calcium carbonate in the presence of at least one silicate and/or silica and/or aluminium hydroxide and/or earth alkali aluminate component(s) are disclosed in WO 2004/083316 A1, the content of this reference herewith being included in the present application.

The surface-reacted calcium carbonate can be kept in suspension, optionally further stabilised by a dispersant. Conventional dispersants known to the skilled person can be used. A preferred dispersant is comprised of polyacrylic acids and/or carboxymethylcelluloses and salts thereof.

Alternatively, the aqueous suspension described above can be dried, thereby obtaining the solid (i.e. dry or containing as little water that it is not in a fluid form) surface-reacted natural or precipitated calcium carbonate in the form of granules or a powder.

The surface-reacted calcium carbonate may have a specific surface area of from 15 $m^2/g$ to 200 $m^2/g$, preferably from 27 $m^2/g$ to 180 $m^2/g$, more preferably from 30 $m^2/g$ to 160 $m^2/g$, even more preferably from 45 $m^2/g$ to 150 $m^2/g$, most preferably from 48 $m^2/g$ to 140 $m^2/g$, measured using nitrogen and the BET method. For example, the surface-reacted calcium carbonate may have a specific surface area of from 75 $m^2/g$ to 100 $m^2/g$, measured using nitrogen and the BET method. The BET specific surface area in the meaning of the present invention is defined as the surface area of the particles divided by the mass of the particles. As used therein the specific surface area is measured by adsorption using the BET isotherm (ISO 9277:2010) and is specified in $m^2/g$.

Furthermore, the surface-reacted calcium carbonate particles may have a volume median grain diameter $d_{50}$ (vol) of from 0.1 to 75 $\mu$m, preferably from 0.5 to 50 $\mu$m, more preferably 1 to 40 $\mu$m, even more preferably from 1.2 to 30 $\mu$m, and most preferably from 1.5 to 15 $\mu$m.

Furthermore, the surface-reacted calcium carbonate particles may have a grain diameter $d_{98}$ (vol) of from 0.2 to 150 $\mu$m, preferably from 1 to 100 $\mu$m, more preferably 2 to 80 $\mu$m, even more preferably from 2.4 to 60 $\mu$m , and most preferably from 3 to 30 $\mu$m.

The value $d_x$ represents the diameter relative to which x % of the particles have diameters less than $d_x$. This means that the $d_{98}$ value is the particle size at which 98 % of all particles are smaller. The $d_{98}$ value is also designated as "top cut". The $d_x$ values may be given in volume or weight percent. The $d_{50}$ (wt) value is thus the weight median particle size, i.e. 50 wt.-% of all grains are smaller than this particle size, and the $d_{50}$ (vol) value is the volume median particle size, i.e. 50 vol.-% of all grains are smaller than this particle size.

Volume median grain diameter $d_{50}$ was evaluated using a Malvern Mastersizer 2000 Laser Diffraction System. The $d_{50}$ or $d_{98}$ value, measured using a Malvern Mastersizer 2000 Laser Diffraction System, indicates a diameter value such that 50 % or 98 % by volume, respectively, of the particles have a diameter of less than this value. The raw data obtained by the measurement are analysed using the Mie theory, with a particle refractive index of 1.57 and an absorption index of 0.005.

The weight median grain diameter is determined by the sedimentation method, which is an analysis of sedimentation behaviour in a gravimetric field. The measurement is made with a Sedigraph™ 5100 or 5120, Micromeritics Instrument Corporation. The method and the instrument are known to the skilled person and are commonly used to determine grain size of fillers and pigments. The measurement is carried out in an aqueous solution of 0.1 wt.-% $Na_4P_2O_7$. The samples were dispersed using a high speed stirrer and sonicated.

The processes and instruments are known to the skilled person and are commonly used to determine grain size of fillers and pigments.

The specific pore volume is measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 $\mu$m (~ nm). The equilibration time used at each pressure step is 20 seconds. The sample material is sealed in a 5 $cm^3$ chamber powder penetrometer for analysis. The data are corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

The total pore volume seen in the cumulative intrusion data can be separated into two regions with the intrusion data from 214 $\mu$m down to about 1 - 4 $\mu$m showing the coarse packing of the sample between any agglomerate structures contributing strongly. Below these diameters lies the fine interparticle packing of the particles themselves. If they also have intraparticle pores, then this region appears bi modal, and by taking the specific pore volume intruded by mercury into pores finer than the modal turning point, i.e. finer than the bi-modal point of inflection, the specific intraparticle pore volume is defined. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired pore volume of the internal pores alone in terms of the pore volume per unit mass (specific pore volume). The same principle of subtraction, of course, applies for isolating any of the other pore size regions of interest.

Preferably, the surface-reacted calcium carbonate has an intra-particle intruded specific pore volume in the range from 0.1 to 2.3 cm$^3$/g, more preferably from 0.2 to 2.0 cm$^3$/g, especially preferably from 0.4 to 1.8 cm$^3$/g and most preferably from 0.6 to 1.6 cm$^3$/g, calculated from mercury porosimetry measurement.

The intra-particle pore size of the surface-reacted calcium carbonate preferably is in a range from 4 to 200 nm, more preferably in a range from 8 to 150 nm, especially preferably from 10 to 100 nm and most preferably from 15 to 50, e.g. 15 to 40 nm, determined by mercury porosimetry measurement. Alternatively, the average pore size d of the surface-reacted calcium carbonate may be in the range from 4 to 200 nm, preferably from 8 to 150 nm, more preferably from 15 to 50 nm, e.g., 15 to 40 nm, calculated from the BET surface area measurement under the assumption of a cylindrical pore shape using the equation d = 4 V/A, wherein A represents the determined BET surface area and V refers to the total pore volume of the particles as determined by the amount of adsorbed nitrogen at $p/p_0$ = 0.99.

It is appreciated that the surface-reacted calcium carbonate can be one kind of surface-reacted calcium carbonate or a mixture of different kinds of surface-reacted calcium carbonate(s). In one embodiment of the present invention, the surface-reacted calcium carbonate comprises, preferably consists of, one kind of surface-reacted calcium carbonate. Alternatively, the surface-reacted calcium carbonate comprises, preferably consists of, two or more kinds of surface-reacted calcium carbonates. For example, the surface-reacted calcium carbonate comprises, preferably consists of, two or three kinds of surface-reacted calcium carbonates. Preferably, the surface-reacted calcium carbonate comprises, more preferably consists of, one kind of surface-reacted calcium carbonate.

In a preferred embodiment, the surface-reacted calcium carbonate has

- a BET specific surface area from 20 to 200 g/m$^2$, preferably 40 to 150 g/m$^2$, more preferably 70 to 120 g/m$^2$; and/or
- a volume median particle size $d_{50}$ from 0.1 to 75 $\mu$m, preferably from 0.5 to 50 $\mu$m, more preferably from 1 to 40 $\mu$m, even more preferably from 1.2 to 30 $\mu$m, and most preferably from 1.5 to 15 $\mu$m; and/or
- a volume top cut particle size $d_{98}$ from 0.2 to 150 $\mu$m, preferably from 1 to 100 $\mu$m, more preferably from 2 to 80 $\mu$m, even more preferably from 2.4 to 60 $\mu$m, and most preferably from 3 to 30 $\mu$m; and/or
- an intra-particle intruded specific pore volume in the range from 0.1 to 2.5 cm$^3$/g, more preferably from 0.2 to 2.2 cm$^3$/g, still more preferably from 0.4 to 2.0 cm$^3$/g and most preferably from 0.6 to 1.8 cm$^3$/g, determined by mercury porosimetry measurement.

In a particularly preferred embodiment, the surface-reacted calcium carbonate has an intra-particle intruded specific pore volume in the range from 0.1 to 2.5 cm$^3$/g, more preferably from 0.2 to 2.2 cm$^3$/g, still more preferably from 0.4 to 2.0 cm$^3$/g and most preferably from 0.6 to 1.8 cm$^3$/g, determined by mercury porosimetry measurement.

Additionally or alternatively, it is preferred that the surface-reacted calcium carbonate has a BET specific surface area from 20 to 200 g/m$^2$, preferably 40 to 150 g/m$^2$, more preferably 70 to 120 g/m$^2$.

According to one embodiment the surface-reacted calcium carbonate is food-grade, i.e. it meets the standards set for human consumption. According to another embodiment the surface-reacted calcium carbonate is pharmacy-grade, i.e. it meets pharmaceutical standards.

**The active ingredient**

According to process step b), an active ingredient or inactive precursor thereof is provided.

The active ingredient or inactive precursor thereof may be selected from any suitable active ingredient or inactive precursor thereof known in the art that can be loaded onto surface-reacted calcium carbonate and retained inside the subsequently assembled microcapsule. The active ingredient or inactive precursor thereof may comprise, preferably consist of, only one type of active ingredient or an inactive precursor thereof. Alternatively, the active ingredient may comprise, preferably consist of, a mixture of two or more types of active ingredients or inactive precursors thereof. Furthermore, also mixtures of one or more active ingredients and one or more inactive precursors of the same or different active ingredient may be provided in process step b). When in the following reference is made to an active ingredient, it is to be understood that also inactive precursors of said active ingredient are to be encompassed.

For example, the active ingredient or inactive precursor thereof may be a pharmaceutically active ingredient, a cosmetic active ingredient, a nutraceutical active ingredient, a biocide, a pesticide, a wetting agent, a UV protecting agent, a scavenger, a pro-drug, a precursor of a cosmetic active ingredient, a precursor of a nutraceutical active ingredient, a precursor of a biocide, a precursor of a pesticide, a precursor of a wetting agent, a precursor of a UV protecting agent, or a precursor of a scavenger, or a mixture thereof. Preferably, the active ingredient or inactive precursor thereof is a macromolecular active ingredient or an inactive precursor thereof, more preferably a protein, and most preferably lactoferrin, bovine serum albumin, or an enzyme

Preferably, the active ingredient or inactive precursor thereof is a pharmaceutically active ingredient, a pro-drug or a mixture thereof, more preferably the active ingredient or inactive precursor thereof is a macromolecular active ingredient or an inactive precursor thereof being a macromolecular pharmaceutically active ingredient, a macromolecular pro-drug or a mixture thereof.

In another preferred embodiment of the present invention, an active ingredient or pro-drug, preferably a macromolecular active ingredient or macromolecular pro-drug is provided in step b). In yet another preferred embodiment of the present invention, an active ingredient is provided in step b). Thus, preferably, in step b), a compound selected from the group consisting of a pharmaceutically active ingredient, a cosmetic active ingredient, a nutraceutical active ingredient, a biocide, a pesticide, a wetting agent, a UV protecting agent, a scavenger, a pro-drug and mixtures thereof, preferably selected from the group consisting of a macromolecular pharmaceutically active ingredient, a macromolecular cosmetic active ingredient, a macromolecular nutraceutical active ingredient, a macromolecular biocide, a macromolecular pesticide, a macromolecular wetting agent, a macromolecular UV protecting agent, a macromolecular scavenger, a macromolecular pro-drug and mixtures thereof, is provided.

In one embodiment, the active ingredient is selected from pharmaceutically active ingredients. A "pharmaceutically active ingredient" in the meaning of the present invention refers to a pharmaceutically active ingredient of synthetic origin, semi-synthetic origin, and/or natural origin as well as a prodrug thereof. The activation of such prodrugs is known to the skilled person and commonly includes, e.g. activation in the stomach and/or gastro-intestinal pathway such as acidic activation or tryptic- or chimotryptic cleavage. It lies within the understanding of the skilled person that the mentioned activation methods are of mere illustrative character and are not intended to be of limiting character.

The pharmaceutically active ingredient may be selected from any suitable compound known to the skilled person, and may include any compound that provides prophylactic and/or therapeutic properties when administered to humans and/or animals.

According to one embodiment, the pharmaceutically active ingredient is an anti-tartar agent. Anti-tartar agents useful herein include phosphates, preferably pyrophosphates, polyphosphates, polyphosphonates, or mixtures thereof. Pyrophosphates are among the best known phosphates for use in dental care products. Pyrophosphate ions delivered to the teeth derive from pyrophosphate salts. The pyrophosphate salts that may be useful in the present invention include dialkali metal pyrophosphate salts, tetra-alkali metal pyrophosphate salts, and mixtures thereof. Disodium dihydrogen pyrophosphate ($Na_2H_2P_2O_7$), tetrasodium pyrophosphate ($Na_4P_2O_7$), and tetrapotassium pyrophosphate ($K_4P_2O_7$) in their non-hydrated as well as hydrated forms may also be preferred. Examples of suitable anticalculus phosphates may include potassium and sodium pyrophosphates, sodium tripolyphosphate, diphosphonates, such as ethane-1-hydroxy-1,1-diphosphonate; 1-azacycloheptane-1,1-diphosphonate, and linear alkyl diphosphonates. Other examples of suitable anti-tartar agents are linear carboxylic acids, sodium citrate, or zinc citrate.

Agents that may be used in place of or in combination with the above pyrophosphate salt include materials such as synthetic anionic polymers including polyacrylates and copolymers of maleic anhydride or acid and methyl vinyl ether, e.g. Gantrez, as described, for example, in U. S. Patent Number 4,627,977, to Gaffar et al., herein incorporated by reference in its entirety as to the description of such agents, as well as e.g. polyamino propane sulphonic acid (AMPS), zinc citrate trihydrate, polyphosphates, e.g. tripolyphosphate and hexametaphosphate, diphosphonates, e.g. EHDP and AMP, polypeptides, such as polyaspartic and polyglutamic acids, and mixtures thereof.

In another embodiment, the pharmaceutically active ingredient is an antimicrobial agent. The antimicrobial agent may be an oral active agent and/or a systemic active agent. Examples of antimicrobial agents may include, but are not limited to, 5-chloro-2-(2,4-dichlorophenoxy)-phenol, commonly referred to as triclosan, chlorhexidine, alexidine, hexetidine, sanguinarine, benzalkonium chloride, salicylamide, domiphen bromide, cetylpyridinium chloride (CPC), tetradecyl pyridiniurn chloride (TPC), N-tetradecyl-4-ethyl pyridinium chloride (TDEPC), octenidine, delmopinol, octapinol, and other piperidino derivatives, niacin preparations, zinc/stannous ion agents, antibiotics such as augmentin, amoxycillin, tetracycline, doxycyline, minocycline, and metronidazole, and analogues, derivatives and salts of the above anti-microbial agents and mixtures thereof.

In still another embodiment, the pharmaceutically active ingredient is an anti-inflammatory agent. Examples of anti-inflammatory agents may include, but are not limited to, non-steroidal anti-inflammatory agents or NSAIDs, such as propionic acid derivatives, acetic acid derivatives, fenamic acid derivatives, biphenylcarboxylic acid derivative, and oxicams. NSAIDs are described e.g. in U.S. patent no. 4 985 459. Examples of suitable NSAIDs include acetylsalicylic acid, ibuprofen, naproxen, benoxaprofen, flurbiprofen, fenoprofen, fenbufen, ketoprofen, indoprofen, pirprofen, carprofen, oxaprozin, pranoprofen, microprofen, tioxaprofen, suprofen, alminoprofen, tiaprofenic acid, fluprofen, bucloxic acid, or mixtures thereof. Other suitable anti-inflammatory agents are steroidal anti-inflammatory agents such as hydrocortisone, and COX-2 inhibitors such as meloxicam, celecoxib, rofecoxib, valdecoxib, etoricoxib, or mixtures thereof.

In yet another embodiment, the pharmaceutically active ingredient is an upper respiratory agent such as phenylephrine, diphenhydramine, dextromethorphan, bromhexine and chlorpheniramine, a gastrointestinal agent such as famotidine, loperamide and simethicone, an antifungal such as miconazole nitrate, an antibiotic or an analgesic such as ketoprofen and fluributoprofen.

In one embodiment of the present invention, the pharmaceutically active ingredient may be selected from vitamin E, i.e. tocopheroles, vitamin C, i.e. ascorbic acid and its salts.

In another embodiment of the present invention, the pharmaceutically active ingredient may be selected from vitamins, such as vitamins B, C and E; minerals, such as fluorides, especially sodium fluoride, sodium monofluorophosphate and stannous fluoride; anti-odours, such as zinc and cyclodextrins; propellants, such as 1,1,2,2-tetrafluoroethane (HFC-134a), optionally being liquefied, and 1,1,1,2,3,3,3-heptafluororpropane (HFC-227), optionally being liquefied.

In yet another embodiment of the present invention, the pharmaceutically active ingredient may be selected from ephedrine, magaldrate, pseudoephedrine, sildenafil, xylocaine, benzalkonium chloride, caffeine, phenylephrine, amfepramone, orlistat, sibutramine, acetaminophen, aspirin, aluminium amino acetate, aluminium amino acetate in combination with magnesium oxide, aluminium oxide hydrate in combination with magnesium oxide, calcium carbonate in combination with magnesium hydroxide, calcium carbonate, dihydroxy aluminium sodium carbonate, magnesium oxide, glitazones, metformin, chlorpromazine, dimenhydrinat, domperidone, meclozine, metoclopramide, odansetron, prednisolone, promethazine, acrivastine, cetirizine, cinnarizine, clemastine, cyclizine, desloratadine, dexchlorpheniramine, dimenhydrinate, ebastine, fexofenadine, ibuprofen, levolevoproricin, loratadine, meclozine, mizolastine, promethazine, miconazole, vitamin B12, folic acid, ferro compounds, vitamin C, chlorhexidine diacetate, fluoride, decapeptide KSL, aluminium fluoride, aminochelated calcium, ammonium fluoride, ammonium fluorosilicate, ammonium monofluorphosphate, calcium fluoride, calcium gluconate, calcium glycerophosphate, calcium lactate, calcium monofluorphosphate, calciumcarbonate, carbamide, cetyl pyridinium chloride, chlorhexidine, chlorhexidine digluconate, chlorhexidine chloride, chlorhexidine diacetate, CPP caseine phospho peptide, hexetedine, octadecentyl ammonium fluoride, potassium fluorosilicate, potassium chloride, potassium monofluorophosphate, sodium bi carbonate, sodium carbonate, sodium fluoride, sodium fluorosilicate, sodium monofluorophosphate, sodium tripolyphosphate, stannous fluoride, stearyl trihydroxyethyl propylenediamine dihydrofluoride, strontium chloride, tetra potassium pyrophosphate, tetra sodium pyrophosphate, tripotassium orthophosphate, trisodium orthophosphate, alginic acid, aluminium hydroxide, sodium bicarbonate, sildenafil, tadalafil, vardenafil, yohimbine, cimetidine, nizatidine, ranitidine, acetylsalicylic acid, clopidogrel, acetylcysteine, bromhexine, codeine, dextromethorphan, diphenhydramine, noscapine, phenylpropanolamine, vitamin D, simvastatin, bisacodyl, lactitol, lactulose, magnesium oxide, sodium picosulfate, senna glycosides, benzocaine, lidocaine, tetracaine, almotriptan, eletriptan, naratriptan, rizatriptan, sumatriptan, zolmitriptan, calcium, chromium, copper, iodine, iron, magnesium, manganese, molybdenium, phosphor, selenium, zinc, chloramine, hydrogenperoxide, metronidazole, triamcinolonacetonide, benzethonium chloride, cetyl pyridinium chloride, chlorhexidine, fluoride, lidocaine, amphotericin, miconazole, nystatin, fish oil, ginkgo biloba, ginseng, ginger, purple coneflower, saw palmetto, cetirizine, levocetirizine, loratadine, diclofenac, flurbiprofen, acrivastine pseudoephedrine, loratadine pseudoephedrine, glucosamine, hyaluronic acid, decapeptide KSL-W, decapeptide KSL, resveratrol, misoprostol, bupropion, ondansetron HCl, esomeprazole, lansoprazole, omeprazole, pantoprazole, rabeprazole, bacteria and the like, loperamide, simethicone, acetylsalicylic acid and others, sucralfate, vitamin A, vitamin B1, vitamin B12, vitamin B2, vitamin B6, biotin, vitamin C, vitamin D, vitamin E, folinic acid, vitamin K, niacin, Q10, clotrimazole, fluconazole, itraconazole, ketoconazole, terbinafine, allopurinol, probenecid, atorvastatin, fluvastatin, lovastatin, nicotinic acid, pravastatin, rosuvastatin, simvastatin, pilocarpine, naproxen, alendronate, etidronate, raloxifene, risedronate, benzodiazepines, disulfiram, naltrexone, buprenorphine, codeine, dextropropoxyphene, fentanyl, hydromorphone, ketobemidone, ketoprofen, methadone, morphine, naproxen, nicomorphine, oxycodone, pethidine, tramadol, amoxicillin, ampicillin, azithromycin, ciprofloxacin, clarithromycin, doxycyclin, erythromycin, fusidic acid, lymecycline, metronidazole, moxifloxacin, ofloxacin, oxytetracycline, phenoxymethylpenicillin, rifamycins, roxithromycin, sulfamethizole, tetracycline, trimethoprim, vancomycin, acarbose, glibenclamide, gliclazide, glimepiride, glipizide, insulin, repaglinide, tolbutamide, oseltamivir, aciclovir, famciclovir, penciclovir, valganciclovir, amlopidine, diltiazem, felodipine, nifedipine, verapamil, finasteride, minoxidil, cocaine, buphrenorphin, clonidine, methadone, naltrexone, calcium antagonists, clonidine, ergotamine, β-blockers, aceclofenac, celecoxib, dexiprofen, etodolac, indometacin, ketoprofen, ketorolac, lornoxicam, meloxicam, nabumetone, oiroxicam, parecoxib, phenylbutazone, piroxicam, tiaprofenic acid, tolfenamic acid, aripiprazole, chlorpromazine, chlorprothixene, clozapine, flupentixol, fluphenazine, haloperidol, lithium carbonate, lithium citrate, melperone, penfluridol, periciazine, perphenazine, pimozide, pipamperone, prochlorperazine, risperidone, thioridizin, fluconazole, itraconazole, ketoconazole, voriconazole, opium, benzodiazepines, hydroxine, meprobamate, phenothiazine, aluminiumaminoacetate, esomeprazole, famotidine, magnesium oxide, nizatide, omeprazole, pantoprazole, fluconazole, itraconazole, ketoconazole, metronidazole, amphetamine, atenolol, bisoprolol fumarate, metoprolol, metropolol, pindolol, propranolol, auranofin, and bendazac. It should be understood that, if the pharmaceutically active ingredient tends to decompose under acidic conditions, e.g. is calcium carbonate in combination with magnesium hydroxide or calcium carbonate, optional step e) of decomposing the template may not be performed.

In still another embodiment of the present invention, the pharmaceutically active ingredient may be selected from the group comprising analgesic, anaesthetic, antipyretic, anti-allergic, anti-arrhythmic, appetite suppressant, antifungal, anti-inflammatory, broncho dilator, cardiovascular, coronary dilator, cerebral dilator, peripheral vasodilator, anti-infective, psychotropic, anti-manic, stimulant, antihistamine, laxative, decongestant, gastro-intestinal sedative, sexual dysfunction agent, desinfectants, anti-diarrheal, anti-anginal, vasodilator, anti-hypertensive, vasoconstrictor, migraine treating, antibiotic, tranquilizer, antipsychotic, anti-tumour, anticoagulant, antithrombotic, hypnotic, sedative, anti-emetic, anti-nauseant, anticonvulsant, neuromuscular, hyper- and hypoglycaemic, thyroid and antithyroid, diuretic, antispasmodic, uterine relaxant, anti-obesity, anoretic, spasnolytics, anabolic, erythropoietic, anti-asthmatic, expectorant, cough suppressant, mucolytic, anti-uricemic agent, dental vehicle, breath freshener, antacid, anti-diuretic, anti-flatulent, betablocker, teeth whitener, enzyme, co-enzyme, protein, energy booster, fibre, probiotic, prebiotic, antimicrobial, NSAID, anti-tussive, decongestant, anti-histamine, expectorant, anti-diarrheal, hydrogen antagonist, proton pump inhibitor, general nonselective CNS depressant, general nonselective CNS stimulant, selectively CNS function modifying, antiparkinsonism, narcotic-analgetic, analgetic-antipyretic, psychopharmacological, and sexual dysfunction agents.

In still another embodiment of the present invention, the pharmaceutically active ingredient may be selected from the group comprising casein glyco-macro-peptide (CGMP), triclosan, cetyl pyridinium chloride, domiphen bromide, quaternary ammonium salts, zinc components, sanguinarine, fluorides, alexidine, octonidine, EDTA, aspirin, acetaminophen, ibuprofen, ketoprofen, diflunisal, fenoprofen calcium, naproxen, tolmetin sodium, indomethacin, benzonatate, caramiphen edisylate, menthol, dextromethorphan hydrobromide, theobromine hydrochloride, chlophendianol hydrochloride, pseudoephedrine hydrochloride, phenylephrine, phenylpropanolamine, pseudoephedrine sulphate, brompheniramine maleate, chlorpheniramine maleate, carbinoxamine maleate, clemastine fumarate, dexchlorpheniramine maleate, dephenhydramine hydrochloride, diphenpyralide hydrochloride, azatadine maleate, diphenhydramine citrate, doxylamine succinate, promethazine hydrochloride, pyrilamine maleate, tripellenamine citrate, triprolidine hydrochloride, acrivastine, loratadine, brompheniramine, dexbrompheniamine, guaifenesin, ipecac, potassium iodide, terpin hydrate, loperamide, famotidine, ranitidine, omeprazole, lansoprazole, aliphatic alcohols, barbiturates, caffeine, strychnine, picrotoxin, pentyenetetrazol, phenyhydantoin, phenobarbital, primidone, carbamazapine, etoxsuximide, methsuximide, phensuximide, trimethadione, diazepam, benzodiazepines, phenacemide, pheneturide, acetazolamide, sulthiame, bromide, levodopa, amantadine, morphine, heroin, hydromorphone, metopon, oxymorphone, levophanol, codeine, hydrocodone, xycodone, nalorphine, naloxone, naltrexone, salicylates, phenylbutazone, indomethacin, phenacetin, chlorpromazine, methotrimeprazine, haloperidol, clozapine, reserpine, imipramine, tranylcypromine, phenelzine, lithium, sildenafil citrate, tadalafil, and vardenafil HCI.

In one embodiment of the present invention, the pharmaceutically active ingredient may be selected from the group comprising ACE-inhibitors, antianginal drugs, anti-arrhythmias, anti-asthmatics, anti-cholesterolemics, analgesics, anesthetics, anticonvulsants, anti-depressants, anti-diabetic agents, anti-diarrhea preparations, antidotes, anti-histamines, anti-hypertensive drugs, anti-inflammatory agents, anti-lipid agents, anti-manics, anti-nauseants, anti-stroke agents, anti-thyroid preparations, anti-tumour drugs, anti-viral agents, acne drugs, alkaloids, amino acid preparations, anti-tussives, anti-uricemic drugs, anti-viral drugs, anabolic preparations, systemic and non-systemic antiinfective agents, anti-neoplasties, antiparkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocriptine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumour drugs, anti-coagulants, antithrombotic drugs, hypnotics, anti-emetics, anti-nauseants, anti-convulsants, neuromuscular drugs, hyper- and hypoglycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

In another embodiment of the present invention, the pharmaceutically active ingredient may be selected from the group comprising antacids, H2-antagonists, and analgesics. For example, antacid dosages can be prepared using the ingredients calcium carbonate alone or in combination with magnesium hydroxide, and/or aluminium hydroxide, or using aluminium hydroxide, dihydroxyaluminium aminoacetate, aminoacetic acid, aluminium phosphate, dihydroxyaluminium sodium carbonate, bicarbonate, bismuth aluminate, bismuth carbonate, bismuth subcarbonate, bismuth subgallate, bismuth subnitrate, bismuth subsilysilate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminium mono-ordibasic calcium phosphate, tricalcium phosphate, potassium bicarbonate, sodium tartrate, sodium bicarbonate, magnesium aluminosilicates, tartaric acids and salts. Moreover, antacids can be used in combination with H2-antagonists, such as cimetidine, ranitidine hydrochloride, famotidine, nizatidien, ebrotidine, mifentidine, roxatidine, pisatidine and aceroxatidine. Analgesics include opiates and opiate derivatives, such as Oxycontin™, ibuprofen, aspirin, acetaminophen, and combinations thereof that may optionally include caffeine. It should be understood that, if the pharmaceutically active ingredient tends to decompose under acidic conditions, e.g. is calcium carbonate in combination with magnesium hydroxide or calcium carbonate, optional step e) of decomposing the template may not be performed.

In yet another embodiment of the present invention, the pharmaceutically active ingredient may be selected from the group comprising anti-diarrheals such as Immodium™ AD, anti-histamines, anti-tussives, decongestants, vitamins, breath fresheners, anxiolytics such as Xanax™; anti-psychotics such as Clozaril™ and Haldol™; non-steroidal anti-inflammatories (NSAIDs) such as ibuprofen, naproxen sodium, Voltaren™ and Lodine™, anti-histamines such as Claritin™, Hismanal™, Relafen™, and Tavist™; antiemetics such as Kytril™ and Cesamet™; bronchodilators such as Bentolin™, Proventil™; anti-depressants such as Prozac™, Zoloft™, and Paxil™; anti-migraines such as Imigra™, ACE-inhibitors such as Vasotec™, Capoten™ and Zestril™; anti-Alzheimer's agents, such as Nicergoline™; CaH-antagonists such as Procardia™, Adalat™, and Calan™; analgesics/anesthetics such as menthol, phenol, hexylresorcinol, benzocaine, dyclonine hydrochloride, benzyl alcohol, salicyl alcohol, and combinations thereof; demulcents such as slippery elm bark, pectin, gelatin, and combinations thereof; antiseptic ingredients such as cetylpyridinium chloride, domiphen bromide, dequalinium chloride, and combinations thereof; antitussive ingredients such as chlophedianol hydrochloride, codeine, codeine phosphate, codeine sulfate, dextromethorphan, dextromethorphan hydrobromide, diphenhydramine citrate, and diphenhydramine hydrochloride, and combinations thereof; and throat soothing agents such as honey, propolis, aloe vera, glycerine, menthol and combinations thereof.

In still another embodiment of the present invention, the pharmaceutically active ingredient may be selected from the group comprising cough suppressants. Such cough suppressants can fall into two groups: those that alter the texture or production of phlegm such as mucolytics and expectorants; and those that suppress the coughing reflex such as codeine (narcotic cough suppressants), antihistamines, dextromethorphan and isoproterenol (non-narcotic cough suppressants). In some embodiments, ingredients from either or both groups can be included.

In yet another embodiment of the present invention, the pharmaceutically active ingredient may be selected from the group comprising antitussives, such as codeine, dextromethorphan, dextrorphan, diphenhydramine, hydrocodone, noscapine, oxycodone, pentoxyverine and combinations thereof; antihistamines, such as acrivastine, azatadine, brompheniramine, chlorphen[ir]amine, clemastine, cyproheptadine, dexbrompheniramine, dimenhydrinate, diphenhydramine, doxylamine, hydroxyzine, meclizine, phenindamine, phenyltoloxamine, promethazine, pyrilamine, tripelennamine, triprolidine and combinations thereof; non-sedating antihistamines such as astemizole, cetirizine, ebastine, fexofenadine, loratidine, terfenadine, and combinations thereof; expectorants such as ammonium chloride, guaifenesin, ipecac fluid extract, potassium iodide and combinations thereof; mucolytics, such as acetylcycsteine, ambroxol, bromhexine and combinations thereof; analgesic, antipyretic and anti-inflammatory agents, such as acetaminophen, aspirin, diclofenac, diflunisal, etodolac, fenoprofen, flurbiprofen, ibuprofen, ketoprofen, ketorolac, nabumetone, naproxen, piroxicam, caffeine and mixtures thereof; local anesthetics, such as lidocaine, benzocaine, phenol, dyclonine, benzonotate and mixtures thereof; nasal decongestants, such as phenylpropanolamine, pseudoephedrine, ephedrine, phenylephrine, oxymetazoline, and combinations thereof; ingredients that provide a perception of nasal clearing such as menthol, camphor, borneol, ephedrine, eucalyptus oil, peppermint oil, methyl salicylate, bornyl acetate, lavender oil, wasabi extracts, horseradish extracts, odoriferous essential oils, extracts from woods, gums, flowers and other botanicals, resins, animal secretions, and synthetic aromatic materials.

Thus, in a preferred embodiment, the pharmaceutically active ingredient is selected from the group comprising anti-tartar agents, anti-microbial agents, anti-inflammatory agents, upper respiratory agents, vitamins, antacids, H2-antagonists, analgesics, anti-diarrheals, cough suppressants and antitussives. In another preferred embodiment of the

14

present invention, the pharmaceutically active ingredient is paracetamol.

In yet another embodiment of the present invention, the active ingredient is a cosmetic active ingredient. The cosmetic active ingredient may be selected from the group consisting of vitamins, vegetable or synthetic oils, fatty alcohols, UV-absorbing agents, and mixtures thereof.

In one embodiment of the present invention, the cosmetic active ingredient is selected from vitamins, such as vitamin A, vitamin B1, vitamin B6, vitamin B12, vitamin B2, vitamin B6, vitamin D, vitamin E, i.e. tocopheroles, vitamin K, thiamine, riboflavin, biotin, folic acid, niacin and pantothenic acid.

In another embodiment of the present invention, the cosmetic active ingredient is selected from vegetable or synthetic oils, e.g., jojoba oil, aloe vera oil, almond oil, amaranth oil, apricot kernel oil, argan oil, avocado oil, açai oil, babassu oil, baobab oil, black cumin oil, black currant seed oil, borageseed oil, brazilnut oil, broccoliseed oil, calendula oil, camelina oil, castor oil, chia oil, chilean hazelnut oil, coconut oil, corn oil, cottonseed oil, crambe oil, cranberry seed oil, evening primrose oil, grapeseed oil, groundnut oil, hazelnut oil, hemp oil, kukuinut oil, laurel oil, linseed oil, MCT oil, macadamianut oil, manketti oil, marula oil, meadowfoam seed oil, milk thistle oil, moringa oil, mustardseed oil, olive oil, olus oil, palm oil, palmkernel oil, palmolein, palmstearin, passionfruit seed oil, peach kernel oil, pecan nut oil, perilla oil, pistachionut oil, plum kernel oil, pomegranate oil, poppyseed oil, pumpkinseed oil, raspberry seed oil, rapeseed oil, rice bran oil, rose hip oil, sacha inchi oil, safflower oil, seabuckthorn pulp oil, sesame oil, soybean oil, St. John's wort oil, sunflower oil, tamanu oil, tigernut oil, walnut oil, wheat germ oil, paraffine, petroleum jelly, mineral oil, white mineral oil and mixtures thereof.

In one embodiment of the present invention, the cosmetic active ingredient is a fragrance. Fragrances are preferably alcohols, aldehydes and/or ketones having a molecular weight of at least about 100 g/mol and which are useful in imparting an odour, fragrance, essence, or scent either alone or in combination with other fragrances. For example, the fragrance can be selected from the group comprising 2,4-dimethyl-3-cyclohexene-1-methanol (floralol), 2,4-dimethyl cyclohexane methanol (dihydro floralol), 5,6-dimethyl-1-methylethenylbicyclo[2.2.1]hept-5-ene-2-methanol (arbozol), $\alpha,\alpha,$-4-trimethyl-3-cyclohexen-1-methanol (a-terpineol), 2,4,6-trimethyl-3-cyclohexene-1-methanol (isocyclo geraniol), 4-(1-methylethyl)cyclohexane methanol (mayol), $\alpha$-3,3-trimethyl-2-norborane methanol, 1,1-dimethyl-1-(4-methylcyclohex-3-enyl)methanol, 2-phenylethanol, 2-cyclohexyl ethanol, 2-(o-methylphenyl)-ethanol, 2-(m-methylphenyl)ethanol, 2-(p-methylphenyl)ethanol, 6,6-dimethylbicyclo-[3.1.1]hept-2-ene-2-ethanol (nopol), 2-(4-methylphenoxy)-ethanol, 3,3-dimethyl-$\Delta^2$-$\beta$-norbornane ethanol (patchomint), 2-methyl-2-cyclohexylethanol, 1-(4-isopropylcyclohexyl)-ethanol, 1-phenylethanol, 1,1-dimethyl-2-phenylethanol, 1,1-dimethyl-2-(4-methyl-phenyl)ethanol, 1-phenylpropanol, 3-phenylpropanol, 2-phenylpropanol (Hydrotropic Alcohol), 2-(cyclododecyl)propan-1-ol (hydroxy-ambran), 2,2-dimethyl-3-(3-methylphenyl)-propan-1-ol (majantol), 2-methyl-3-phenylpropanol, 3-phenyl-2-propen-1-ol (cinnamyl alcohol), 2-methyl-3-phenyl-2-propen-1-ol (methylcinnamyl alcohol), $\alpha$-n-pentyl-3-phenyl-2-propen-1-ol ($\alpha$-amyl-cinnamyl alcohol), ethyl-3-hydroxy-3-phenyl propionate, 2-(4-methylphenyl)-2-propanol, 3-(4-methylcyclohex-3-ene)butanol, 2-methyl-4-(2,2,3-trimethyl-3-cyclopenten-1-yl)butanol, 2-ethyl-4-(2,2,3-trimethyl-cyclopent-3-enyl)-2-buten-1-ol, 3-methyl-2-buten-1-ol (prenol), 2-methyl-4-(2,2,3-trimethyl-3-cyclopenten-1-yl)-2-buten-1-ol, ethyl 3-hydroxybutyrate, 4-phenyl-3-buten-2-ol, 2-methyl-4-phenylbutan-2-ol, 4-(4-hydroxyphenyl)butan-2-one, 4-(4-hydroxy-3-methoxyphenyl)-butan-2-one, 3-methyl-pentanol, 3-methyl-3-penten-1-ol, 1-(2-propenyl)cyclopentan-1-ol (plinol), 2-methyl-4-phenylpentanol (pamplefleur), 3-methyl-5-phenylpentanol (phenoxanol), 2-methyl-5-phenylpentanol, 2-methyl-5-(2,3-dimethyltricyclo[2.2.1.0.sup.(2,6)]hept-3-yl)-2-penten-1-ol (santalol), 4-methyl-1-phenyl-2-pentanol, 5-(2,2,3-trimethyl-3-cyclopentenyl)-3-methylpentan-2-ol (sandalore), (1-methyl-bicyclo[2.1.1]hepten-2-yl)-2-methylpent-1-en-3-ol, 3-methyl-1-phenylpentan-3-ol, 1,2-dimethyl-3-(1-methylethenyl)cyclopentan-1-ol, 2-isopropyl-5-methyl-2-hexenol, cis-3-hexen-1-ol, trans-2-hexen-1-ol, 2-isoproenyl-4-methyl-4-hexen-1-ol (lavandulol), 2-ethyl-2-prenyl-3-hexenol, 1-hydroxymethyl-4-isopropenyl-1-cyclohexene (dihydrocuminyl alcohol), 1-methyl-4-isopropenylcyclohex-6-en-2-ol (carvenol), 6-methyl-3-isopropenylcyclohexan-1-ol (dihydrocarveol), 1-methyl-4-iso-propenylcyclohexan-3-ol, 4-isopropyl-1-methylcyclohexan-3-ol, 4-tert-butylcyclo-hexanol, 2-tert-butylcyclohexanol, 2-tert-butyl-4-methylcyclohexanol (rootanol), 4-isopropyl-cyclohexanol, 4-methyl-1-(1-methylethyl)-3-cyclohexen-1-ol, 2-(5,6,6-trimethyl-2-norbornyl)cyclohexanol, isobornylcyclohexanol, 3,3,5-trimethylcyclohexanol, 1-methyl-4-isopropylcyclohexan-3-ol, 1-methyl-4-isopropylcyclohexan-8-ol (dihydroterpineol), 1,2-dimethyl-3-(1-methylethyl)cyclohexan-1-ol, heptanol, 2,4-dimethylheptan-1-ol, 6-heptyl-5-hepten-2-ol (isolinalool), 2,4-dimethyl-2,6-heptandienol, 6,6-dimethyl-2-oxymethyl-bicyclo[3.1.1]hept-2-ene (myrtenol), 4-methyl-2,4-heptadien-1-ol, 3,4,5,6,6-pentamethyl-2-heptanol, 3,6-dimethyl-3-vinyl-5-hepten-2-ol, 6,6-dimethyl-3-hydroxy-2-methylenebicyclo[3.1.1]heptane, 1,7,7-trimethylbicyclo[2.2.1]heptan-2-ol, 2,6-dimethylheptan-2-ol (dimetol), 2,6,6-trimethylbicyclo[1,3.3]heptan-2-ol, octanol, 2-octenol, 2-methyloctan-2-ol, 2-methyl-6-methylene-7-octen-2-ol (myrcenol), 7-methyloctan-1-ol, 3,7-dimethyl-6-octenol, 3,7-dimethyl-7-octenol, 3,7-dimethyl-6-octen-1-ol (citronellol), 3,7-dimethyl-2,6-octadien-1-ol (geraniol), 3,7-dimethyl-2,6-octadien-1-ol (nerol), 3,7-dimethyl-7-methoxyoctan-2-ol (osyrol), 3,7-dimethyl-1,6-octadien-3-ol (linalool), 3,7-dimethyloctan-1-ol (pelargol), 3,7-dimethyloctan-3-ol (tetrahydrolinalool), 2,4-octadien-1-ol, 3,7-dimethyl-6-octen-3-ol (dihydrolinalool), 2,6-dimethyl-7-octen-2-ol (dihydromyrcenol), 2,6-dimethyl-5,7-octadien-2-ol, 4,7-dimethyl-4-vinyl-6-octen-3-ol, 3-methyloctan-3-ol, 2,6-dimethyloctan-2-ol, 2,6-dimethyloctan-3-ol, 3,6-dimethyloctan-3-ol, 2,6-dimethyl-7-octen-2-ol, 2,6-dimethyl-3,5-octadien-2-ol (muguol), 3-methyl-1-octen-3-ol, 7-hydroxy-3,7-dimethyloctanal, 3-nonanol, 2,6-nonadien-1-ol, cis-6-nonen-1-ol, 6,8-dimethylnonan-2-ol, 3-(hydroxymethyl)-2-nonanone, 2-nonen-1-ol, 2,4-nonadien-1-ol, 3,7-dimethyl-1,6-nonadien-3-ol, decanol, 9-decenol, 2-benzyl-M-dioxa-5-ol, 2-decen-1-ol, 2,4-decadien-1-ol, 4-methyl-3-decen-5-ol, 3,7,9-trimethyl-1,6-decadien-3-ol (isobutyl linalool), undecanol, 2-undecen-1-ol, 10-undecen-1-ol, 2-dodecen-1-ol, 2,4-dodecadien-1-ol, 2,7,11-trimethyl-2,6,10-dodecatrien-1-ol (farnesol), 3,7,11-trimethyl-1,6,10,-dodecatrien-3-ol (nerolidol), 3,7,11,15-tetramethylhexadec-2-en-1-ol (phytol), 3,7,11,15-tetramethylhexadec-1-en-3-ol (iso phytol), benzyl alcohol, p-methoxy benzyl alcohol (anisyl alcohol), para-cymen-7-ol (cuminyl alcohol), 4-methyl benzyl alcohol, 3,4-methylenedioxy benzyl alcohol, methyl salicylate, benzyl salicylate, cis-3-hexenyl salicylate, n-pentyl salicylate, 2-phenylethyl salicylate, n-hexyl salicylate, 2-methyl-5-isopropylphenol, 4-ethyl-2-methoxyphenol, 4-allyl-2-methoxyphenol (eugenol), 2-methoxy-4-(1-propenyl)phenol (isoeugenol), 4-allyl-2,6-dimethoxy-phenol, 4-tert-butylphenol, 2-ethoxy-4-methylphenol, 2-methyl-4-vinylphenol, 2-isopropyl-5-methylphenol (thymol), pentyl-ortho-hydroxy benzoate, ethyl 2-hydroxybenzoate, methyl 2,4-dihydroxy-3,6-dimethylbenzoate, 3-hydroxy-5-methoxy-1-methylbenzene, 2-tert-butyl-4-methyl-1-hydroxybenzene, 1-ethoxy-2-hydroxy-4-propenylbenzene, 4-hydroxytoluene, 4-hydroxy-3-methoxybenzaldehyde, 2-ethoxy-4-hydroxybenzaldehyde, decahydro-2-naphthol, 2,5,5-trimethyl-octahydro-2-naphthol, 1,3,3-trimethyl-2-norbornanol (fenchol), 3a,4,5,6,7,7a-hexahydro-2,4-dimethyl-4,7-methano-1H-inden-5-ol, 3a,4,5,6,7,7a-hexahydro-3,4-dimethyl-4,7-methano-1H-inden-5-ol, 2-methyl-2-vinyl-5-(1-hydroxy-1-methylethyl)tetra-hydrofuran, β-caryophyllene alcohol, vanillin, ethyl vanillin, cinnamaldehyde, benzaldehyde, phenyl acetaldehyde, heptylaldehyde, octylaldehyde, decylaldehyde, undecylaldehyde, undecylenic aldehyde, dodecylaldehyde, tridecylaldehyde, methylnonyl aldehyde, didecylaldehyde, anisaldehyde, citronellal, citronellyloxyaldehyde, cyclamen aldehyde, α-hexyl cinnamaldehyde, hydroxycitronellal, α-methyl cinnamaldehyde, methylnonyl acetaldehyde, propylphenyl aldehyde, citral, perilla aldehyde, tolylaldehyde, tolylacetaldehyde, cuminaldehyde, LILIAL®, salicyl aldehyde, α-amylcinnamaldehyde and heliotropin and mixtures thereof.

In still another embodiment of the present invention, the cosmetic active ingredient is selected from fatty alcohols, preferably straight-chain fatty alcohols having between 8 and 22 carbon atoms, for example, cetyl alcohol, myristyl alcohol, stearyl alcohol, behenyl alcohol, or mixtures thereof.

In yet another embodiment of the present invention, the cosmetic active ingredient is selected from UV-absorbing compounds, such as titanium dioxide, zinc oxide, benzophenone derivatives, bis-ethylhexyloxyphenol methoxyphenyl triazine (Tinosorb S), drometrizole trisiloxane (Meroxyl XL), terephthalylidene dicamphorsulfonic acid (Mexoryl SX), ethylhexyl triazone (Uvinul T 150), butyl methoxydibenzoylmethane (Avobenzone), diethylamino hydroxybenzoyl hexyl benzoate (Uvinul A Plus), diethylhexyl butamido triazone (Iscotrizinol), phenylbenzimidazole sulfonic acid (Enzulisol) and mixtures thereof.

In another embodiment of the present invention, the active ingredient is a nutraceutical active ingredient.

In one embodiment of the present invention, the nutraceutical active ingredient is selected from vitamins, such as vitamin A, vitamin B1, vitamin B6, vitamin B12, vitamin B2, vitamin B6, vitamin C, vitamin D, vitamin E, i.e. tocopheroles, vitamin K, thiamine, riboflavin, biotin, folic acid, niacin, pantothenic acid, Q10, alpha lipoic acid, dihydrolipoic acid, curcumin, xanthophylls, beta cryptoxanthin, lycopene, lutein, zeaxanthin, astaxanthin, beta-carotene, carotenes, mixed carotenoids, polyphenols, flavonoids and mixtures thereof; minerals, such as sodium, potassium, calcium, magnesium, sulphur, chlorine, choline, manganese, iron, zinc, copper, fluorine, molybdenum, iodine, cobalt, chromium, selenium, phosphorous, and mixtures thereof; and/or phytochemicals, such as carotenoids, chlorophyll, chlorophyllin, fibre, flavanoids, anthocyanins, cyaniding, delphinidin, malvidin, pelargonidin, peonidin, petunidin, flavanols, catechin, epicatechin, epigallocatechin, epigallocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof. Further examples of nutraceutical active ingredients suitable for use in the present invention are set forth in US 2003/0157213 A1, US 2003/0206993 A1 and US 2003/0099741 A1.

In a preferred embodiment of the present invention, the nutraceutical active ingredient is selected from the group comprising ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

In yet another embodiment of the present invention, the nutraceutical active ingredient is a phytochemical preferably selected from the group comprising carotenoids, chlorophyll, chlorophyllin, fibre, flavanoids, anthocyanins, cyanidin, delphinidin, malvidin, pelargonidin, peonidin, petunidin, flavanols, catechin, epicatechin, epigallocatechin, epigallocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof.

In still another embodiment of the present invention, the nutraceutical active ingredient is selected from the group comprising L-carnitine, choline, coenzyme Q10, alpha-lipoic acid, omega-3 - fatty acids, pepsin, phytase, trypsin, lipases, proteases, cellulases, and combinations thereof.

In one embodiment of the present invention, the nutraceutical active ingredient is a flavoring agent. Suitable flavouring agents include the compounds described as fragrances hereinabove.

In still another embodiment of the present invention, the nutraceutical active ingredient is an essential oil. The essential oil may be a herbal extract and/or fruit extract. Essential oils are generally extracts of aromatic plants, plant parts, fruit or fruit parts that can be used medicinally or for flavouring. Suitable herbal extracts and/or fruit extracts can be used singly or in various mixtures.

Examples of suitable essential oils are alfalfa oil, allspice oil, almond oil, ambrette (seed) oil, angelica root oil, angelica seed oil, angelica stem oil, angostura (cusparia bark) oil, anise oil, asafetida oil, balm (lemon balm) oil, balsam of peru, basil oil, bay leaves oil, bay (myrcia) oil, bergamot (bergamot orange) oil, bitter almond oil (free from prussic acid), bois de rose oil, cacao oil, camomile (chamomile) flowers (Hungarian) oil, camomile (chamomile) flowers (Roman or English) oil, cananga oil, capsicum oil, caraway oil, cardamom seed (cardamon) oil, carob bean oil, carrot oil, cascarilla bark oil, cassia bark (Chinese) oil, cassia bark (Padang or Batavia) oil, cassia bark (Saigon) oil, celery seed oil, wild cherry bark oil, chervil oil, chicory oil, cinnamon bark (Ceylon) oil, cinnamon bark (Chinese) oil, cinnamon bark (Saigon) oil, cinnamon leaf (Ceylon) oil, cinnamon leaf (Chinese) oil, cinnamon leaf (Saigon) oil, citronella oil, citrus peels oil, clary (clary sage) oil, clover oil, coca oil (decocainized), coffee oil, cola nut oil, coriander oil, cumin oil, curacao orange peel (orange, bitter peel) oil, cusparia bark oil, dandelion oil, dandelion root oil, dog grass (quackgrass, triticum) oil, elder flowers oil, estragole oil, estragon (tarragon) oil, fennel oil, fenugreek oil, galanga (galangal) oil, geranium oil, geranium (East Indian) oil, geranium rose oil, ginger oil, grapefruit oil, guava oil, hickory bark oil, horehound (hoarhound) oil, hops oil, horsemint oil, hyssop oil, immortelle oil, jasmine oil, juniper (berries) oil, kola nut oil, laurel berries oil, laurel leaves oil, lavender oil, lavender (spike) oil, lavandin oil, lemon oil, lemon balm oil, lemon grass oil, lemon peel oil, lime oil, linden flowers oil, locust bean oil, lupulin oil, mace oil, mandarin oil, marjoram oil, mate oil, melissa oil, menthol oil, menthyl acetate oil, molasses (extract), mustard oil, naringin oil, neroli oil, bigarade oil, nutmeg oil, onion oil, bitter orange flowers oil, bitter orange peel oil, orange leaf oil, sweet orange oil, sweet orange flowers oil, sweet orange peel oil, origanum oil, palmarosa oil, paprika oil, parsley oil, black pepper oil, white pepper oil, peppermint oil, peruvian balsam oil, petitgrain oil, petitgrain lemon oil, petitgrain mandarin or tangerine oil, pimenta oil, pimenta leaf oil, pipsissewa leaves oil, pomegranate oil, prickly ash bark oil, rose absolute oil, rose oil, rose buds oil, rose flowers oil, rose fruit (hips) oil, rose geranium oil, rose leaves oil, rosemary oil, saffron oil, sage oil, sage (Greek) oil, sage (Spanish) oil, St. John's wort oil, summer savory oil, winter savory oil, Schinus Molle oil, sloe berries (blackthorn berries) oil, spearmint oil, spike lavender oil, tamarind oil, tangerine oil, tarragon oil, tea oil, thyme oil, thyme (white) oil, thyme (wild or creeping) oil, triticum oil, tuberose oil, turmeric oil, vanilla oil, violet flowers oil, violet leaves oil, violet leaves absolute oil, wild cherry bark oil, ylang-ylang oil, zedoary bark oil, echinacea oil, goldenseal oil, calendula oil, kava kava oil, aloe oil, blood root oil, grapefruit seed extract oil, black cohosh oil, ginseng oil, guarana oil, cranberry oil, ginko biloba oil, evening primrose oil oil, yohimbe bark oil, green tea oil, ma huang oil, maca oil, bilberry oil, lutein oil, and ginger oil.

In another embodiment of the present invention, the active ingredient is a biocide. The biocide may be selected from the group comprising glutardialdehyde (GDA), isothiazlinones such as 2-methyl-2H-isothiazol-3-one (MIT), 5-chloro-2-methyl-2H-isothiazol-3-one (CMIT), benzisothiazolinone (BIT), octyl-isothiazolinone (OIT), 4,5-dichloro-2-n-octyl-4-isothiazol-3-one (DCOIT), 2-bromo-2-nitro-1,3-propandiol (bronopol), 2,2-dibromo-3-nitrilopropionamide (DBNPA), o-phenylphenol (OPP) and its salts, phenoxyethanol, formaldehyde, ethyleneglycolhemiformals, 1-(3-chloroallyl)-3,5,7-Triaza-1-azoniaadamantane chloride, tetrakishydroxymethyl phosphonium sulfate (THPS), 4,4-dimethyloxazolidine (DMO), hexahydro-1,3,5-tris(2-hydroxyethyl)-s-triazine, hexahydro-1,3,5-triethyl-s-triazine (HTT), tetrahydro-3,5-dimethyl-2H-1,3,5-thiadiazine-2-thione (DAZOMET), 3-iodo-2-propynyl butyl carbamate (IPBC), 5-chloro-2-(2,4-dichlorophenoxy)-phenol (triclosan); and derivatives, salts and mixtures thereof. In still another embodiment, the biocide may be selected from preservatives including sodium pyrosulphite, butylhydroxytoluene, butylated hydroxyanisole, parabenes, benzalkonium chloride, chlorbutanol, benzyl alcohol, beta-phenylethyl alcohol, cetylpyridinium chloride, citric acid, tartaric acid, lactic acid, malic acid, acetic acid, benzoic acid, and sorbic acid and their salts; and chelating agents, such as EDTA; and gallates, such as propyl gallate.

In yet another embodiment of the present invention, the active ingredient is a pesticide. The pesticide may be selected from the group comprising any known herbicide, insecticide, insect growth regulator, nematicide, termiticide, molluscicide, piscicide, avicide, rodenticide, predacide, bactericide, insect repellent, animal repellent, antimicrobial, fungicide, disinfectant (antimicrobial), and sanitizer known to the skilled person. It is to be noted that the preserving agent may be any such compound known to the skilled person. For example, preserving agents may include, but are not limited to, phenoxyethanol, ethylhexylglycerin, parabens such as methyl paraben, ethyl paraben, propyl paraben, butyl paraben and mixtures thereof, benzalkonium chloride, chlorbutanol, benzyl alcohol, cetylpyridinium chloride, tartaric acid, lactic acid, malic acid, acetic acid, benzoic acid, sodium benzoate, sorbic acid, potassium sorbatem, and mixtures thereof.

In still another embodiment of the present invention, the active ingredient is a wetting agent. Wetting agents are substances lowering the surface tension of a liquid and allowing it to spread more easily. In a preferred embodiment, the wetting agent is selected from the group comprising ammonium lauryl sulfate, sodium lauryl sulfate, sodium laureth sulfate, sodium myreth sulfate, docusate, perfluorooctanesulfonate (PFOS), perfluorobutanesulfonate, alkyl-aryl ether phosphates, alkyl ether phosphates, sodium lauroyl sarcosinate, cetrimonium bromide (CTAB), cetylpyridinium chloride (CPC), benzalkonium chloride (BAC), benzethonium chloride (BZT), dimethyldioctadecylammonium chloride, dioctadecyldimethylammonium bromide (DODAB), CHAPS (3-[(3-cholamidopropyl)dimethylammonio]-1-propanesulfonate), cocamidopropyl hydroxysultaine, betaines such as cocamidopropyl betaine, phospholipids such as phosphatidylserine, phosphatidylethanolamine, phosphatidylcholine, and sphingomyelins, fatty alcohol ethoxylates, narrow-range ethoxylate, octaethylene glycol monododecyl ether, pentaethylene glycol monododecyl ether, alkylphenol ethoxylates (apes), nonoxynols, and triton X-100.

In yet another embodiment of the present invention, the active ingredient is a UV protecting agent. Preferably, the UV protecting agent is selected from titanium dioxide, zinc oxide, benzophenone derivatives, bis-ethylhexyloxyphenol methoxyphenyl triazine (Tinosorb S), drometrizole trisiloxane (Meroxyl XL), terephthalylidene dicamphorsulfonic acid (Mexoryl SX), ethylhexyl triazone (Uvinul T 150), butyl methoxydibenzoylmethane (Avobenzone), diethylamino hydroxybenzoyl hexyl benzoate (Uvinul A Plus), diethylhexyl butamido triazone (Iscotrizinol), phenylbenzimidazole sulfonic acid (Enzulisol) and mixtures thereof.

In another embodiment of the present invention, the active ingredient is a scavenger. Scavengers are chemical substances added to a mixture in order to remove or de-activate impurities and unwanted reaction products, for example oxygen, to make sure that they will not cause any unfavorable reactions. In a preferred embodiment, the scavenger is selected from the group comprising hydrazine, ascorbic acid, tocopherol, naringenin, organotin compounds, iron powder, and glutathione.

According to a preferred embodiment, the active ingredient is a macromolecular active ingredient or an inactive precursor thereof. For the purposes of the present invention, a macromolecular active ingredient is defined as a molecule having a molecular weight of more than 500 g/mol, preferably more than 800 g/mol, more preferably more than 1500 g/mol, and most preferably more than 10000 g/mol. It is appreciated that, e.g., natural or synthetic polymers, proteins, peptides, mucoproteins, enzymes, nucleic acids and polysaccharides are considered macromolecules for the purposes of the present invention.

The macromolecular active ingredient may be a macromolecular pharmaceutically active ingredient, a macromolecular cosmetic active ingredient, a macromolecular nutraceutical active ingredient, a macromolecular biocide, a macromolecular pesticide, a macromolecular wetting agent, a macromolecular UV-protecting agent, a macromolecular scavenger, a macromolecular pro-drug, a macromolecular precursor of a cosmetic active ingredient, a macromolecular precursor of a nutraceutical active ingredient, a macromolecular precursor of a biocide, a macromolecular precursor of a pesticide, a macromolecular precursor of a wetting agent, a macromolecular precursor of a UV-protecting agent, or a precursor of a macromolecular scavenger.

Macromolecular pharmaceutically active ingredients may include the macromolecular pharmaceutically active ingredients as defined hereinabove, prokaryotic proteins, eukaryotic proteins (e.g., mammalian, plant), chimeric proteins, viral proteins and peptides, antibodies, hormones, growth factors, proteases, extra-cellular matrix proteins (e.g., collagen), enzymes, the infectious bursal disease virus viral protein VPII, Human interferon beta, Human clotting factor, Human factor X, Human lysosomal enzyme, Human glucocerebrosidase, human alpha galactosidase, and acetyl choline esterase and high mannose proteins, e.g., Human Cox-2, Human EGF, Human uterine tissue plasminogen activator (tPA), Human DNase I, recombinant g 120, Human tissue plasminogen activator, Human thyro globulin (hTG), Human CD4 and Human plasminogen; peginterferon alfa-2a, peginterferon alfa-2b, pegaspargase, colestipol, inulin, icodextrin, hydroxypropyl cellulose, iron dextran, quinidine, pramlintide, tolevamer, cholestyramine, cellobiose, maltotetraose, trehalose-6-phosphate, cellotetraose, maltose, paraformaldehyde, crofelemer, SP1049C, PEG-uricase, carbopol 974P, PRO 2000, LJP 1082, MAXY-G34, IT-101, XMT-1001, sugammadex, endostatin, tyloxapol, pegsunercept, sodium cellulose phosphate, nonoxynol-9, polidocanol, povidone, povidone-iodine, peginesatide, certolizumab pegol, ferric carboxymaltose, naloxegol, hydroxyethyl starch, methoxy polyethylene glycol-epoetin beta, pentastarch, peginterferon beta-1a, pegloticase, dextran, patiromer, polyethylene glycol, polycarbophil, ferumoxsil, ferumoxides, simethicone, karaya gum, tragacanth, carob, carboxymethylcellulose, polyvinyl alcohol, povidone, povidone iodine, polysorbate 80, dimethicone, hypromellose, polyethylene glycol 400, turpentine, pectin, polyethylene glycol 300, rosin, polysorbate 20, tolu balsam, methylcellulose, poliglusam, poloxamer 407, poloxamer 188,gleptoferron, poloxalene, balsam of peru, insulin peglispro, drometrizole trisiloxane, starch, corn, hydroxyethyl cellulose, astodrimer, 2-octyl cyanoacrylate, trehalose, enbucrilate, pegamotecan, sizofiran, guar gum, sodium apolate, lentinan, dextranomer, oxidized cellulose, nonacog beta pegol, carbomer homopolymer type C, cyclomethicone, hydroxyethyl ethylcellulose, microcrystalline cellulose, amber, antihemophilic factor (recombinant), pegylated, calcium polycarbophil, triglyme, triethylene glycol, betadex,calaspargase pegol, pegargiminase, phenyl trimethicone, dextrin 2-sulfate, peginterferon lambda-1a, polyacrylamide (crosslinked; 2 mole percent bisacrylamide), polydioxanone, polydextrose, prm-151, amylopectin, etirinotecan pegol, firtecan pegol, poly(lactic acid), somatropin pegol, amylose, hydroxypropyl betadex, lulizumab pegol, pegfilgrastim, endostar, brexanolone, lipegfilgrastim, ocrylate, bempegaldesleukin, pegilodecakin, pegbelfermin, silk sericin or combinations thereof. Suitable macromolecular inactive precursors of pharmaceutically active ingredients include the macromolecular pharmaceutically active ingredients as defined hereinabove, which are attached (e.g., covalently or non-covalently attached) to a macromolecule and can be released upon exposure to a chemical stimulus, e.g., an acid or an enzyme, or upon exposure to visible or UV light.

Macromolecular nutraceutical active ingredients, e.g., for use in a foodstuff or a feedstuff, include the macromolecular nutraceutical active ingredients as defined hereinabove, food and/or feed proteins, enzymes, α-lactalbumin, beta-lactoglobulin, casein, conalbumin, lysozyme, ovoglobulin, ovomucoid, avidin, protamines, histones, plant-derived proteins such as edestin from hemp, amandin from almonds, concanavalin A and B and canavalin from jack beans, cereal seed proteins such as gliadin, soy protein, wheat protein, nut proteins, seed proteins and fruit proteins. Enzymes can include coenzyme Q10, pepsin, phytase, trypsin, lipases, proteases, cellulases, lactase and combinations thereof. Food and/or feed proteins are those found in high quality protein feed such as soy bean meal, bean meal, cottonseed meal, feather meal, blood meal, silages, meat and bone meal, sunflower seed meal, canola meal, peanut meal, safflower meal, linseed meal, sesame meal, early bloom legumes, fish products, milk products, poultry products, hays, corn, wheat, alfalfa, barley, milo, sorghum and mixtures thereof. Suitable macromolecular inactive precursors of nutraceutical active ingredients include the macromolecular nutraceutical active ingredients as defined hereinabove, which are attached (e.g., covalently or non-covalently attached) to a macromolecule and can be released upon exposure to a chemical stimulus, e.g., an acid or an enzyme, or upon exposure to visible or UV light.

Particularly preferred macromolecular active ingredients are bovine serum albumin and lactoferrin.

Suitable macromolecular cosmetic active ingredients, macromolecular biocides, macromolecular pesticides, macromolecular wetting agents, macromolecular UV protecting agents and macromolecular scavengers are those as defined hereinabove. Suitable macromolecular inactive precursors of cosmetic active ingredients, biocides, pesticides, wetting agents, UV protecting agents and scavengers include the cosmetic active ingredients, biocides, pesticides, wetting agents, UV protecting agents and scavengers as defined hereinabove, which are attached (e.g., covalently or non-covalently attached) to a macromolecule and can be released upon exposure to a chemical stimulus, e.g., an acid or an enzyme, or upon exposure to visible or UV light.

In a particularly preferred embodiment of the present invention, the active ingredient is a macromolecular pharmaceutically active ingredient, most preferably bovine serum albumin and lactoferrin.

**The multilayer shell**

According to the present invention the loaded surface-reacted calcium carbonate is encased with a multilayer shell. The multilayer shell is formed by depositing at least two complementary layers using layer-by-layer assembly onto the loaded surface-reacted calcium carbonate. It is to be understood that each of the "complementary layers" is formed from at least one "encapsulant". The encapsulants are selected such that complementary interactions between the encapsulants enable the consecutive deposition of the at least two complementary layers.

The present invention is not limited to any particular type of complementary layer. For example, the layers may be formed from encapsulants, which are polyelectrolytes being organic polyelectrolytes, e.g., polyacrylic acid, which dissociates in a polar solvent to yield protons and a negatively charged polyacrylate ion, neutral molecules, e.g., polysulfoxides, may be used, as well as inorganic macromolecules, such as polyphosphates, inosilicates or phyllosilicates. A prominent example of a phyllosilicate is montmorillonite, empirical formula $(Na,Ca)_{0.33}(Al,Mg)_2(Si_4O_{10})(OH)_2 \cdot nH_2O$, wherein, e.g., the sodium ions may dissociate to yield a negatively charged polyelectrolyte.

Generally, the skilled person will select the complementary layers according to the specific requirements or purposes of the multilayer shell. Examples of suitable complementary layers can be found in DE 198 12 083 A1. For example, the complementary layers may be chosen such as to allow for controlled or immediate release of the active ingredient or inactive precursor thereof contained in the microcapsule. Release of the active ingredient or inactive precursor thereof may be triggered by mechanical means, such as crushing or rupturing the particles by pressure or shear stress, dissolution or decomposition of the multilayer shell under certain conditions, e.g., by interaction with an acid, a base, a solvent, or an enzyme, or by interaction with radiation, such as UV radiation or visible light.

In one embodiment, the at least two complementary layers are formed from encapsulants independently selected from the group consisting of nanomaterials, macromolecular encapsulants and mixtures thereof, and preferably the at least two complementary layers are formed from encapsulants independently selected from the group consisting of polysaccharides, polyphenols, proteins, nucleic acids, cationic polyelectrolytes, anionic polyelectrolytes, nanomaterials and mixtures thereof, preferably selected from the group consisting of polyphenols and proteins.

In a preferred embodiment of the present invention, at least one encapsulant is selected from the group consisting of tannic acid, polyacrylic acid, poly(4-vinylpyridine), poly(glutamic acid), poly(lactic acid), chondroitin sulphate, dextran sulphate, poly(styrene sulfonate), sodium alginate, hyaluronic acid, polyphosphoric acid, polyvinyl sulfonic acid, polyvinyl phosphonic acid, polysulfates, dextrans, gum arabic, pectin, anionic cellulose derivatives, preferably carboxymethyl cellulose, pepsin, poly(dimethylammonium chloride), poly(ethylene oxide), poly(allylamine hydrochloride), poly(lysine), poly(lactic-co-glycolic acid), poly(arginine), protamine sulphate, polyethyleneimine, chitosan, glycol chitosan, montmorillonite, polyvinylamine, amine-containing polymers, preferably polyamidoamine-epichlorohydrin or poly[2-(dimethylamino)ethyl methacrylate], bovine serum albumin, corresponding salts thereof, metal nanoparticles, metal oxide nanoparticles, carbon nanotubes, graphene, graphene oxide, nanoclays, and mixtures thereof. More preferably, the complementary layers are formed from macromolecular encapsulants independently selected from the group consisting of tannic acid, poly(styrene sulfonate), poly(allylamine hydrochloride), bovine serum albumin, pepsin, corresponding salts thereof, and mixtures thereof, and most preferably the complementary layers are formed from macromolecular encapsulants independently selected from the group consisting of tannic acid, pepsin, and corresponding salts thereof. The skilled person will appreciate that the expression "corresponding salts thereof" refers only to salts which are to be known in the art as being indeed derivable from the afore-mentioned compounds. In other words, said expression refers only to salts of the afore-mentioned compounds, which are actually existing and available to skilled person. Examples of said corresponding salts are acid salts, nucleic acid salts, salts of anionic polyelectrolytes, or salts of cationic polyelectrolytes.

According to one embodiment of the present invention, at least one encapsulant is a polysaccharide. A "polysaccharide" (or "glycan") is understood to refer to a compound consisting of at least three, more preferably at least 10 monosaccharides, or non-ionic derivatives thereof, linked glycosidically. Polysaccharides suitable for use in the present invention include dextrans, gum Arabic, pectin, chitosan, glycol chitosan and mixtures thereof.

According to another embodiment of the present invention, at least one encapsulant is a polyphenol. A "polyphenol" is understood to be a macromolecular material comprising multiple phenol structural units, e.g., derived from gallic acid, catechins, epicatechins, anthocyanidins, flavonoids, isoflavonoids or neoflavonoids. Polyphenols suitable for use in the present invention include tannic acid, elagitannin, tannins, proanthocyanidins, and mixtures thereof. A particularly preferred polyphenol for use in the present invention is tannic acid or a corresponding salt thereof.

According to yet another embodiment of the present invention, the at least one encapsulant is a protein. A "protein" is understood to be a synthetic or naturally occurring macromolecular polypeptide composed of amino acids linked together by an amide bond. Proteins suitable for use in the present invention include poly(lysine), poly(arginine), poly(glutamic acid), pepsin, casein, bovine serum albumin (BSA), ovalbumin, lysozyme, chymotrypsin, β-lactoglobulin, trypsin, ubiquitin, lectin, lactoperoxidase, myosin, κ-casein, β-casein, α-lactalbumin, elastin, ferritin and mixtures thereof. Preferred proteins include those that are stable or essentially stable under gastric conditions, e.g., pepsin and bovine serum albumin.

According to still another embodiment of the present invention, the at least one encapsulant is a nucleic acid. A "nucleic acid" is understood to be a macromolecule composed of nucleotide units, which are hydrolysable into certain pyrimidine or purine bases (usually adenine, cytosine, guanine, thymine, uracil), D-ribose or 2-deoxy-D-ribose and phosphoric acid. Suitable nucleic acids include ribonucleic acids and desoxyribonucleic acids.

According to one embodiment of the present invention, at least one encapsulant is an anionic polyelectrolyte. Anionic polyelectrolytes may include organic and inorganic macromolecules, which contain net anionic charges or are capable of dissociating into cations and polyanions bearing a net anionic charge. Preferably, the anionic polyelectrolyte is selected from the group consisting of polyacrylic acid, poly(glutamic acid), poly(lactic acid), poly(lactic-co-glycolic acid), chondroitin sulphate, dextran sulphate, poly(styrene sulfonate), alginic acid, sodium alginate, polyphosphoric acid, polyvinyl sulfonic acid, polyvinyl phosphonic acid, polysulfates, anionic cellulose derivatives, preferably carboxymethyl cellulose, corresponding salts thereof, and mixtures thereof. Preferred anionic polyelectrolytes may include poly(styrene sulfonate), corresponding salts thereof, and mixtures thereof.

According to another embodiment of the present invention, at least one encapsulant is a cationic polyelectrolyte. Cationic polyelectrolytes may include organic and inorganic macromolecules, which contain net cationic charges or are capable of dissociating into anions and polycations bearing a net positive charge, or comprise Lewis- or Brønsted-basic moieties capable of forming cationic moieties by binding protons or metal ions. Preferably, the cationic polyelectrolyte is selected from the group consisting of poly(dimethylammonium chloride), poly(allylamine hydrochloride), poly(lysine), poly(arginine), protamine sulphate, polyethyleneimine, poly(4-vinylpyridine), polyvinyl amine, amine-containing polymers, preferably polyamidoamine-epichlorohydrin or poly[2-(dimethylamino)ethyl methacrylate], corresponding salts thereof, and mixtures thereof. Preferred cationic polyelectrolytes may include poly(allylamine hydrochloride), corresponding salts thereof, and mixtures thereof.

In another embodiment of the present invention, the at least one encapsulant is a nanomaterial, preferably selected from the group consisting of metal nanoparticles, metal oxide nanoparticles, carbon nanotubes, graphene, graphene oxide, nanoclays, and mixtures thereof.

The multilayer shell comprises at least two individual layers, e.g., from 2 to 1000 individual layers, preferably from 2 to 100 individual layers and more preferably from 4 to 50 individual layers. The skilled person will adjust the number of the polyelectrolyte layers according to the desired multilayer shell thickness and permeability.

In a particularly preferred embodiment, the multilayer shell comprises alternating polyphenol layers, preferably tannic acid layers, and protein layers, preferably pepsin layers. In this embodiment, it is preferred that the multilayer shell comprises from 2 to 20, more preferably from 8 to 12 individual layers. The multilayer shell comprising pepsin and tannic acid may be able to withstand challenging environmental conditions, e.g., those found in gastric fluid, having a pH value of down to 1.5 and comprising digestive enzymes. Therefore, such multilayer shell may be able to protect the active ingredient or inactive precursor thereof from gastric digestive enzymes at highly acidic conditions for a sufficient amount of time. At the same time, the pepsin-tannic acid multilayer may allow for enhanced retention in the intestinal epithelium, where the pepsin layers are cleaved by the selective interaction of digestive proteins, such as α-chymotrypsin, followed by controlled release of the active ingredient or inactive precursor thereof, and subsequent uptake into the bloodstream, or lymphatic system, or subsequent contribution to gut microbiota, etc.

**The process for the production of a microcapsule**

According to one aspect of the present invention, a process for the production of a microcapsule comprising an active ingredient or inactive precursor thereof is provided, wherein the process comprises the following steps:

a) providing a surface-reacted calcium carbonate, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment,
b) providing an active ingredient or inactive precursor thereof,
c) contacting the active ingredient or inactive precursor thereof with the surface-reacted calcium carbonate to obtain a loaded surface-reacted calcium carbonate, and
d) encasing the loaded surface-reacted calcium carbonate with a multilayer shell by consecutively depositing at least two complementary layers using layer-by-layer assembly onto the loaded surface-reacted calcium carbonate.

**Steps a) and b)**

The inventive process comprises a step a), wherein a surface-reacted calcium carbonate is provided as described hereinabove. Furthermore, the inventive process comprises a step b), wherein an active ingredient or inactive precursor thereof is provided as described hereinabove.

The surface-reacted calcium carbonate may be provided in any form, for example, as suspension, slurry, dispersion, paste, powder, moist filter cake or in pressed or granulated form. According to one embodiment, the surface-reacted calcium carbonate is provided in dry form, preferably in form of a powder.

According to another embodiment, the surface-reacted calcium carbonate is provided in form of an aqueous suspension. According to one embodiment, the surface-reacted calcium carbonate is in form of an aqueous suspension having a solids content within the range from 1 to 90 wt.-%, preferably from 3 to 60 wt.-%, more preferably from 5 to 40 wt.-%, and most preferably from 10 to 25 wt.-%, based on the weight of the aqueous suspension. According to a preferred embodiment of the present invention, the aqueous suspension consists of water and the surface-reacted calcium carbonate. Alternatively, the aqueous suspension of the surface-reacted calcium carbonate may comprise further additives, for example, a dispersant. A suitable dispersant may be selected from the group comprising homopolymers or copolymers of polycarboxylic acid or salts thereof based on, for example, acrylic acid, methacrylic acid, maleic acid, fumaric acid or itaconic acid, and acrylamide, or mixtures thereof. Homopolymers or copolymers of acrylic acid are especially preferred. The homopolymer or copolymer can be fully in the acidic form or partially or fully neutralized. The weight average molecular weight $M_w$ of such products is preferably in the range from 2 000 to 15 000 g/mol, with a weight average molecular weight $M_w$ from 3 000 to 7 000 g/mol or 3 500 to 6 000 g/mol being especially preferred. According to an exemplary embodiment, the dispersant is sodium polyacrylate having a weight average molecular weight $M_w$ from 2 000 to 15 000 g/mol, preferably from 3 000 to 7 000 g/mol, and most preferably from 3 500 to 6 000 g/mol.

The active ingredient or inactive precursor thereof may be provided in any form, for example, as solution, suspension, slurry, dispersion, paste, powder, moist filter cake or in pressed or granulated form. According to one embodiment, the active ingredient or inactive precursor thereof is provided in dry form, preferably in form of a powder or granulate.

According to another embodiment, the active ingredient or inactive precursor thereof is provided in form of a suspension, preferably an aqueous suspension. Suitable solvents are known to the skilled person and may comprise water, methanol, ethanol, acetone, acetonitrile, tetrahydrofuran, butanone, ethyl acetate, dimethyl sulfoxide, dimethyl formamide, and mixtures thereof.

According to one embodiment, the active ingredient or inactive precursor thereof is in form of a suspension, preferably aqueous suspension, having a solids content within the range from 1 to 90 wt.-%, preferably from 3 to 60 wt.-%, more preferably from 5 to 40 wt.-%, and most preferably from 10 to 25 wt.-%, based on the weight of the aqueous suspension, or in an amount from 1 to 10 wt.-%, preferably 1 to 3 wt.-%, based on the total weight of the aqueous suspension. According to a preferred embodiment of the present invention, the aqueous suspension consists of water and the active ingredient or inactive precursor thereof.

The aqueous suspension of the active ingredient or inactive precursor thereof may comprise further additives, for example, one of the aforementioned dispersants.

According to still another embodiment, the active ingredient or inactive precursor thereof is provided in form of a solution, preferably an aqueous solution. Suitable solvents are known to the skilled person and may comprise water, methanol, ethanol, acetone, acetonitrile, tetrahydrofuran, butanone, ethyl acetate, dimethyl sulfoxide, dimethyl formamide, and mixtures thereof.

According to a preferred embodiment, the active ingredient or inactive precursor thereof is in form of a solution, preferably aqueous solution, comprising the active ingredient in an amount from 1 to 95 wt.-%, preferably from 10 to 90 wt.-%, more preferably from 20 to 80 wt.-%, and most preferably from 30 to 70 wt.-%, based on the weight of the aqueous solution, or in an amount from 1 to 10 wt.-%, preferably 1 to 3 wt.-%, based on the total weight of the aqueous solution.

According to one embodiment, the active ingredient or inactive precursor thereof is provided in an amount from 5 to 200 wt.-%, preferably 10 to 150 wt.-%, more preferably 15 to 100 wt.-%, and most preferably 20 to 50 wt.-%, relative to the weight of the surface-reacted calcium carbonate.

**Step c)**

According to step c) of the inventive process, the active ingredient or inactive precursor thereof is contacted with the surface-reacted calcium carbonate to obtain a loaded surface-reacted calcium carbonate.

In general, the surface-reacted calcium carbonate and the active ingredient or inactive precursor thereof can be brought into contact by any conventional means known to the skilled person. For example, the surface-reacted calcium carbonate and the active ingredient or inactive precursor thereof may be mixed in the absence or presence of a solvent. Suitable mixing devices are known to the skilled person and may include mixers or blenders, e.g., a tumbling mixer. The skilled person will adapt the mixing conditions (such as the configuration of mixing speed) according to his needs and available equipment.

The contacting of surface-reacted calcium carbonate and the active ingredient or inactive precursor thereof in step c) may be carried out in any order. According to one embodiment, the surface-reacted calcium carbonate is provided in a first step, and subsequently, the active agent or inactive precursor thereof is added. According to another embodiment, the active ingredient or inactive precursor thereof is provided in a first step, and subsequently, the surface-reacted calcium carbonate is provided. According to still another embodiment, the surface-reacted calcium carbonate and the active ingredient or inactive precursor thereof are contacted simultaneously, for example, by adding both compounds simultaneously into a vessel.

According to one exemplary embodiment, the active ingredient or inactive precursor thereof is in form of a suspension or solution, and the contacting step is carried by dropwise addition of the active ingredient or inactive precursor thereof to an agitated powder of the surface-reacted calcium carbonate. According to another exemplary embodiment, the active ingredient or inactive precursor thereof is provided in molten form and added to the surface-reacted calcium carbonate.

According to still another exemplary embodiment, the active ingredient or inactive precursor thereof is provided in form of a suspension or solution and the surface-reacted calcium carbonate is added to said suspension or solution, wherein preferably the suspension or solution is agitated. The solution or suspension may comprise the active ingredient or inactive precursor thereof in a concentration from 0.1 to 500 mg/mL, preferably from 0.5 to 250 mg/mL, more preferably from 1 to 250 mg/mL, yet more preferably from 5 to 100 mg/mL, and most preferably from 10 to 50 mg/mL. According to one embodiment, the active ingredient or inactive precursor thereof is comprised in the solution or suspension in an amount from 5 to 200 wt.-%, preferably 10 to 150 wt.-%, more preferably 15 to 100 wt.-%, and most preferably 20 to 50 wt.-%, relative to the weight of the surface-reacted calcium carbonate.

Preferably, the surface-reacted calcium carbonate is provided in solid form and suspended in the suspension or solution of active ingredient or inactive precursor thereof.

The contacting step c) may be carried out for a time period in the range of several seconds to several minutes, e.g. 20 s or more, preferably 30 s or more, more preferably 60 s or more, and most preferably for a period of 120 s or more. According to one embodiment step c) is carried out for at least 3 min, at least 4 min, at least 5 min, at least 10 min, at least 20 min, or at least 30 min.

Contacting step c) may be carried out at a temperature of up to 200 °C, preferably from -20 °C to 150 °C, more preferably from 0 °C to 100 °C, for example from 20 °C to 50 °C. Especially preferably, contacting step c) is performed at room temperature, i.e., at 25 ± 5 °C.

It is appreciated that during contacting of the active ingredient or inactive precursor thereof with the surface-reacted calcium carbonate, the active ingredient or inactive precursor thereof will be deposited within the accessible pores and/or onto the surface of the surface-reacted calcium carbonate, i.e., will be adsorbed onto and/or absorbed into the surface-reacted calcium carbonate. The loaded surface-reacted calcium carbonate may comprise the active ingredient or inactive precursor thereof in an amount from at least 1.5 wt.%, preferably at least 3 wt.-%, more preferably at least 5 wt.-%, and most preferably of at least 10 wt.-% to, e.g., less than 50 wt.-%.

After the contacting step, the loaded surface-reacted calcium carbonate may be separated from the liquid medium, if present. For example, the loaded surface-reacted calcium carbonate may be separated from the liquid medium by evaporating the liquid medium, by filtration and/or by centrifugation. Furthermore, the loaded surface-reacted calcium carbonate may be washed after separation, for example, with water.

**Step d)**

According to method step d), the loaded surface-reacted calcium carbonate is encased with a multilayer shell by consecutively depositing at least two complementary layers using layer-by-layer assembly onto the loaded surface-reacted calcium carbonate.

The layer-by-layer assembly is a well-established thin film fabrication technique known to the skilled person. Typically, films are formed by depositing alternating layers of complementary encapsulants onto a template with washing steps in between. The properties of the layer-by-layer-assembled films such as composition, thickness, and function, can be readily tuned by simply varying the type of species adsorbed, the number of layers deposited, and the conditions employed during the assembly process (see, e.g., Wang et al., "Template Synthesis of Nanostructured Materials via Layer-by-Layer Assembly", Chem. Mater. 2008, 20, 848-858, and "Multilayer Thin Films: Sequential Assembly of Nanocomposite Materials", 2nd Edition, Eds: G. Decher, J. Schlenoff. Weinheim: Wiley, 2012).

A first layer may be formed onto the loaded surface-reacted calcium carbonate by depositing a first encapsulant on the surface of the loaded surface-reacted calcium carbonate, whereas further layers are formed by depositing further encapsulants onto the first layer. The deposition of the encapsulants is governed by electrostatic forces, formation of covalent bonds or hydrogen bonds, hydrophobic interactions, such as $\pi$-$\pi$ interactions, complementary base pairing, other types of interactions, or a combination of the aforementioned interactions. Therefore, it is a requirement that the consecutively deposited layers are complementary to each other, that is, attractive interactions between the consecutively deposited layers are present.

For example, if the loaded surface-reacted calcium carbonate is positively charged, e.g., due to its zeta potential or due to the charge of the loaded active ingredient or inactive precursor thereof, the first layer may be formed from an anionic polyelectrolyte. If the loaded surface-reacted calcium carbonate is negatively charged, e.g., due to its zeta potential or due to the charge of the loaded active ingredient or inactive precursor thereof, the first layer may be formed from a cationic polyelectrolyte. Likewise, if the outermost layer of the multilayer shell is positively charged, the immediately following encapsulant to be deposited may be anionic and vice versa. Suitable encapsulants are those described hereinabove.

In another illustrative example, the surface-reacted calcium carbonate comprises functional groups, such as hydroxyl groups or carbonate groups, on its surface, which may act as hydrogen bond donors or acceptors, and/or the loaded active ingredient or inactive precursor thereof comprises functional groups, which may act as hydrogen bond donors and/or acceptors. Then, the first layer may be formed from an encapsulant capable of forming hydrogen bonds with the surface-reacted calcium carbonate and/or the active ingredient and/or inactive precursor thereof. Likewise, if the outermost layer of the multilayer shell comprises moieties, which may act as hydrogen bond donors or acceptors, the immediately following encapsulant to be deposited may be capable of forming hydrogen bonds with the immediately preceding layer. Suitable encapsulants are those described hereinabove, and preferably include proteins and polyphenols.

According to one embodiment of the inventive process, step d) comprises the sub-steps of

i) incubating the loaded surface-reacted calcium carbonate in a first liquid medium comprising a first encapsulant to encase the loaded surface-reacted calcium carbonate with a first layer,
ii) removing the first liquid medium and washing the loaded surface-reacted calcium carbonate obtained in step i) one or more times with a first solvent, preferably water,
iii) incubating the loaded surface-reacted calcium carbonate obtained in step ii) in a second liquid medium comprising a second encapsulant being complementary to the first encapsulant to encase the loaded surface-reacted calcium carbonate with a second layer,
iv) removing the second liquid medium and washing the loaded surface-reacted calcium carbonate obtained in step iii) one or more times with a second solvent, preferably water, and
v) optionally repeating steps i) to iv) one or more times with the same or different first and second encapsulants and the same or different first and second liquid media and/or solvents.

According to one embodiment, steps i) and iii) are performed by adding the loaded surface-reacted calcium carbonate to an agitated liquid medium comprising the first encapsulant or second encapsulant, respectively. The first liquid medium may comprise the first encapsulant in a concentration from 0.1 to 50 mg/mL, preferably from 0.5 to 30 mg/mL, more preferably from 1 to 20 mg/mL, even more preferably from 1.5 to 10 mg/mL, and most preferably from 1.5 to 5 mg/mL. The second liquid medium may comprise the second encapsulant in a concentration from 0.1 to 50 mg/mL, preferably from 0.5 to 30 mg/mL, more preferably from 1 to 20 mg/mL, even more preferably from 1.5 to 10 mg/mL, and most preferably from 1.5 to 5 mg/mL. The loaded surface-reacted calcium carbonate may be separated from the first and/or second liquid medium by any suitable means known to the skilled person, e.g., by filtration or centrifugation.

In a preferred embodiment, the first encapsulant is a protein and the second encapsulant is a polyphenol; or the first encapsulant is a polyphenol and the second encapsulant is a protein. In another preferred embodiment, the first encapsulant is a cationic polyelectrolyte and the second encapsulant is an anionic polyelectrolyte; or the first encapsulant is an anionic polyelectrolyte and the second encapsulant is a cationic polyelectrolyte.

In a particularly preferred embodiment of the present invention, one of the first encapsulant or the second encapsulant is selected from the group consisting of tannic acid, polyacrylic acid, poly(glutamic acid), poly(lactic acid), poly(lactic-co-glycolic acid), chondroitin sulphate, dextran sulphate, poly(styrene sulfonate), montmorillonite, alginic acid, sodium alginate, polyphosphoric acid, polyvinyl sulfonic acid, polyvinyl phosphonic acid, polysulfates, gum arabic, poly(ethylene oxide), anionic cellulose derivatives, preferably carboxymethyl cellulose, corresponding salts thereof, and mixtures thereof, preferably the one of the first encapsulant or the second encapsulant is selected from the group consisting of tannic acid, poly(styrene sulfonate), corresponding salts thereof, and mixtures thereof, and the other one of the first or the second encapsulant is selected from the group consisting of pepsin, poly(dimethylammonium chloride), poly(allylamine hydrochloride), poly(lysine), poly(arginine), protamine sulphate, polyethyleneimine, poly(4-vinylpyridine), chitosan, glycol chitosan, polyvinylamine, amine-containing polymers, preferably polyamidoamine-epichlorohydrin or poly[2-(dimethylamino)ethyl methacrylate], bovine serum albumin, corresponding salts thereof, and mixtures thereof, preferably the other one of first encapsulant or the second encapsulant is selected from the group consisting of pepsin, poly(allylamine hydrochloride), corresponding salts thereof, and mixtures thereof.

During steps ii) and iv), the surface-reacted calcium carbonate may be washed by agitating the first solvent or second solvent, respectively. The washed loaded surface-reacted calcium carbonate may be separated from the first and/or second solvent by any suitable means known to the skilled person, e.g., by filtration or centrifugation.

It is appreciated that each of steps i) to iv) may be performed in the same or different liquid medium and/or solvent. Examples of a suitable liquid medium and/or solvent are water, an aqueous solution of sodium chloride, acetone, butanone, ethanol, methanol, 1-propanol, 2-propanol, 1-butanol, dimethyl sulfoxide, N-methyl pyrrolidone, dimethyl formamide, dimethyl acetamide, dimethyl sulfone, or mixtures thereof. Preferably, one or more or all steps i) to iv) may be performed in water. The use of water as liquid medium and/or solvent may be particularly preferred, if the active ingredient or inactive precursor thereof and/or at least one of the encapsulants is a protein. According to one embodiment, the first liquid medium, the second liquid medium, the first solvent, and the second solvent are selected independently from each other from the group consisting of water, an aqueous solution of sodium chloride, acetone, butanone, ethanol, methanol, 1-propanol, 2-propanol, 1-butanol, dimethyl sulfoxide, N-methyl pyrrolidone, dimethyl formamide, dimethyl acetamide, dimethyl sulfone, or mixtures thereof, and preferably the first liquid medium, the second liquid medium, the first solvent, and the second solvent are water. Preferably, the water for any of the purposes and embodiments set out herein is selected from tap water, deionized water, distilled water or reverse-osmosis water.

Alternatively, it may be preferred that the first liquid medium and the second liquid medium are water and the first solvent and the second solvent are independently from each other selected from water and an aqueous solution of sodium chloride. In the case that the first solvent is an aqueous solution of sodium chloride, it is preferred that the ionic strength of the first liquid medium and the first solvent do not differ by more than 10%, more preferably not more than 5 %, and most preferably not more than 2% from each other. In the case that the second solvent is an aqueous solution of sodium chloride, it is preferred that the ionic strength of the second liquid medium and the second solvent do not differ by more than 10%, more preferably not more than 5 %, and most preferably not more than 2% from each other. The ionic strength I is defined on a molar basis as

$$I = \frac{1}{2}\sum_{i=1}^{n} c_i z_i^2 \ ,$$

wherein i = ion i, $c_i$ = molar concentration of ion i, $Z_i$ = charge of ion i, and n = number of different ions i in the solution.

It is further appreciated that each of steps i) to iv) may be repeated with the same or different first and second encapsulants and the same or different first and second liquid media and/or solvents. Thus, a flexible process is provided, which may allow fine-tuning of the properties of the microcapsule, such as wall thickness, wall structure, wall composition, permeability and porosity. The choice of the first and second encapsulants may also influence the stability of the multilayer shell, which may be stable or labile under the influence of acids, bases, solvents, UV light, visible light, enzymes, pressure and/or shearing forces and may allow for obtaining a controlled burst release or sustained release profile.

**Optional steps**

The surface-reacted calcium carbonate may be removed after the loaded surface-reacted calcium carbonate has been encased by the multilayer shell. Thereby, a microcapsule comprising an active ingredient or inactive precursor thereof, which is essentially free of calcium carbonate and/or other inorganic calcium-containing compounds, may be obtained. In such an embodiment, the surface-reacted calcium carbonate acts as a removable template.

Many conventionally employed templates often require harsh conditions in order to dissolve them or to decompose them. For example, porous silica templates are typically dissolved with hydrofluoric acid. In contrast, it was found by the inventors of the present invention that the employed surface-reacted calcium carbonate template can be decomposed using relatively mild conditions, such as by the interaction with less aggressive acids, acidic salts or calcium scavenging agents, such as chelating agents. The multilayer shell obtained by the inventive process may generally allow the diffusion of small molecules and ions, such as hydronium ions or calcium ions and suitable counter ions, from the outer side of the microcapsule to the inside and vice versa.

According to a preferred embodiment, the method further comprises the step e) of reacting the microcapsule obtained in step d) with an acidic compound to decompose the loaded surface-reacted calcium carbonate within the shell. Any suitable acidic compound known in the art may be used. According to one embodiment, the acidic compound is selected from the group consisting of hydrochloric acid, hydrobromic acid, sulphuric acid, acetic acid, formic acid, calcium scavenging agents, preferably ethylene diamine tetraacetic acid, hydrogen sulfate, dihydrogen phosphate, hydrogen phosphate, and mixtures thereof. In a preferred embodiment, the acidic compound is hydrochloric acid. It should be understood that during the decomposition step, the surface-reacted calcium carbonate undergoes a chemical reaction, yielding gaseous carbon dioxide and a soluble calcium salt, both of which may be removed from the microcapsule.

Calcium scavenging agents in the meaning of the present invention include chelants, such as ethylene diamine tetraacetic acid, nitrilotriacetic acid (NTA), iminodisuccinic acid (IDS), polyaspartic acid, S,S-ethylenediamine-N,N'-disuccinic acid (EDDS), methylglycinediacetic acid (MGDA), L-glutamic acid N,N-diacetic acid (GLDA), and salts thereof.

The acidic compound may be applied in concentrated form or in diluted form. According to one embodiment of the present invention, the acidic compound is dissolved in water and/or a solvent. Suitable solvents are known in the art and are, for example, aliphatic alcohols, ethers and diethers having from 4 to 14 carbon atoms, glycols, alkoxylated glycols, glycol ethers, alkoxylated aromatic alcohols, aromatic alcohols, mixtures thereof, or mixtures thereof with water. According to a preferred embodiment, the acidic compound is in form of an aqueous solution.

According to one embodiment, the acidic compound is provided in form of a solution, preferably an aqueous solution, comprising the acidic compound in an amount from 0.1 to 99 wt.-%, based on the total weight of the liquid treatment composition, preferably in an amount from 1 to 80 wt.-%, more preferably in an amount from 2 to 50 wt.-%, and most preferably in an amount from 5 to 30 wt.-%.

Process step e) may be carried out at mild reaction conditions in order to avoid degradation or decomposition of the multilayer shell and/or the active ingredient or inactive precursor thereof. For example, it may be preferred that the pH is not decreased below 2.5 during the whole process step e). This may be particularly important for multilayer shells comprising proteins or active ingredients or inactive precursors thereof being proteins, which may be denatured by the interaction with a strongly acidic solution. According to one embodiment, process step e) is carried out by adding the acidic compound until the pH of the aqueous medium is in the range from 1 to 6, more preferably from 1.5 to 5, even more preferably from 2 to 4.5, and most preferably from 2.5 to 3.5. According to another embodiment, process step e) is carried out by adding the acidic compound until the pH of the aqueous medium is in the range from 4 to 6, more preferably from 4.2 to 5.5, and most preferably from 4.5 to 5. Preferably, process step e) is performed at a temperature in the range from 10 to 30 °C, e.g., at room temperature.

After process step e) the obtained microcapsule may be separated from the acidic compound by any suitable means known to the skilled person, e.g., by filtration or centrifugation, optionally followed by one or more washing steps with water until the pH of the supernatant after washing reaches a desired pH, preferably a pH from 4.5 to 6.5.

According to a further embodiment, the process of the present invention comprises a step f) of drying the microcapsule. The process step f) may be carried out after step d) or after step e), if present. Any suitable drying method known in the art may be used. Examples of suitable drying methods are convective drying, preferably spray drying, contact drying, preferably drum drying or vacuum drying, microwave drying, supercritical drying or freeze drying. The skilled person will select the drying process such as to prevent the multilayer shell and the active ingredient or inactive precursor thereof from evaporating or decomposing during the drying process. According to a preferred embodiment, the drying is freeze drying.

Optionally, the inventive microcapsule may be formulated with further ingredients and/or excipients to obtain a product comprising the inventive microcapsule. The present invention is not limited to any specific ingredients and/or excipients and includes all ingredients and/or excipients known to the skilled person, e.g., fillers, disintegrants, lubricants, glidants, binders, coatings, solubilizers, stabilizers, buffers, tonicity modifiers, bulking agents, viscosity enhancers, viscosity reducers, surfactants, chelating agents, adjuvants and mixtures thereof.

In a preferred embodiment of the present aspect of the invention, the process comprises the steps of:

a) providing a surface-reacted calcium carbonate having a BET specific surface area from 20 to 200 g/m$^2$, preferably 40 to 150 g/m$^2$, more preferably 70 to 120 g/m$^2$,
b) providing an active ingredient or inactive precursor thereof, preferably a macromolecular active ingredient, more preferably a macromolecular pharmaceutically active ingredient, e.g., lactoferrin or bovine serum albumin,
c) contacting the active ingredient or inactive precursor thereof with the surface-reacted calcium carbonate to obtain a loaded surface-reacted calcium carbonate, and
d) encasing the loaded surface-reacted calcium carbonate with a multilayer shell by consecutively depositing at least two complementary layers using layer-by-layer assembly onto the loaded surface-reacted calcium carbonate, wherein the encapsulants are independently selected from the group consisting of polysaccharides, polyphenols, proteins, nucleic acids, cationic polyelectrolytes, anionic polyelectrolytes, nanomaterials and mixtures thereof, preferably selected from the group consisting of polyphenols and proteins, e.g., tannic acid, corresponding salts thereof, and pepsin.

In another preferred embodiment of the present aspect of the invention, the process comprises the steps of:

a) providing a surface-reacted calcium carbonate having a BET specific surface area from 20 to 200 g/m$^2$, preferably 40 to 150 g/m$^2$, more preferably 70 to 120 g/m$^2$,
b) providing an active ingredient or inactive precursor thereof, preferably a macromolecular active ingredient, more preferably a macromolecular pharmaceutically active ingredient, e.g., lactoferrin or bovine serum albumin,
c) contacting the active ingredient or inactive precursor thereof with the surface-reacted calcium carbonate to obtain a loaded surface-reacted calcium carbonate,
d) encasing the loaded surface-reacted calcium carbonate with a multilayer shell by consecutively depositing at least two complementary layers using layer-by-layer assembly onto the loaded surface-reacted calcium carbonate, wherein the encapsulants are independently selected from the group consisting of polysaccharides, polyphenols, proteins, nucleic acids, cationic polyelectrolytes, anionic polyelectrolytes, nanomaterials and mixtures thereof, preferably selected from the group consisting of polyphenols and proteins, e.g., tannic acid, corresponding salts thereof, and pepsin, and
e) reacting the microcapsule obtained in step d) with an acidic compound to decompose the loaded surface-reacted calcium carbonate within the multilayer shell, preferably the acidic compound is selected from the group consisting of hydrochloric acid, hydrobromic acid, sulphuric acid, nitric acid, acetic acid, formic acid, hydrogen sulfate, dihydrogen phosphate, hydrogen phosphate, calcium scavenging agents, preferably ethylene diamine tetraacetic acid, and salts and mixtures thereof, and more preferably the acidic compound is hydrochloric acid, optionally wherein step e) is carried out in an aqueous medium, preferably wherein the acidic compound is added until the pH of the aqueous medium is in the range from 1 to 6, more preferably from 1.5 to 5, even more preferably from 2 to 4.5, and most preferably from 2.5 to 3.5.

**The inventive microcapsule**

According to a further aspect of the present invention, a microcapsule comprising an active ingredient or inactive precursor thereof obtainable by a process according to the present invention is provided.

Thus, a microcapsule containing an active ingredient or inactive precursor thereof is provided, comprising a surface-reacted calcium carbonate, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment,
an active ingredient or inactive precursor thereof loaded onto the surface-reacted calcium carbonate, and
a multilayer shell encasing the loaded surface-reacted calcium carbonate, wherein the shell comprises at least two complementary layers.
According to a further embodiment a microcapsule containing an active ingredient or inactive precursor thereof is provided, comprising
an active ingredient or inactive precursor thereof, and
a multilayer shell encasing the active ingredient or inactive precursor thereof, wherein the shell comprises at least two complementary layers,
wherein the microcapsule contains the active ingredient in an amount of at least 5 wt.-%, based on the total weight of the microcapsule.

The inventors of the present invention surprisingly found that the inventive process allows for obtaining microcapsules, which contain increased amounts of the active ingredient or inactive precursor thereof, compared to conventional approaches. Thus, in a preferred embodiment, the microcapsule comprises the active ingredient or inactive precursor thereof in an amount of at least 10 wt.-%, more preferably at least 25 wt.-%, and most preferably at least 50 wt.-%, based on the total weight of the microcapsule.

In particular, it was found that the present invention yields microcapsules having a higher content of an active ingredient or inactive precursor thereof compared to the same loaded microcapsule utilizing a vaterite microparticle as template. Vaterite microparticles are typically obtained by colloidal crystallization from a solution prepared by rapid mixing of calcium chloride and sodium carbonate solutions, as described in D. V. Volodkin et al., "Matrix Polyelectrolyte Microcapsules: New System for Macromolecule Encapsulation", Langmuir 2004, 20, 3398-3406. According to one embodiment, the inventive microcapsule comprises at least 2 times, preferably at least 5 times, more preferably at least 8 times, and most preferably 10 times, the amount of the active ingredient or inactive precursor thereof, compared to a microcapsule utilizing a vaterite microparticle as template.

The microcapsule of the present invention may have a particle size of less than 100 $\mu$m. According to one embodiment, the microcapsule has a particle size in the range from 0.2 to 80 $\mu$m, preferably 0.5 to 60 $\mu$m, more preferably from 1 to 40 $\mu$m, even more preferably from 1 to 20 $\mu$m, and most preferably from 2 to 10 $\mu$m, determined by scanning electron microscopy. For the purposes of the present invention, the particle size is determined by obtaining a two-dimensional micrograph of the three-dimensional particle using scanning electron microscopy. The particle size is defined as the longest straight line that can be drawn between the edges of the particle whilst passing through the centre of mass of the two-dimensional image of the particle (also termed particle diameter). Alternatively, particle size can be determined as hydrodynamic diameter using known techniques, e.g. static light scattering. Thus, in another embodiment of the present invention, the microcapsule has a particle size of less than 100 $\mu$m , preferably in the range from 0.2 to 80 $\mu$m , more preferably 0.5 to 60 $\mu$m, even more preferably from 1 to 40 $\mu$m, still more preferably from 1 to 20 $\mu$m, and most preferably from 2 to 10 $\mu$m, determined by static light scattering with a Mastersizer 2000 (Malvern Panalytical Ltd, UK).

The present inventors found that the inventive process provides the possibility to produce very small microcapsules. In particular, it was found that the inventive process allows for encasing individual loaded surface-reacted calcium carbonate particles, i.e., one individual loaded surface-reacted calcium carbonate particles or 2 to 5 individual loaded surface-reacted calcium carbonate particles. Consequently, in another embodiment of the present invention, the inventive microcapsule comprises from 1 to 5, preferably 1 or 2 individual loaded surface-reacted calcium carbonate particles.

It is also appreciated that the surface-structure of the loaded surface-reacted calcium carbonate particles influences the final structure of the multilayer shell, e.g., by defining certain regions of the particle, on which the polyelectrolyte layer is preferably formed or deposited. For example, the porous structure of the surface-reacted calcium carbonate particles may be resembled in the multilayer shell structure.

In a preferred embodiment of the present aspect of the invention, the microcapsule of the present invention is obtained by a process comprising the steps of:

a) providing a surface-reacted calcium carbonate having a BET specific surface area from 20 to 200 g/m$^2$, preferably 40 to 150 g/m$^2$, more preferably 70 to 120 g/m$^2$,
b) providing an active ingredient or inactive precursor thereof, preferably a macromolecular active ingredient, more preferably a macromolecular pharmaceutically active ingredient, e.g., lactoferrin or bovine serum albumin,
c) contacting the active ingredient or inactive precursor thereof with the surface-reacted calcium carbonate to obtain a loaded surface-reacted calcium carbonate, and
d) encasing the loaded surface-reacted calcium carbonate with a multilayer shell by consecutively depositing at least two complementary layers using layer-by-layer assembly onto the loaded surface-reacted calcium carbonate, wherein the encapsulants are independently selected from the group consisting of polysaccharides, polyphenols, proteins, nucleic acids, cationic polyelectrolytes, anionic polyelectrolytes, nanomaterials and mixtures thereof, preferably selected from the group consisting of polyphenols and proteins, e.g., tannic acid, corresponding salts thereof, and pepsin.

In another preferred embodiment of the present aspect of the invention, the microcapsule of the present invention is obtained by a process comprising the steps of:

a) providing a surface-reacted calcium carbonate having a BET specific surface area from 20 to 200 g/m$^2$, preferably 40 to 150 g/m$^2$, more preferably 70 to 120 g/m$^2$,
b) providing an active ingredient or inactive precursor thereof, preferably a macromolecular active ingredient, more preferably a macromolecular pharmaceutically active ingredient, e.g., lactoferrin or bovine serum albumin,
c) contacting the active ingredient or inactive precursor thereof with the surface-reacted calcium carbonate to obtain a loaded surface-reacted calcium carbonate,

d) encasing the loaded surface-reacted calcium carbonate with a multilayer shell by consecutively depositing at least two complementary layers using layer-by-layer assembly onto the loaded surface-reacted calcium carbonate, wherein the encapsulants are independently selected from the group consisting of polysaccharides, polyphenols, proteins, nucleic acids, cationic polyelectrolytes, anionic polyelectrolytes, nanomaterials and mixtures thereof, preferably selected from the group consisting of polyphenols and proteins, e.g., tannic acid, corresponding salts thereof, and pepsin, and

e) reacting the microcapsule obtained in step d) with an acidic compound to decompose the loaded surface-reacted calcium carbonate within the multilayer shell, preferably the acidic compound is selected from the group consisting of hydrochloric acid, hydrobromic acid, sulphuric acid, nitric acid, acetic acid, formic acid, hydrogen sulfate, dihydrogen phosphate, hydrogen phosphate, calcium scavenging agents, preferably ethylene diamine tetraacetic acid, and salts and mixtures thereof, and more preferably the acidic compound is hydrochloric acid, optionally wherein step e) is carried out in an aqueous medium, preferably wherein the acidic compound is added until the pH of the aqueous medium is in the range from 1 to 6, more preferably from 1.5 to 5, even more preferably from 2 to 4.5, and most preferably from 2.5 to 3.5.

In yet another preferred embodiment of the present invention, the microcapsule containing an active ingredient or inactive precursor thereof comprises

a surface-reacted calcium carbonate having a BET specific surface area from 20 to 200 $g/m^2$, preferably 40 to 150 $g/m^2$, more preferably 70 to 120 $g/m^2$,

an active ingredient or inactive precursor thereof, preferably a macromolecular active ingredient, more preferably a macromolecular pharmaceutically active ingredient, e.g., lactoferrin or bovine serum albumin, loaded onto the surface-reacted calcium carbonate, and

a multilayer shell encasing the loaded surface-reacted calcium carbonate, wherein the shell comprises at least two complementary layers formed from encapsulants independently selected from the group consisting of polysaccharides, polyphenols, proteins, nucleic acids, cationic polyelectrolytes, anionic polyelectrolytes, nanomaterials and mixtures thereof, preferably selected from the group consisting of polyphenols and proteins, e.g., tannic acid, corresponding salts thereof, and pepsin.

In still another preferred embodiment of the present invention, the microcapsule containing an active ingredient or inactive precursor thereof comprises

an active ingredient or inactive precursor thereof, preferably a macromolecular active ingredient, more preferably a macromolecular pharmaceutically active ingredient, e.g., lactoferrin or bovine serum albumin, and

a multilayer shell encasing the active ingredient or inactive precursor thereof, wherein the shell comprises at least two complementary layers formed from encapsulants independently selected from the group consisting of polysaccharides, polyphenols, proteins, nucleic acids, cationic polyelectrolytes, anionic polyelectrolytes, nanomaterials and mixtures thereof, preferably selected from the group consisting of polyphenols and proteins, e.g., tannic acid, corresponding salts thereof, and pepsin,

wherein the microcapsule contains the active ingredient in an amount of at least 5 wt.-%, preferably at least 10 wt.-%, more preferably at least 25 wt.-%, and most preferably at least 50 wt.-%, based on the total weight of the microcapsule.

A third aspect of the present invention relates to the use of a surface-reacted calcium carbonate as a template for encapsulating an active ingredient or inactive precursor thereof using layer-by-layer assembly, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment.

It is appreciated that the surface-reacted calcium carbonate, and the active ingredient or inactive precursor thereof are as defined hereinabove. Thus, the surface-reacted calcium carbonate may be used as a template in a process as described hereinabove, and allows for obtaining a microcapsule comprising an active ingredient or inactive precursor thereof.

A fourth aspect of the present invention relates to a product comprising the inventive microcapsule as described hereinabove. The product may be a pharmaceutical product, a foodstuff, a feedstuff, a food supplement, a feed supplement, a cosmetic product, a paper product, a painting product, a coating product, or an agricultural product.

Pharmaceutical products may include tablets, mini-tablets, pellets, capsules, granules, tablet-in-cups, gastroretentive formulations, sustained-release formulations, immediate-release formulations, aqueous or non-aqueous solutions, crèmes, ointments, lotions, gels, pastes, toothpastes, mouthwashes or eye drops. The pharmaceutical products may be packaged in container closure systems for extended periods of time. Said container closure systems include blisters, bottles, vials, IV bags, ampules, syringes, and cartridges. The container closure systems can be made from a variety of materials including glass, plastic, and metal.

Cosmetic products may include lotions, crèmes, ointments, skin-care compositions, makeup, perfume, soaps, cleansing creams, lipstick, shampoos, shower gels, antiperspirants, mouthwashes, depilatories, nail polish, astringents, or bath crystals.

Agricultural products may include any raw material or composition used in agriculture, e.g., fertilizers, or plant protection agents.

**Clauses**

The present invention is further defined by the following clauses.

Clause 1. A microcapsule containing an active ingredient or inactive precursor thereof, comprising a surface-reacted calcium carbonate, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment, an active ingredient or inactive precursor thereof loaded onto the surface-reacted calcium carbonate, and a multilayer shell encasing the loaded surface-reacted calcium carbonate, wherein the shell comprises at least two complementary layers.

Clause 2. The microcapsule according to clause 1, wherein the surface-reacted calcium carbonate has a BET specific surface area from 20 to 200 g/m$^2$, preferably 40 to 150 g/m$^2$, more preferably 70 to 120 g/m$^2$; and/or a volume median particle size $d_{50}$ from 0.1 to 75 $\mu$m, preferably from 0.5 to 50 $\mu$m, more preferably from 1 to 40 $\mu$m, even more preferably from 1.2 to 30 $\mu$m, and most preferably from 1.5 to 15 $\mu$m; and/or a volume top cut particle size $d_{98}$ from 0.2 to 150 $\mu$m, preferably from 1 to 100 $\mu$m, more preferably from 2 to 80 $\mu$m, even more preferably from 2.4 to 60 $\mu$m, and most preferably from 3 to 30 $\mu$m; and/or an intra-particle intruded specific pore volume in the range from 0.1 to 2.5 cm$^3$/g, more preferably from 0.2 to 2.2 cm$^3$/g, still more preferably from 0.4 to 2.0 cm$^3$/g and most preferably from 0.6 to 1.8 cm$^3$/g, determined by mercury porosimetry measurement.

Clause 3. The microcapsule according to clause 1 or 2, wherein the active ingredient is a pharmaceutically active ingredient, a cosmetic active ingredient, a nutraceutical active ingredient, a biocide, a pesticide, a wetting agent, a UV protecting agent, a scavenger, a pro-drug, a precursor of a cosmetic active ingredient, a precursor of a nutraceutical active ingredient, a precursor of a biocide, a precursor of a pesticide, a precursor of a wetting agent, a precursor of a UV protecting agent, a precursor of a scavenger, or a mixture thereof, preferably the active ingredient or inactive precursor thereof is a macromolecular active ingredient or an inactive precursor thereof, more preferably a protein, and most preferably lactoferrin, bovine serum albumin, or an enzyme.

Clause 4. The microcapsule according to any one of clauses 1 to 3, wherein the at least two complementary layers are formed from encapsulants independently selected from the group consisting of nanomaterials, macromolecular encapsulants and mixtures thereof, preferably the encapsulants are independently selected from the group consisting of polysaccharides, polyphenols, proteins, nucleic acids, cationic polyelectrolytes, anionic polyelectrolytes, nanomaterials and mixtures thereof, more preferably the encapsulants are independently selected from the group consisting of tannic acid, polyacrylic acid, poly(4-vinylpyridine), poly(glutamic acid), poly(lactic acid), chondroitin sulphate, dextran sulphate, poly(styrene sulfonate), sodium alginate, hyaluronic acid, polyphosphoric acid, polyvinyl sulfonic acid, polyvinyl phosphonic acid, polysulfates, dextrans, gum arabic, pectin, anionic cellulose derivatives, preferably carboxymethyl cellulose, pepsin, poly(dimethylammonium chloride), poly(ethylene oxide), poly(allylamine hydrochloride), poly(lysine), poly(lactic-co-glycolic acid), poly(arginine), protamine sulphate, polyethyleneimine, chitosan, glycol chitosan, montmorillonite, polyvinylamine, amine-containing polymers, such as polyamidoamine-epichlorohydrin and poly[2-(dimethylamino)ethyl methacrylate], bovine serum albumin, corresponding salts thereof, metal nanoparticles, metal oxide nanoparticles, carbon nanotubes, graphene, graphene oxide, nanoclays, and mixtures thereof, even more preferably the encapsulants are selected from the group consisting of tannic acid, poly(styrene sulfonate), poly(allylamine hydrochloride), bovine serum albumin, pepsin, corresponding salts thereof, and mixtures thereof, and most preferably the encapsulants are independently selected from the group consisting of tannic acid, pepsin, and corresponding salts thereof.

Clause 5. The microcapsule according to clause 4, wherein the first encapsulant is a protein and the second encapsulant is a polyphenol; or the first encapsulant is a polyphenol and the second encapsulant is a protein; or the first encapsulant is a cationic polyelectrolyte and the second encapsulant is an anionic polyelectrolyte; or the first encapsulant is an anionic polyelectrolyte and the second encapsulant is a cationic polyelectrolyte; preferably one of the first encapsulant or the second encapsulant is selected from the group consisting of tannic acid, polyacrylic acid, poly(glutamic acid), poly(lactic acid), poly(lactic-co-glycolic acid), chondroitin sulphate, dextran sulphate, poly(styrene sulfonate), montmorillonite, alginic acid, sodium alginate, polyphosphoric acid, polyvinyl sulfonic acid, polyvinyl phosphonic acid, polysulfates, gum arabic, poly(ethylene oxide), anionic cellulose derivatives, preferably carboxymethyl cellulose, corresponding salts thereof, and mixtures thereof, preferably the one of the first encapsulant or the second encapsulant is selected from the group consisting of tannic acid, poly(styrene sulfonate), corresponding salts thereof, and mixtures thereof, and the other one of the first or the second encapsulant is selected from the group consisting of pepsin, poly(dimethylammonium chloride), poly(allylamine hydrochloride), poly(lysine), poly(arginine), protamine sulphate, polyethyleneimine, poly(4-vinylpyridine), chitosan, glycol chitosan, polyvinylamine, amine-containing polymers, preferably polyamidoamine-epichlorohydrin or poly[2-(dimethylamino)ethyl methacrylate], bovine serum albumin, corresponding salts thereof, and mixtures thereof, preferably the other one of the first encapsulant or the second encapsulant is selected from the group consisting of pepsin, poly(allylamine hydrochloride), corresponding salts thereof, and mixtures thereof.

Clause 6. The microcapsule according to any one of clauses 1 to 5, wherein the microcapsule has a particle size in the range from 0.2 to 80 $\mu$m, preferably 0.5 to 60 $\mu$m, more preferably from 1 to 40 $\mu$m, even more preferably from 1 to 20 $\mu$m, and most preferably from 2 to 10 $\mu$m, determined by scanning electron microscopy, and/or wherein the microcapsule comprises from 1 to 5, preferably 2 or 3 individual loaded surface-reacted calcium carbonate particles.

Clause 7. The microcapsule according to any one of clauses 1 to 6, wherein the microcapsule comprises the active ingredient or inactive precursor thereof in an amount of at least 5 wt.-%, preferably at least 10 wt.-%, more preferably at least 25 wt.-%, and most preferably at least 50 wt.-%, based on the total weight of the microcapsule.

Clause 8. A microcapsule containing an active ingredient or inactive precursor thereof, comprising an active ingredient or inactive precursor thereof, and a multilayer shell encasing the active ingredient or inactive precursor thereof, wherein the shell comprises at least two complementary layers, wherein the microcapsule contains the active ingredient in an amount of at least 5 wt.-%, based on the total weight of the microcapsule.

Clause 9. The microcapsule according to clause 8, wherein the active ingredient is a pharmaceutically active ingredient, a cosmetic active ingredient, a nutraceutical active ingredient, a biocide, a pesticide, a wetting agent, a UV protecting agent, a scavenger, a pro-drug, a precursor of a cosmetic active ingredient, a precursor of a nutraceutical active ingredient, a precursor of a biocide, a precursor of a pesticide, a precursor of a wetting agent, a precursor of a UV protecting agent, a precursor of a scavenger, or a mixture thereof, preferably the active ingredient or inactive precursor thereof is a macromolecular active ingredient or an inactive precursor thereof, more preferably a protein, and most preferably lactoferrin, bovine serum albumin, or an enzyme.

Clause 10. The microcapsule according to clause 8 or 9, wherein the at least two complementary layers are formed from encapsulants independently selected from the group consisting of nanomaterials, macromolecular encapsulants and mixtures thereof, preferably the encapsulants are independently selected from the group consisting of polysaccharides, polyphenols, proteins, nucleic acids, cationic polyelectrolytes, anionic polyelectrolytes, nanomaterials and mixtures thereof, more preferably the encapsulants are independently selected from the group consisting of tannic acid, polyacrylic acid, poly(4-vinylpyridine), poly(glutamic acid), poly(lactic acid), chondroitin sulphate, dextran sulphate, poly(styrene sulfonate), sodium alginate, hyaluronic acid, polyphosphoric acid, polyvinyl sulfonic acid, polyvinyl phosphonic acid, polysulfates, dextrans, gum arabic, pectin, anionic cellulose derivatives, preferably carboxymethyl cellulose, pepsin, poly(dimethylammonium chloride), poly(ethylene oxide), poly(allylamine hydrochloride), poly(lysine), poly(lactic-co-glycolic acid), poly(arginine), protamine sulphate, polyethyleneimine, chitosan, glycol chitosan, montmorillonite, polyvinylamine, amine-containing polymers, such as polyamidoamine-epichlorohydrin and poly[2-(dimethylamino)ethyl methacrylate], bovine serum albumin, corresponding salts thereof, metal nanoparticles, metal oxide nanoparticles, carbon nanotubes, graphene, graphene oxide, nanoclays, and mixtures thereof, even more preferably the encapsulants are selected from the group consisting of tannic acid, poly(styrene sulfonate), poly(allylamine hydrochloride), bovine serum albumin, pepsin, corresponding salts thereof, and mixtures thereof, and most preferably the encapsulants are independently selected from the group consisting of tannic acid, pepsin, and corresponding salts thereof.

Clause 11. The microcapsule according to clause 10, wherein the first encapsulant is a protein and the second encapsulant is a polyphenol; or the first encapsulant is a polyphenol and the second encapsulant is a protein; or the first encapsulant is a cationic polyelectrolyte and the second encapsulant is an anionic polyelectrolyte; or the first encapsulant is an anionic polyelectrolyte and the second encapsulant is a cationic polyelectrolyte; preferably one of the first encapsulant or the second encapsulant is selected from the group consisting of tannic acid, polyacrylic acid, poly(glutamic acid), poly(lactic acid), poly(lactic-co-glycolic acid), chondroitin sulphate, dextran sulphate, poly(styrene sulfonate), montmorillonite, alginic acid, sodium alginate, polyphosphoric acid, polyvinyl sulfonic acid, polyvinyl phosphonic acid, polysulfates, gum arabic, poly(ethylene oxide), anionic cellulose derivatives, preferably carboxymethyl cellulose, corresponding salts thereof, and mixtures thereof, preferably the one of the first encapsulant or the second encapsulant is selected from the group consisting of tannic acid, poly(styrene sulfonate), corresponding salts thereof, and mixtures thereof, and the other one of the first or the second encapsulant is selected from the group consisting of pepsin, poly(dimethylammonium chloride), poly(allylamine hydrochloride), poly(lysine), poly(arginine), protamine sulphate, polyethyleneimine, poly(4-vinylpyridine), chitosan, glycol chitosan, polyvinylamine, amine-containing polymers, preferably polyamidoamine-epichlorohydrin or poly[2-(dimethylamino)ethyl methacrylate], bovine serum albumin, corresponding salts thereof, and mixtures thereof, preferably the other one of the first encapsulant or the second encapsulant is selected from the group consisting of pepsin, poly(allylamine hydrochloride), corresponding salts thereof, and mixtures thereof.

Clause 12. The microcapsule according to any one of clauses 8 to 11, wherein the microcapsule has a particle size in the range from 0.2 to 80 $\mu$m, preferably 0.5 to 60 $\mu$m, more preferably from 1 to 40 $\mu$m, even more preferably from 1 to 20 $\mu$m, and most preferably from 2 to 10 $\mu$m, determined by scanning electron microscopy, and/or wherein the microcapsule comprises from 1 to 5, preferably 2 or 3 individual loaded surface-reacted calcium carbonate particles.

Clause 13. The microcapsule according to any one of clauses 8 to 12, wherein the microcapsule comprises the active ingredient or inactive precursor thereof in an amount of at least 10 wt.-%, preferably at least 25 wt.-%, and most preferably at least 50 wt.-%, based on the total weight of the microcapsule.

Clause 14. Use of a surface-reacted calcium carbonate as a template for encapsulating an active ingredient or inactive precursor thereof using layer-by-layer assembly, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment.

Clause 15. The use according to clause 14, wherein the surface-reacted calcium carbonate has a BET specific surface area from 20 to 200 g/m$^2$, preferably 40 to 150 g/m$^2$, more preferably 70 to 120 g/m$^2$; and/or a volume median particle size $d_{50}$ from 0.1 to 75 $\mu$m, preferably from 0.5 to 50 $\mu$m, more preferably from 1 to 40 $\mu$m, even more preferably from 1.2 to 30 $\mu$m, and most preferably from 1.5 to 15 $\mu$m; and/or a volume top cut particle size $d_{98}$ from 0.2 to 150 $\mu$m, preferably from 1 to 100 $\mu$m, more preferably from 2 to 80 $\mu$m, even more preferably from 2.4 to 60 $\mu$m, and most preferably from 3 to 30 $\mu$m; and/or an intra-particle intruded specific pore volume in the range from 0.1 to 2.5 cm$^3$/g, more preferably from 0.2 to 2.2 cm$^3$/g, still more preferably from 0.4 to 2.0 cm$^3$/g and most preferably from 0.6 to 1.8 cm$^3$/g, determined by mercury porosimetry measurement.

Clause 16. The use according to clause 14 or 15, wherein the active ingredient is a pharmaceutically active ingredient, a cosmetic active ingredient, a nutraceutical active ingredient, a biocide, a pesticide, a wetting agent, a UV protecting agent, a scavenger, a pro-drug, a precursor of a cosmetic active ingredient, a precursor of a nutraceutical active ingredient, a precursor of a biocide, a precursor of a pesticide, a precursor of a wetting agent, a precursor of a UV protecting agent, a precursor of a scavenger, or a mixture thereof, preferably the active ingredient or inactive precursor thereof is a macromolecular active ingredient or an inactive precursor thereof, more preferably a protein, and most preferably lactoferrin, bovine serum albumin, or an enzyme.

Clause 17. The use according to any one of clauses 14 to 16, wherein the active ingredient is encapsulated by a multilayer shell, wherein the shell comprises at least two complementary layers, preferably the at least two complementary layers are formed from encapsulants independently selected from the group consisting of nanomaterials, macromolecular encapsulants and mixtures thereof, preferably the encapsulants are independently selected from the group consisting of polysaccharides, polyphenols, proteins, nucleic acids, cationic polyelectrolytes, anionic polyelectrolytes, nanomaterials and mixtures thereof, more preferably the encapsulants are independently selected from the group consisting of tannic acid, polyacrylic acid, poly(4-vinylpyridine), poly(glutamic acid), poly(lactic acid), chondroitin sulphate, dextran sulphate, poly(styrene sulfonate), sodium alginate, hyaluronic acid, polyphosphoric acid, polyvinyl sulfonic acid, polyvinyl phosphonic acid, polysulfates, dextrans, gum arabic, pectin, anionic cellulose derivatives, preferably carboxymethyl cellulose, pepsin, poly(dimethylammonium chloride), poly(ethylene oxide), poly(allylamine hydrochloride), poly(lysine), poly(lactic-co-glycolic acid), poly(arginine), protamine sulphate, polyethyleneimine, chitosan, glycol chitosan, montmorillonite, polyvinylamine, amine-containing polymers, such as polyamidoamine-epichlorohydrin and poly[2-(dimethylamino)ethyl methacrylate], bovine serum albumin, corresponding salts thereof, metal nanoparticles, metal oxide nanoparticles, carbon nanotubes, graphene, graphene oxide, nanoclays, and mixtures thereof, even more preferably the encapsulants are selected from the group consisting of tannic acid, poly(styrene sulfonate), poly(allylamine hydrochloride), bovine serum albumin, pepsin, corresponding salts thereof, and mixtures thereof, and most preferably the encapsulants are independently selected from the group consisting of tannic acid, pepsin, and corresponding salts thereof.

Clause 18. The use according to any one of clauses 14 to 17, wherein the encapsulation comprises the steps of i) incubating the loaded surface-reacted calcium carbonate in a first liquid medium comprising a first encapsulant to encase the loaded surface-reacted calcium carbonate with a first layer, ii) removing the first liquid medium and washing the loaded surface-reacted calcium carbonate obtained in step i) one or more times with a first solvent, preferably water, iii) incubating the loaded surface-reacted calcium carbonate obtained in step ii) in a second liquid medium comprising a second encapsulant being complementary to the first encapsulant to encase the loaded surface-reacted calcium carbonate with a second layer, iv) removing the second liquid medium and washing the loaded surface-reacted calcium carbonate obtained in step iii) one or more times with a second solvent, preferably water, and v) optionally repeating steps i) to iv) one or more times with the same or different first and second encapsulants and the same or different first and second liquid media and/or solvents.

Clause 19. The use according to clause 17 or 18, wherein the first encapsulant is a protein and the second encapsulant is a polyphenol; or the first encapsulant is a polyphenol and the second encapsulant is a protein; or the first encapsulant is a cationic polyelectrolyte and the second encapsulant is an anionic polyelectrolyte; or the first encapsulant is an anionic polyelectrolyte and the second encapsulant is a cationic polyelectrolyte; preferably one of the first encapsulant or the second encapsulant is selected from the group consisting of tannic acid, polyacrylic acid, poly(glutamic acid), poly(lactic acid), poly(lactic-co-glycolic acid), chondroitin sulphate, dextran sulphate, poly(styrene sulfonate), montmorillonite, alginic acid, sodium alginate, polyphosphoric acid, polyvinyl sulfonic acid, polyvinyl phosphonic acid, polysulfates, gum arabic, poly(ethylene oxide), anionic cellulose derivatives, preferably carboxymethyl cellulose, corresponding salts thereof, and mixtures thereof, preferably the one of the first encapsulant or the second encapsulant is selected from the group consisting of tannic acid, poly(styrene sulfonate), corresponding salts thereof, and mixtures thereof, and the other one of the first or the second encapsulant is selected from the group consisting of pepsin, poly(dimethylammonium chloride), poly(allylamine hydrochloride), poly(lysine), poly(arginine), protamine sulphate, polyethyleneimine, poly(4-vinylpyridine), chitosan, glycol chitosan, polyvinylamine, amine-containing polymers, preferably polyamidoamine-epichlorohydrin or poly[2-(dimethylamino)ethyl methacrylate], bovine serum albumin, corresponding salts thereof, and mixtures thereof, preferably the other one of the first encapsulant or the second encapsulant is selected from the group consisting of pepsin, poly(allylamine hydrochloride), corresponding salts thereof, and mixtures thereof.

Clause 20. The use according to any one of clauses 14 to 19, wherein the surface-reacted calcium carbonate template is removed by reaction with an acidic compound to decompose the loaded surface-reacted calcium carbonate within the multilayer shell, preferably the acidic compound is selected from the group consisting of hydrochloric acid, hydrobromic acid, sulphuric acid, nitric acid, acetic acid, formic acid, hydrogen sulfate, dihydrogen phosphate, hydrogen phosphate, calcium scavenging agents, preferably ethylene diamine tetraacetic acid, and salts and mixtures thereof, and more preferably the acidic compound is hydrochloric acid.

Clause 21. The use according to clause 20, wherein the removal is carried out in an aqueous medium, preferably wherein the acidic compound is added until the pH of the aqueous medium is in the range from 1 to 6, more preferably from 1.5 to 5, even more preferably from 2 to 4.5, and most preferably from 2.5 to 3.5.

Clause 22. The use according to any one of clauses 14 to 21, wherein the encapsulation yields a microcapsule, which is further dried, preferably the drying is freeze-drying.

Clause 23. The use according to any one of clauses 14 to 22, wherein the encapsulation yields a microcapsule having a particle size in the range from 0.2 to 80 $\mu$m, preferably 0.5 to 60 $\mu$m, more preferably from 1 to 40 $\mu$m, even more preferably from 1 to 20 $\mu$m, and most preferably from 2 to 10 $\mu$m, determined by scanning electron microscopy, and/or wherein the microcapsule comprises from 1 to 5, preferably 2 or 3 individual loaded surface-reacted calcium carbonate particles.

Clause 24. The use according to any one of clauses 14 to 23, wherein the encapsulation yields a microcapsule comprising the active ingredient or inactive precursor thereof in an amount of at least 5 wt.-%, preferably at least 10 wt.-%, more preferably at least 25 wt.-%, and most preferably at least 50 wt.-%, based on the total weight of the microcapsule.

Clause 25. A process for the production of a microcapsule comprising an active ingredient or inactive precursor thereof, wherein the process comprises the following steps: a) providing a surface-reacted calcium carbonate, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment, b) providing an active ingredient or inactive precursor thereof, c) contacting the active ingredient or inactive precursor thereof with the surface-reacted calcium carbonate to obtain a loaded surface-reacted calcium carbonate, and d) encasing the loaded surface-reacted calcium carbonate with a multilayer shell by consecutively depositing at least two complementary layers using layer-by-layer assembly onto the loaded surface-reacted calcium carbonate.

Clause 26. The process according to clause 25, wherein the process further comprises the step of e) reacting the microcapsule obtained in step d) with an acidic compound to decompose the loaded surface-reacted calcium carbonate within the multilayer shell, preferably the acidic compound is selected from the group consisting of hydrochloric acid, hydrobromic acid, sulphuric acid, nitric acid, acetic acid, formic acid, hydrogen sulfate, dihydrogen phosphate, hydrogen phosphate, calcium scavenging agents, preferably ethylene diamine tetraacetic acid, and salts and mixtures thereof, and more preferably the acidic compound is hydrochloric acid.

Clause 27. The process according to clause 26, wherein step e) is carried out in an aqueous medium, preferably wherein the acidic compound is added until the pH of the aqueous medium is in the range from 1 to 6, more preferably from 1.5 to 5, even more preferably from 2 to 4.5, and most preferably from 2.5 to 3.5.

Clause 28. The process according to any one of clauses 25 to 27, wherein the surface-reacted calcium carbonate has a BET specific surface area from 20 to 200 g/m$^2$, preferably 40 to 150 g/m$^2$, more preferably 70 to 120 g/m$^2$; and/or a volume median particle size dso from 0.1 to 75 $\mu$m, preferably from 0.5 to 50 $\mu$m, more preferably from 1 to 40 $\mu$m, even more preferably from 1.2 to 30 $\mu$m, and most preferably from 1.5 to 15 $\mu$m; and/or a volume top cut particle size $d_{98}$ from 0.2 to 150 $\mu$m, preferably from 1 to 100 $\mu$m, more preferably from 2 to 80 $\mu$m, even more

preferably from 2.4 to 60 $\mu$m, and most preferably from 3 to 30 $\mu$m; and/or an intra-particle intruded specific pore volume in the range from 0.1 to 2.5 cm$^3$/g, more preferably from 0.2 to 2.2 cm$^3$/g, still more preferably from 0.4 to 2.0 cm$^3$/g and most preferably from 0.6 to 1.8 cm$^3$/g, determined by mercury porosimetry measurement.

Clause 29. The process according to any one of clauses 25 to 28, wherein the active ingredient is a pharmaceutically active ingredient, a cosmetic active ingredient, a nutraceutical active ingredient, a biocide, a pesticide, a wetting agent, a UV protecting agent, a scavenger, a pro-drug, a precursor of a cosmetic active ingredient, a precursor of a nutraceutical active ingredient, a precursor of a biocide, a precursor of a pesticide, a precursor of a wetting agent, a precursor of a UV protecting agent, a precursor of a scavenger, or a mixture thereof, preferably the active ingredient or inactive precursor thereof is a macromolecular active ingredient or an inactive precursor thereof, more preferably a protein, and most preferably lactoferrin, bovine serum albumin, or an enzyme.

Clause 30. The process according to any one of clauses 25 to 29, wherein the at least two complementary layers are formed from encapsulants independently selected from the group consisting of nanomaterials, macromolecular encapsulants and mixtures thereof, preferably the encapsulants are independently selected from the group consisting of polysaccharides, polyphenols, proteins, nucleic acids, cationic polyelectrolytes, anionic polyelectrolytes, nanomaterials and mixtures thereof, more preferably the encapsulants are independently selected from the group consisting of tannic acid, polyacrylic acid, poly(4-vinylpyridine), poly(glutamic acid), poly(lactic acid), chondroitin sulphate, dextran sulphate, poly(styrene sulfonate), sodium alginate, hyaluronic acid, polyphosphoric acid, polyvinyl sulfonic acid, polyvinyl phosphonic acid, polysulfates, dextrans, gum arabic, pectin, anionic cellulose derivatives, preferably carboxymethyl cellulose, pepsin, poly(dimethylammonium chloride), poly(ethylene oxide), poly(allylamine hydrochloride), poly(lysine), poly(lactic-co-glycolic acid), poly(arginine), protamine sulphate, polyethyleneimine, chitosan, glycol chitosan, montmorillonite, polyvinylamine, amine-containing polymers, such as polyamidoamine-epichlorohydrin and poly[2-(dimethylamino)ethyl methacrylate], bovine serum albumin, corresponding salts thereof, metal nanoparticles, metal oxide nanoparticles, carbon nanotubes, graphene, graphene oxide, nanoclays, and mixtures thereof, even more preferably the encapsulants are selected from the group consisting of tannic acid, poly(styrene sulfonate), poly(allylamine hydrochloride), bovine serum albumin, pepsin, corresponding salts thereof, and mixtures thereof, and most preferably the encapsulants are independently selected from the group consisting of tannic acid, pepsin, and corresponding salts thereof.

Clause 31. The process according to any one of clauses 25 to 30, wherein step d) comprises the steps of i) incubating the loaded surface-reacted calcium carbonate in a first liquid medium comprising a first encapsulant to encase the loaded surface-reacted calcium carbonate with a first layer, ii) removing the first liquid medium and washing the loaded surface-reacted calcium carbonate obtained in step i) one or more times with a first solvent, preferably water, iii) incubating the loaded surface-reacted calcium carbonate obtained in step ii) in a second liquid medium comprising a second encapsulant being complementary to the first encapsulant to encase the loaded surface-reacted calcium carbonate with a second layer, iv) removing the second liquid medium and washing the loaded surface-reacted calcium carbonate obtained in step iii) one or more times with a second solvent, preferably water, and v) optionally repeating steps i) to iv) one or more times with the same or different first and second encapsulants and the same or different first and second liquid media and/or solvents.

Clause 32. The process according to clause 31, wherein the first encapsulant is a protein and the second encapsulant is a polyphenol; or the first encapsulant is a polyphenol and the second encapsulant is a protein; or the first encapsulant is a cationic polyelectrolyte and the second encapsulant is an anionic polyelectrolyte; or the first encapsulant is an anionic polyelectrolyte and the second encapsulant is a cationic polyelectrolyte; preferably one of the first encapsulant or the second encapsulant is selected from the group consisting of tannic acid, polyacrylic acid, poly(glutamic acid), poly(lactic acid), poly(lactic-co-glycolic acid), chondroitin sulphate, dextran sulphate, poly(styrene sulfonate), montmorillonite, alginic acid, sodium alginate, polyphosphoric acid, polyvinyl sulfonic acid, polyvinyl phosphonic acid, polysulfates, gum arabic, poly(ethylene oxide), anionic cellulose derivatives, preferably carboxymethyl cellulose, corresponding salts thereof, and mixtures thereof, preferably the one of the first encapsulant or the second encapsulant is selected from the group consisting of tannic acid, poly(styrene sulfonate), corresponding salts thereof, and mixtures thereof, and the other one of the first or the second encapsulant is selected from the group consisting of pepsin, poly(dimethylammonium chloride), poly(allylamine hydrochloride), poly(lysine), poly(arginine), protamine sulphate, polyethyleneimine, poly(4-vinylpyridine), chitosan, glycol chitosan, polyvinylamine, amine-containing polymers, preferably polyamidoamine-epichlorohydrin or poly[2-(dimethylamino)ethyl methacrylate], bovine serum albumin, corresponding salts thereof, and mixtures thereof, preferably the other one of the first encapsulant or the second encapsulant is selected from the group consisting of pepsin, poly(allylamine hydrochloride), corresponding salts thereof, and mixtures thereof.

Clause 33. The process according to any one of clauses 25 to 32, wherein the process further comprises the step of f) drying the microcapsule, preferably the drying is freeze-drying.

The following examples are intended to further illustrate the present invention. However, these examples should not be construed as limiting the scope of the present invention in any way.

**Examples**

**1. Measurement methods**

The following measurement methods were used to evaluate the parameters given in the examples and claims.

BET specific surface area

The BET specific surface area was measured via the BET process according to ISO 9277:2010 using nitrogen and an ASAP 2020 Micropore instrument (Micromeritics GmbH, Germany). The average pore size d was calculated from the BET surface area measurement under the assumption of a cylindrical pore shape using the equation d = 4 V/A, wherein A represents the determined BET surface area and V refers to the total pore volume of the particles as determined by the amount of adsorbed nitrogen at $p/p_0$ = 0.99.

Particle size distribution ($d_{10}$, $d_{50}$, $d_{90}$) of a particulate material

Volume median grain diameter and grain diameter distribution were determined via static light scattering. The measurement was made with a Mastersizer 2000 (Malvern Panalytical Ltd, UK) assuming a particle refractive index of 1.59 and particle adsorption index of 0.1. The method and the instrument are known to the skilled person and are commonly used to determine particle size distributions in fine powders. The measurement is carried out in water. The samples are dispersed using a high speed stirrer.

pH measurement

The pH of the aqueous samples is measured by using a standard pH-meter at approximately 25°C.

Centrifugation of samples

For centrifugation of large volumes, a swing-rotor large bench centrifuge (Multifuge 3SR from Heraeus) was used. For centrifugation of small volumes, a table centrifuge from Eppendorf was used.

UV-vis spectroscopy

UV spectra of samples were recorded on a UV-2450 UV-visible spectrophotometer from Shimadzu using precision cells made of quartz with optical path length 2 mm from Helma. Concentration of Lactoferrin (Lf) was determined from the intensity of absorption band peaked at 280 nm using extinction coefficient of 1.20 $(mg/mL)^{-1}cm^{-1}$. The wt.-% of Lf absorbed by and retained in samples was calculated using the known weight of initial powders, volumes of added Lf solution, and washing waters. Also the wt.-% of Lf was checked measuring the amount of Lf released from samples upon the addition of 2M hydrochloric acid solution until the pH reached 2.5.

High-performance liquid chromatography (HPLC)

HPLC was carried out on a 2695 Alliance System equipped with a 2996 photo diode array detector from Waters. The column used was an Aeris XB-C8, particle diameter 3.6 $\mu$m WIDEPORE from Phenomenex, 4.6 mm x 100 mm. Prior to analysis, samples are filtered through a 0.45 $\mu$m syringe filter, and the injection volume was 50 $\mu$l, if not stated otherwise, and the detection wavelength was 210 nm. Continuous gradient elution at 35°C and 1.0 ml/min flow rate was performed with 0.1 wt.-% of aqueous trifluoroacetic acid (TFA) solution in the mobile phase A, and 90 wt.-% acetonitrile and 10 wt.-% of aqueous TFA in the mobile phase B, following the gradient of linearly increasing the mobile phase B from 20 to 50% within 15 min, then decreasing the mobile phase B back to 20% within 20 min of elution.

Scanning Electron Microscopy and Energy Dispersive X-Ray Analysis (EDX)

The surface morphology of all tested microparticles and capsules were investigated by field emission gun scanning electron microscopy (FEGSEM, JEOL JSM-7600F) in secondary electron imaging mode at accelerating voltage of 5-15 kV. Samples were prepared by depositing a drop of a suspension on a silicon wafer and leaving it to dry at a slightly elevated temperature (~35-40 °C). Before imaging, the samples were coated with Au film through sputtering gold for 30 s by sputter coater (JEOL, JFC-1200). Elemental analysis was conducted with an Energy Dispersive X-ray Spectrometer (EDX) detector (Oxford Instruments).

## 2. Materials

Three different SRCC samples were obtained: SRCC1, SRCC2, and SRCC3, as described in Example 1.

Porous vaterite microparticles were purchased from TheraCross Technologies Pte Ltd (Singapore).

The bioactive lactoferrin from bovine whey is commercially available from Sigma-Aldrich under the CAS number 936541-36-5. Tannic acid (ACS grade), pepsin from porcine gastric mucosa (lyophilized powder, 3,200-4,500 units/mg protein), acetonitrile (for HPLC, ≥99.9%), hydrochloric acid were purchased from Sigma-Aldrich. A 0.1% aqueous solution of trifluoroacetic acid (TFA, LC/MS grade, ≥99.99%) was purchased from VWR Chemicals. All chemicals above were used as received without further purification. Deionized (DI) water (specific resistivity higher than 18.2 MΩcm) from Milli-Q plus 185 (Millipore) purification system was used to make all the solutions if not specified otherwise. In some experiments, reversed osmosis (RO) water was used.

## 3. Examples

Example 1: Determination of loading efficiency of surface-reacted calcium carbonate and vaterite samples with Lactoferrin (Lf).

300 mg of SRCC1 (a surface-reacted calcium carbonate having a $d_{10}$ of 1.04 μm, a dso of 19.37 μm, and a $d_{90}$ of 35.91 μm, and a BET specific surface area of 52.2 m$^2$/g) were suspended in 3 mL of an aqueous solution comprising 30 mg/ml of lactoferrin (Lf), and shaken for 15 min in a rotator. Then, the SRCC1 particles were collected by centrifugation, the supernatant was removed and residual Lf was removed by two consecutive washings with deionized (DI) water. Finally, the Lf-loaded SRCC1 particles were re-suspended in 1 ml of deionized water and 2M hydrochloric acid was added drop-wise until the pH reached 2.5, at which point the loaded SRCC1 particles completely decomposed. The Lf concentration in all supernatants and the released solution was determined by UV-vis spectroscopy.

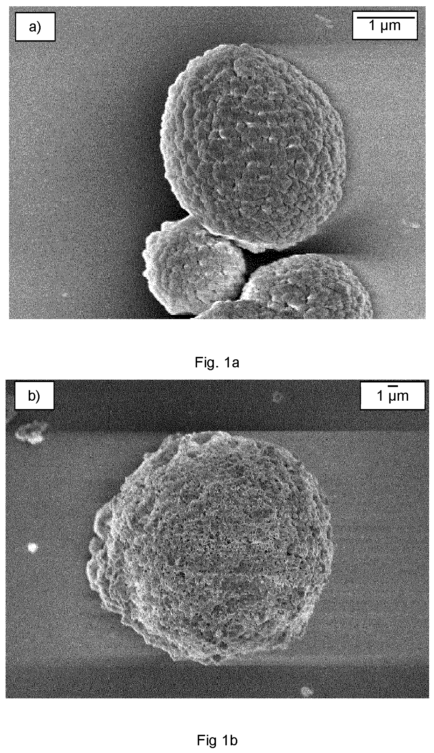

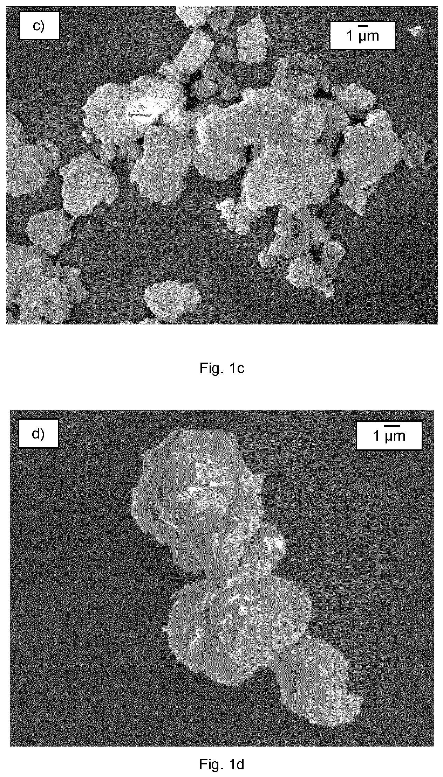

The same procedure as for SRCC1 was followed for SRCC2 (a surface-reacted calcium carbonate having a $d_{10}$ of 2.54 μm, a dso of 7.26 μm, and a $d_{90}$ of 14.92 μm, and a BET specific surface area of 24.5 m$^2$/g); SRCC3 (a surface-reacted calcium carbonate having a $d_{10}$ 3.76 μm, a dso of 8.69 μm, and a $d_{90}$ of 16.77 μm) and for vaterite (having a $d_{10}$ of 1.50 μm, a $d_{50}$ of 4.40 μm, and a $d_{90}$ of 8.15 μm, and a BET specific surface area of 2.35 m$^2$/g), with the exception that for vaterite the hydrochloric acid dosing was done until a pH of 3.5 was reached. The physical data of vaterite, SRCC1, SRCC2, and SRCC3 are summarized in Table 1. SEM micrographs of vaterite, SRCC1, SRCC2 and SRCC3 are shown in Fig. 1, a, b, c, and d respectively.

**Table 1:** Characteristics of the particle size distribution, pore size and surface area of the samples.

| Sample | $d_{10}$, μm | $d_{50}$, μm | $d_{90}$, μm | Pore size, nm | BET surface area, m$^2$/g |
|---|---|---|---|---|---|
| Vaterite | 1.50 | 4.40 | 8.15 | 87.2 | 2.35 |
| SRCC1 | 1.04 | 19.37 | 35.91 | 23.3 | 52.2 |
| SRCC2 | 2.54 | 7.26 | 14.92 | 20.4 | 24.5 |
| SRCC3 | 3.76 | 8.69 | 16.77 | -- | -- |

Lactoferrin adsorption:

The wt.-% of Lf (lactoferrin) absorbed by 300 mg of the sample powders from 3 mL of 30 mg/mL solutions, retained upon further washings with 5 mL of deionized water and released upon treatment with HCl are shown in Table 2.

**Table 2:** Lf retained in samples upon washing with deionized water.

| sample | Lf absorbed by samples, wt.% | | | |
|---|---|---|---|---|
| | Absorbed | Retained after 1st wash | Retained after 2nd wash | Released upon HCl treatment |
| Vaterite | 3.64 ± 0.07 | 1.29 ± 0.14 | 1.06 ± 0.16 | 0.86 ± 0.01 |
| SRCC 1 | 10.61 ± 0.25 | 8.69 ± 0.27 | 8.50 ± 0.27 | 6.84 ± 0.20 |
| SRCC 2 | 8.72 ± 0.37 | 5.59 ± 0.50 | 5.28 ± 0.54 | 5.89 ± 0.15 |

(continued)

| sample | Lf absorbed by samples, wt.% | | | |
|---|---|---|---|---|
| | Absorbed | Retained after 1st wash | Retained after 2nd wash | Released upon HCl treatment |
| SRCC 3 | $10.90 \pm 0.04$ | $7.42 \pm 0.10$ | $6.98 \pm 0.10$ | $7.96 \pm 0.11$ |

SRCC1 and SRCC3 demonstrate the highest wt. % of absorbed Lf and the highest retention upon consecutive washings with DI water (65-80% of initially absorbed Lf is retained). Vaterite demonstrates the lowest wt. % of absorbed Lf and the lowest retention upon consecutive washings with DI water (~25-30 %). Therefore Lf-loaded SRCC1 and SRCC3 were chosen as templates for subsequent LbL assembly of four TA-pepsin bi-layers (TA-pepsin)$_4$.

Example 2: Layer-by-layer (LbL) assembly of tannic acid (TA) and pepsin multilayer shell over Lf-loaded SRCC1

The layer-by-layer assembly of tannic acid and pepsin shells (TA-pepsin shells) was performed as follows. First, 100 g of SRCC1 particles were purified from fines by elutriation in an expanded bed at 75 mL/min flow rate, as shown in the Table 3.

**Table 3:** Particle size distribution in the initial SRCC1 powder, expanded bed, and compacted bed.

| Sample | $d_{10}$, $\mu$m | $d_{50}$, $\mu$m | $d_{90}$, $\mu$m |
|---|---|---|---|
| SRCC1 | 1.04 | 19.37 | 35.91 |
| expanded bed - 10 min | 2.1 | 5.1 | 13.6 |
| expanded bed - 30 min | 2.4 | 6.5 | 15.1 |
| expanded bed - 60 min | 3.0 | 8.2 | 17.8 |
| Compacted bed | 14.9 | 26.4 | 43.9 |

Then, the compacted bed was re-suspended in 1 liter of 30 g/l of Lf solution in reverse-osmosis purified water (RO water) and stirred for 15 min at a speed of 450 rpm. The suspension was transferred into two 600 ml centrifuge cans; the supernatant was separated using a swing-rotor large bench centrifuge Multifuge 3SR from Heraeus at 4300 rpm for 5 min. The particles were washed two times with 1 L of RO water to remove unabsorbed Lf. Each layer was assembled by 10 min incubation of particles in 1 L of a respective TA (3 g/L) or pepsin (2 g/L) solution followed by two washing steps with 1L of RO water. After the shell comprised four TA/Pepsin bi-layers, the particles were re-suspended in 800 mL of RO water and concentrated hydrochloric acid (37 wt.-% HCl in $H_2O$) was slowly added under vigorous agitation until the pH reached 2.5 to decompose the template material. Subsequently, the capsules were washed with RO water until the pH reached about 5.0, separated from the supernatant and the obtained wet cake was freeze dried.
The EDX analysis of blank SRCC1 particles and Layer-by-layer (LbL) assembled Lf-loaded microcapsules prepared using SRCC1 particles as template is shown in Table 4.

**Table 4:** EDX analysis of blank SRCC1 particles and Layer-by-layer (LbL) assembly of Lf-loaded SRCC1 particles

| Sample | wt. % of the elements | | | | | |
|---|---|---|---|---|---|---|
| | Ca | C | O | P | N | Fe |
| SRCC1 | 38.7 | 11.3 | 44.9 | 5.1 | 0 | 0 |
| Lf/(TA-pepsin)4 | 0 | 56.4 | 31.8 | 0 | 11.4 | 0.2 |

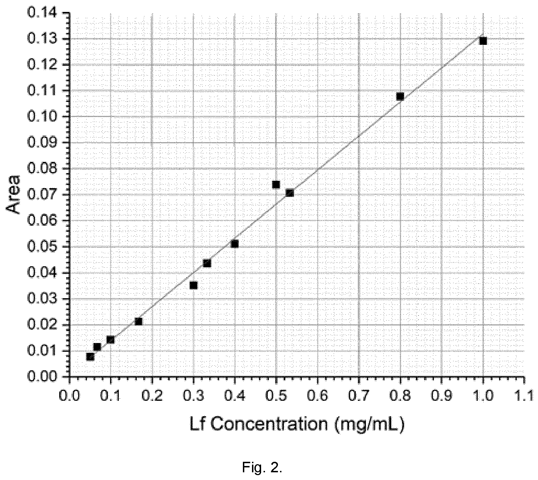

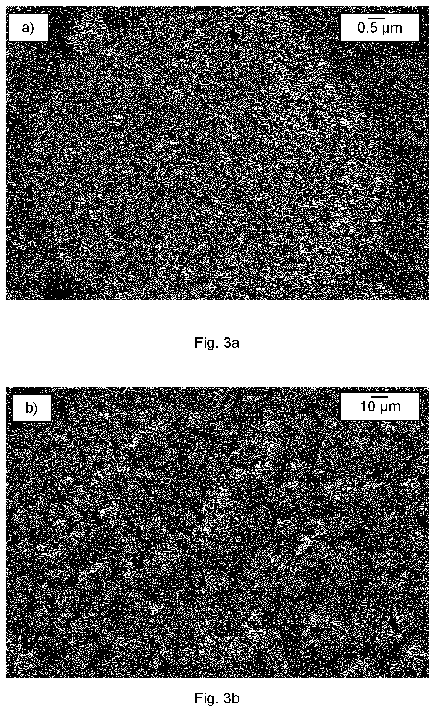

The SRCC1 particles consist of calcium carbonate and hydroxyapatite, therefore the main elements detected were Ca, C, O, and P. The LbL assembled capsules do not have detectable levels of Ca and P, which shows the complete removal of the SRCC1 template material. The capsules contain significant amounts of N from both encapsulated Lf and pepsin of the shells. EDX detected minor amounts of Fe in the capsules. Without being bound by any theory, the inventors believe that the iron signal may come from Lf, as it is an iron-binding protein of transferrin family, or TA of the shell, as tannins are well-documented antioxidants able to scavenge $Fe^{2+}$ ions from water.
To quantify the Lf content in LbL-assembled capsules, the capsules were decomposed in 8M urea solution at pH 8.4 and the amount of released Lf was determined by HPLC using calibration curve as shown in Fig. 2. The capsules were found to contain 57.4 ± 0.95 wt.% of Lf, based on the total weight of the capsules. SEM micrographs of the obtained material at different levels of magnification are shown in Fig. 3.

Protective efficiency of the microcapsules in gastric digestion:

In vitro gastric digestion of encapsulated Lactoferrin was studied following the INFOGEST protocol (A. Brodkorb et al., "INFOGEST static in vitro simulation of gastrointestinal food digestion", Nature Protocols 2019, 14, 991-1014). 30 mg of encapsulated Lf was dispersed in 150 mM NaCl solution at pH 3 (acidified by HCl) and 37 °C. Then pepsin solution was added followed by the gastric lipase solution to achieve an activity of 2,000 U/mL and 60 U/mL in the final digestion mixture, respectively. The samples were incubated at 37 °C upon agitation for 2 h from the point at which pepsin was added. Digestion was stopped by adjusting the pH to 7.0 with NaOH.

The capsules treated with simulated gastric fluid were washed twice with RO water and then treated with 8M urea solution in tris buffer (pH 8.4) for 1 hr, at which point all the capsules were decomposed. The amount of released lactoferrin was quantified by HPLC. The sample was found to contain 36 ± 4 wt.% of Lf, based on the weight of initial capsules. Therefore, protection efficiency of capsules against gastric digestion was found to be 63%.

Example 3: Laver-bv-laver (LbL) assembly of tannic acid (TA) and pepsin multilayer shell over Lf-loaded SRCC3

The layer-by-layer assembly of tannic acid and pepsin shells (TA-pepsin shells) was performed as follows. First, 100 g of SRCC3 particles were re-suspended in 1 liter of 30 g/L of Lf solution in DI water and stirred for 15 min at a speed of 450 rpm. The suspension was transferred into six 250 ml centrifuge cans; the supernatant was separated using a centrifuge Rotanta 460 from Hettich at 4000 rpm for 4 min. The particles were washed two times with 1 L of DI water to remove unabsorbed Lf. Each layer was assembled by 10 min incubation of particles in 1 L of a respective TA (3 g/L) or pepsin (2 g/L) solution followed by two washing steps with 1L of DI water. After the shell comprised four TA/Pepsin bi-layers, the particles were re-suspended in 800 mL of DI water and concentrated hydrochloric acid (37 wt.-% HCl in $H_2O$) was slowly added under vigorous agitation until the pH reached 2.5 to decompose the template material. Subsequently, the capsules were washed with DI water until the pH reached about 5.0, separated from the supernatant and the obtained wet cake was freeze dried.

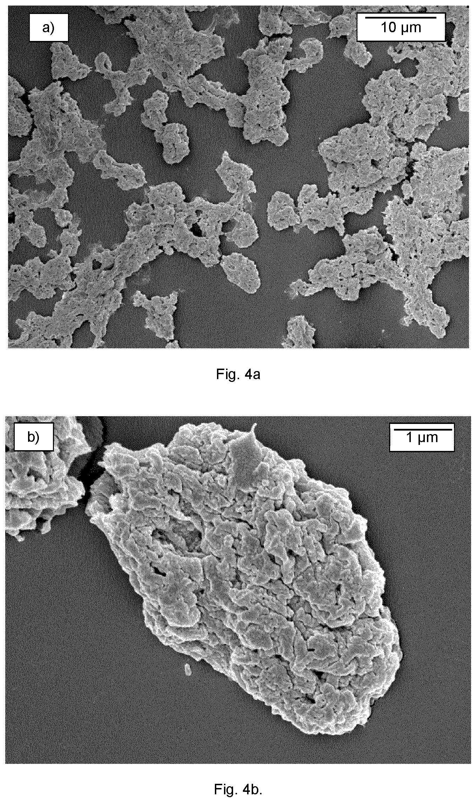

To quantify the Lf content in LbL-assembled capsules, the capsules were decomposed in 8M urea solution at pH 8.4 and the amount of released Lf was determined by HPLC. The capsules were found to contain 42.6 ± 6.6 wt. % of Lf, based on the total weight of the capsules. SEM micrographs of the obtained material at different levels of magnification are shown in Fig. 4.

Protective Efficiency of the microcapsules in gastric digestion:

In vitro gastric digestion of encapsulated Lactoferrin was studied following the INFOGEST protocol (A. Brodkorb et al., "INFOGEST static in vitro simulation of gastrointestinal food digestion", Nature Protocols 2019, 14, 991-1014). 30 mg of encapsulated Lf was dispersed in 150 mM NaCl solution at pH 3 (acidified by HCl) and 37 °C. Then pepsin solution was added followed by the gastric lipase solution to achieve an activity of 2,000 U/mL and 60 U/mL in the final digestion mixture, respectively. The samples were incubated at 37 °C upon agitation for 2 h from the point at which pepsin was added. Digestion was stopped by adjusting the pH to 7.0 with NaOH.

The capsules treated with simulated gastric fluid were washed twice with DI water and then treated with 8M urea solution in tris buffer (pH 8.4) for 1 hr, at which point all the capsules were decomposed. The amount of released lactoferrin was quantified by HPLC. The sample was found to contain 27.7 ± 1.5 wt. % of Lf, based on the weight of initial capsules. Therefore, protection efficiency of capsules against gastric digestion was found to be 65%.

**Claims**

1. A process for the production of a microcapsule comprising an active ingredient or inactive precursor thereof, wherein the process comprises the following steps:

a) providing a surface-reacted calcium carbonate, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment,

b) providing an active ingredient or inactive precursor thereof,

c) contacting the active ingredient or inactive precursor thereof with the surface-reacted calcium carbonate to obtain a loaded surface-reacted calcium carbonate, and

d) encasing the loaded surface-reacted calcium carbonate with a multilayer shell by consecutively depositing at least two complementary layers using layer-by-layer assembly onto the loaded surface-reacted calcium carbonate.

2. The process according to claim 1, wherein the process further comprises the step of

e) reacting the microcapsule obtained in step d) with an acidic compound to decompose the loaded surface-reacted calcium carbonate within the multilayer shell, preferably the acidic compound is selected from the group consisting of hydrochloric acid, hydrobromic acid, sulphuric acid, nitric acid, acetic acid, formic acid, hydrogen sulfate, dihydrogen phosphate, hydrogen phosphate, calcium scavenging agents, preferably ethylene diamine tetraacetic acid, and salts and mixtures thereof, and more preferably the acidic compound is hydrochloric acid.

3. The process according to claim 2, wherein step e) is carried out in an aqueous medium, preferably wherein the acidic compound is added until the pH of the aqueous medium is in the range from 1 to 6, more preferably from 1.5 to 5, even more preferably from 2 to 4.5, and most preferably from 2.5 to 3.5.

4. The process according to any one of the preceding claims, wherein the surface-reacted calcium carbonate has

- a BET specific surface area from 20 to 200 $g/m^2$, preferably 40 to 150 $g/m^2$, more preferably 70 to 120 $g/m^2$; and/or
- a volume median particle size $d_{50}$ from 0.1 to 75 $\mu$m, preferably from 0.5 to 50 $\mu$m, more preferably from 1 to 40 $\mu$m, even more preferably from 1.2 to 30 $\mu$m, and most preferably from 1.5 to 15 $\mu$m; and/or
- a volume top cut particle size $d_{98}$ from 0.2 to 150 $\mu$m, preferably from 1 to 100 $\mu$m, more preferably from 2 to 80 $\mu$m, even more preferably from 2.4 to 60 $\mu$m, and most preferably from 3 to 30 $\mu$m; and/or
- an intra-particle intruded specific pore volume in the range from 0.1 to 2.5 $cm^3/g$, more preferably from 0.2 to 2.2 $cm^3/g$, still more preferably from 0.4 to 2.0 $cm^3/g$ and most preferably from 0.6 to 1.8 $cm^3/g$, determined by mercury porosimetry measurement.

5. The process according to any one of the preceding claims, wherein the active ingredient is a pharmaceutically active ingredient, a cosmetic active ingredient, a nutraceutical active ingredient, a biocide, a pesticide, a wetting agent, a UV protecting agent, a scavenger, a pro-drug, a precursor of a cosmetic active ingredient, a precursor of a nutraceutical active ingredient, a precursor of a biocide, a precursor of a pesticide, a precursor of a wetting agent, a precursor of a UV protecting agent, a precursor of a scavenger, or a mixture thereof, preferably the active ingredient or inactive precursor thereof is a macromolecular active ingredient or an inactive precursor thereof, more preferably a protein, and most preferably lactoferrin, bovine serum albumin, or an enzyme.

6. The process according to any one of the preceding claims, wherein the at least two complementary layers are formed from encapsulants independently selected from the group consisting of nanomaterials, macromolecular encapsulants and mixtures thereof, preferably the encapsulants are independently selected from the group consisting of polysaccharides, polyphenols, proteins, nucleic acids, cationic polyelectrolytes, anionic polyelectrolytes, nanomaterials and mixtures thereof, more preferably the encapsulants are independently selected from the group consisting of tannic acid, polyacrylic acid, poly(4-vinylpyridine), poly(glutamic acid), poly(lactic acid), chondroitin sulphate, dextran sulphate, poly(styrene sulfonate), sodium alginate, hyaluronic acid, polyphosphoric acid, polyvinyl sulfonic acid, polyvinyl phosphonic acid, polysulfates, dextrans, gum arabic, pectin, anionic cellulose derivatives, preferably carboxymethyl cellulose, pepsin, poly(dimethylammonium chloride), poly(ethylene oxide), poly(allylamine hydrochloride), poly(lysine), poly(lactic-co-glycolic acid), poly(arginine), protamine sulphate, polyethyleneimine, chitosan, glycol chitosan, montmorillonite, polyvinylamine, amine-containing polymers, such as polyamidoamine-epichlorohydrin and poly[2-(dimethylamino)ethyl methacrylate], bovine serum albumin, corresponding salts thereof, metal nanoparticles, metal oxide nanoparticles, carbon nanotubes, graphene, graphene oxide, nanoclays, and mixtures thereof, even more preferably the encapsulants are selected from the group consisting of tannic acid, poly(styrene sulfonate), poly(allylamine hydrochloride), bovine serum albumin, pepsin, corresponding salts thereof, and mixtures thereof, and most preferably the encapsulants are independently selected from the group consisting of tannic acid, pepsin, and corresponding salts thereof.

7. The process according to any one of the preceding claims, wherein step d) comprises the steps of

i) incubating the loaded surface-reacted calcium carbonate in a first liquid medium comprising a first encapsulant to encase the loaded surface-reacted calcium carbonate with a first layer,

ii) removing the first liquid medium and washing the loaded surface-reacted calcium carbonate obtained in step i) one or more times with a first solvent, preferably water,

iii) incubating the loaded surface-reacted calcium carbonate obtained in step ii) in a second liquid medium comprising a second encapsulant being complementary to the first encapsulant to encase the loaded surface-reacted calcium carbonate with a second layer,

iv) removing the second liquid medium and washing the loaded surface-reacted calcium carbonate obtained in step iii) one or more times with a second solvent, preferably water, and

v) optionally repeating steps i) to iv) one or more times with the same or different first and second encapsulants and the same or different first and second liquid media and/or solvents.

8.  The process according to claim 7, wherein the first encapsulant is a protein and the second encapsulant is a polyphenol; or the first encapsulant is a polyphenol and the second encapsulant is a protein; or the first encapsulant is a cationic polyelectrolyte and the second encapsulant is an anionic polyelectrolyte; or the first encapsulant is an anionic polyelectrolyte and the second encapsulant is a cationic polyelectrolyte; preferably

- one of the first encapsulant or the second encapsulant is selected from the group consisting of tannic acid, polyacrylic acid, poly(glutamic acid), poly(lactic acid), poly(lactic-co-glycolic acid), chondroitin sulphate, dextran sulphate, poly(styrene sulfonate), montmorillonite, alginic acid, sodium alginate, polyphosphoric acid, polyvinyl sulfonic acid, polyvinyl phosphonic acid, polysulfates, gum arabic, poly(ethylene oxide), anionic cellulose derivatives, preferably carboxymethyl cellulose, corresponding salts thereof, and mixtures thereof, preferably the one of the first encapsulant or the second encapsulant is selected from the group consisting of tannic acid, poly(styrene sulfonate), corresponding salts thereof, and mixtures thereof, and

the other one of the first or the second encapsulant is selected from the group consisting of pepsin, poly(dimethylammonium chloride), poly(allylamine hydrochloride), poly(lysine), poly(arginine), protamine sulphate, polyethyleneimine, poly(4-vinylpyridine), chitosan, glycol chitosan, polyvinylamine, amine-containing polymers, preferably polyamidoamine-epichlorohydrin or poly[2-(dimethylamino)ethyl methacrylate], bovine serum albumin, corresponding salts thereof, and mixtures thereof, preferably the other one of the first encapsulant or the second encapsulant is selected from the group consisting of pepsin, poly(allylamine hydrochloride), corresponding salts thereof, and mixtures thereof.

9.  The process according to any one of the preceding claims, wherein the process further comprises the step of f) drying the microcapsule, preferably the drying is freeze-drying.

10. A microcapsule comprising an active ingredient or inactive precursor thereof obtainable by a process according to any one of claims 1 to 9.

11. The microcapsule according to claim 10, wherein the microcapsule has a particle size in the range from 0.2 to 80 $\mu$m, preferably 0.5 to 60 $\mu$m, more preferably from 1 to 40 $\mu$m, even more preferably from 1 to 20 $\mu$m, and most preferably from 2 to 10 $\mu$m, determined by scanning electron microscopy, and/or wherein the microcapsule comprises from 1 to 5, preferably 2 or 3 individual loaded surface-reacted calcium carbonate particles.

12. The microcapsule according to claim 10 or 11, wherein the microcapsule comprises the active ingredient or inactive precursor thereof in an amount of at least 5 wt.-%, preferably at least 10 wt.-%, more preferably at least 25 wt.-%, and most preferably at least 50 wt.-%, based on the total weight of the microcapsule.

13. Use of a surface-reacted calcium carbonate as a template for encapsulating an active ingredient or inactive precursor thereof using layer-by-layer assembly, wherein the surface-reacted calcium carbonate is a reaction product of natural ground calcium carbonate or precipitated calcium carbonate with carbon dioxide and one or more $H_3O^+$ ion donors, wherein the carbon dioxide is formed in situ by the $H_3O^+$ ion donors treatment.

14. A product comprising the microcapsule according to any one of claims 10 to 12, wherein the product is a pharmaceutical product, a foodstuff, a feedstuff, a food supplement, a feed supplement, a cosmetic product, a paper product, a painting product, a coating product, or an agricultural product.

15. Use of a microcapsule according to any one of claims 10 to 12 in pharmaceutical, cosmetic, nutraceutical, paper, paint, coating, biological, industrial or agricultural applications.

Fig. 1a

Fig 1b

Fig. 1c

Fig. 1d

Fig. 2.

Fig. 3a

Fig. 3b

a)

10 µm

Fig. 4a

b)

1 µm

Fig. 4b.

Europäisches
Patentamt

European
Patent Office

Office européen
des brevets

# EUROPEAN SEARCH REPORT

Application Number

EP 20 18 1641

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ELBAZ NANCY M ET AL: "Controlled synthesis of calcium carbonate nanoparticles and stimuli-responsive multi-layered nanocapsules for oral drug delivery", INTERNATIONAL JOURNAL OF PHARMACEUTICS, ELSEVIER, NL, vol. 574, 23 November 2019 (2019-11-23), XP085989592, ISSN: 0378-5173, DOI: 10.1016/J.IJPHARM.2019.118866 [retrieved on 2019-11-23] * page 3 - page 4 * * pages 6, 10 * * figure 1 * | 1-15 | INV. B01J13/10 B01J13/22 A01N25/28 A23P10/30 A61K8/11 A61K9/50 |
| A | DATABASE WPI Week 2020 Thomson Scientific, London, GB; AN 2020-04132N XP002801181, -& CN 110 639 442 A (UNIV TIANJIN POLYTECHNIC) 3 January 2020 (2020-01-03) * the whole document * | 1-15 | |
| A | WO 2014/062689 A1 (ULTRA INK LLC [US]) 24 April 2014 (2014-04-24) * the whole document * | 1-15 | TECHNICAL FIELDS SEARCHED (IPC) B01J A01N A23P A61K A61Q |
| A | DATABASE WPI Week 201419 Thomson Scientific, London, GB; AN 2013-W95726 XP002801182, -& CN 103 329 896 A (BEIJING INST TECHNOLOGY) 2 October 2013 (2013-10-02) * the whole document * | 1-15 | |

-/--

The present search report has been drawn up for all claims

| Place of search | Date of completion of the search | Examiner |
|---|---|---|
| The Hague | 20 November 2020 | Tarallo, Anthony |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 20 18 1641

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2007/207212 A1 (HAYNIE DONALD T [US] ET AL) 6 September 2007 (2007-09-06) * the whole document * | 1-15 | |
| A | EP 3 622 966 A1 (OMYA INT AG [CH]) 18 March 2020 (2020-03-18) * the whole document * | 1-15 | |
| A | WO 2013/043812 A1 (UNIV TEXAS [US]; APPLEFORD MARK [US]; KELLEY MARIE-MICHELLE [US]) 28 March 2013 (2013-03-28) * the whole document * | 1-15 | |

TECHNICAL FIELDS
SEARCHED     (IPC)

The present search report has been drawn up for all claims

| Place of search | Date of completion of the search | Examiner |
|---|---|---|
| The Hague | 20 November 2020 | Tarallo, Anthony |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 18 1641

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2020

| Patent document cited in search report | | Publication date | Patent family member(s) | | Publication date |
|---|---|---|---|---|---|
| CN 110639442 | A | 03-01-2020 | NONE | | |
| WO 2014062689 | A1 | 24-04-2014 | AU | 2013331432 A1 | 21-05-2015 |
| | | | AU | 2017203567 A1 | 22-06-2017 |
| | | | CA | 2927441 A1 | 24-04-2014 |
| | | | EP | 2906645 A1 | 19-08-2015 |
| | | | JP | 2015535012 A | 07-12-2015 |
| | | | US | 2015265508 A1 | 24-09-2015 |
| | | | WO | 2014062689 A1 | 24-04-2014 |
| CN 103329896 | A | 02-10-2013 | NONE | | |
| US 2007207212 | A1 | 06-09-2007 | AU | 2006346493 A1 | 31-01-2008 |
| | | | CA | 2628574 A1 | 31-01-2008 |
| | | | CN | 101309670 A | 19-11-2008 |
| | | | DK | 1957050 T3 | 20-01-2014 |
| | | | EP | 1957050 A2 | 20-08-2008 |
| | | | ES | 2444572 T3 | 25-02-2014 |
| | | | IL | 191152 A | 31-08-2014 |
| | | | JP | 5189493 B2 | 24-04-2013 |
| | | | JP | 2009515528 A | 16-04-2009 |
| | | | US | 2007207212 A1 | 06-09-2007 |
| | | | WO | 2008013558 A2 | 31-01-2008 |
| EP 3622966 | A1 | 18-03-2020 | NONE | | |
| WO 2013043812 | A1 | 28-03-2013 | US | 2013101669 A1 | 25-04-2013 |
| | | | WO | 2013043812 A1 | 28-03-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20080020051 A1
- WO 2010097814 A2
- JP 2011144056 A
- FR 2787802 B1
- WO 0039222 A1
- US 20040020410 A1
- WO 2018011343 A1
- EP 2591772 A1
- WO 2014057026 A1
- WO 2016096997 A1
- WO 2004083316 A1

- WO 2005121257 A2
- WO 2009074492 A1
- EP 2264108 A1
- EP 2264109 A1
- US 4627977 A, Gaffar
- US 4985459 A
- US 20030157213 A1
- US 20030206993 A1
- US 20030099741 A1
- DE 19812083 A1

### Non-patent literature cited in the description

- **LANGMUIR.** *Matrix Polyelectrolyte Microcapsules: New System for Macromolecule Encapsulation,* 2004, vol. 20, 3398-3406
- **VERT et al.** Terminology for biorelated polymers and applications (IUPAC Recommendations 2012). *Pure Appl. Chem.,* 2012, vol. 84 (2), 377-410
- **WANG et al.** Template Synthesis of Nanostructured Materials via Layer-by-Layer Assembly. *Chem. Mater.,* 2008, vol. 20, 848-858
- Multilayer Thin Films: Sequential Assembly of Nanocomposite Materials. Wiley, 2012, 1-19
- **GANE, P.A.C. ; KETTLE, J.P. ; MATTHEWS, G.P. ; RIDGWAY, C.J.** Void Space Structure of Compressible Polymer Spheres and Consolidated Calcium Carbonate Paper-Coating Formulations. *Industrial and Engineering Chemistry Research,* 1996, vol. 35 (5), 1753-1764

- Multilayer Thin Films: Sequential Assembly of Nanocomposite Materials. Wiley, 2012
- **D. V. VOLODKIN et al.** Matrix Polyelectrolyte Microcapsules: New System for Macromolecule Encapsulation. *Langmuir,* 2004, vol. 20, 3398-3406
- **A. BRODKORB et al.** INFOGEST static in vitro simulation of gastrointestinal food digestion. *Nature Protocol,* 2019, vol. 14, 991-1014
- **A. BRODKORB et al.** INFOGEST static in vitro simulation of gastrointestinal food digestion. *Nature Protocols,* 2019, vol. 14, 991-1014